# EUROPEAN PATENT SPECIFICATION

(11) **EP 0 686 632 B1**
(45) Date of publication and mention of the grant of the patent: **06.08.2003**
(21) Application number: 95903941.3
(22) Date of filing: 22.12.1994
(51) Int. Cl.: C07D 279/02, C07D 417/06, C07D 513/10, A61K 31/54

(54) **BENZOTHIAZINE DERIVATIVE**
BENZOTHIAZINDERIVATE
DERIVE DE BENZOTHIAZINE

(30) Priority: 24.12.1993 JP 34586593
(43) Date of publication of application: 13.12.1995
(73) Proprietor: Daiichi Suntory Pharma Co., Ltd., Tokyo 102-8530 (JP)
(72) Inventor: MIZUNO, Akira 1-1-807, Oharano-Higashitakenosato, Kyoto 610-11 (JP); SHIBATA, Makoto, Osaka 563 (JP); IWAMORI, Tomoe, Osaka 567 (JP); INOMATA, Norio, Osaka 562 (JP)
(74) Representative: VOSSIUS & PARTNER
(86) International application number: JP9402194
(87) International publication number: WO95018117

(56) References cited:
- WO-A-93/16073
- FR-A- 2 675 801
- JP-A- 1 311 059
- JP-A- 2 067 274
- JP-A- 3 255 063
- Eur. J. Pharmacol., Vol. 204, No. 1, 1991, ROGER GODBOUT et al., pp. 97-100.
- J. Med. Chem., Vol. 36, Apr. 1993, JEAN-LUC MALLERON et al., pp. 1194-1206.
- J. Med. Chem., Vol. 34, 1991, JEAN-LUC MALLERON et al., pp. 2477-2483.

## Description

### Technical Field

This invention relates to novel benzothiazine derivatives. More specifically, this invention is concerned with benzothiazine derivatives and salts thereof, said derivatives and salts being useful for the prevention or treatment of ischemic heart diseases such as angina pectoris, arrhythmia, myocardial infarction, congestive heart failure and post-PTCA restenosis, cerebrovascular disturbances such as cerebral infarction and cerebral sequelae after subarachnoid hemorrhage, and/or peripheral circulatory disturbances such as arteriosclerosis obliterans, Raynaud disease, Buerger disease and thrombophlebitis; their preparation process; and therapeutics for circulatory diseases, said therapeutics containing them as effective ingredients.

### Background Art

Serotonin is a compound contained abundantly in platelets, which are a blood component, and in a central nervous system, it acts as a neurotransmitter. In platelets, it is released upon stimulation by throm-boxane A₂, ADP, collagen and synergistically acts on various platelet aggregation factors and vasoconstrictors through activation of serotonin-2 receptors in the platelets and vascular smooth muscle cells, thereby inducing strong platelet aggregation and vasoconstriction [P.M. Vanhoutte, "Journal of Cardiovascular Pharmacology", Vol. 17 (Supple. 5), S6-S12 (1991)].

Serotonin is also known to potentiate proliferation of vascular smooth muscle cells [S. Araki et al., "Atherosclerosis", Vol. 83, p29-p34(1990)]. It has been considered that, particularly when endothelial cells are injured as in arteriosclerosis or myocardial infarction, the vasoconstricting action and thrombus forming action of serotonin are exasperated, thereby reducing or even stopping blood supply to myocardial, cerebral and peripheral organs [P. Golino et al., "The New England Journal of Medicine", Vol. 324, No. 10, p641-p648(1991), Y. Takiguchi et al., "Thrombosis and Haemostasis", Vol. 68(4), p460-p463(1992), A.S. Weyrich et al., "American Journal of Physiology", Vol. 263, H349-H358(1992)].

Being attracted by such actions of serotonin or serotonin-2 receptors, various attempts are now under way to use a serotonin-2 receptor antagonist as a pharmaceutical for ischemic diseases of the heart, the brain and peripheral tissues.

Ketanserin which has therapeutically been used as a hypotensive drug is known as a compound having antagonistic action against a serotonin-2 receptor. Ketanserin has strong antagonistic action against a sympathetic nerve α₁ receptor and also against histamine 1 and dopamine receptors in addition to as antagonistic action against serotonin-2 receptors, so that there is the potential problem of developing excessive hypotensive action, neuroleptic action or the like when used for the treatment of ischemic heart disease or peripheral circulatory disturbance. Ketanserin is therefore not preferred.

In addition, several compounds, including sarpogrelate, are known to have serotonin-2 receptor antagonistic action. They are however accompanied with problems in the potency, the selectivity to other receptors, toxicity, side effects. Thus, there remains still much room for improvements.

### Disclosure of the Invention

In view of the foregoing circumstances, the present inventors synthesized various compounds and investigated their pharmacological effects. As a result, it has been found that specific benzothiazine derivatives have strong serotonin-2 receptor antagonistic action, are excellent in the selectivity to a serotonin-2 receptor in the antagonistic action against various receptors, particularly in the selectivity to a serotonin-2 receptor in the antagonistic action against α₁ receptors, and have low toxicity, leading to the completion of the present invention.

The present invention has been completed based on the above described findings. A first object of the present invention is to provide a benzothiazine derivative represented by the following formula (I): 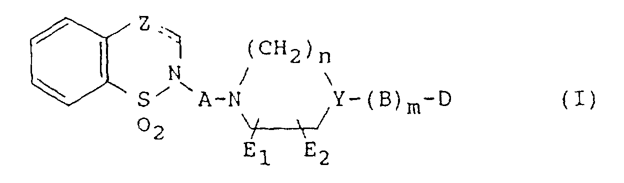 wherein the dashed line indicates the presence or absence of a bond, and
when the dashed line indicates the presence of the bond, Z represents one of the following groups: 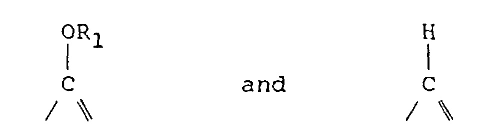 in which R₁ represents a substituted or unsubstituted alkyl group or a substituted or unsubstituted aralkyl group but, when the dashed line indicates the absence of the bond, Z represents one of the following groups: 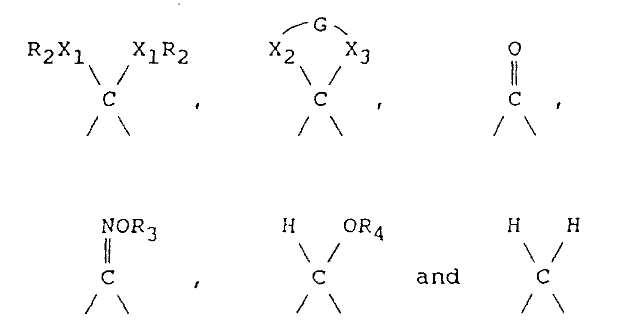 in which R₂ represents a substituted or unsubstituted alkyl group, a substituted or unsubstituted aryl group or a substituted or unsubstituted aralkyl group, R₃ represents a hydrogen atom, a substituted or unsubstituted alkyl group, a substituted or unsubstituted aryl group or a substituted or unsubstituted aralkyl group, R₄ represents a hydrogen atom, a substituted or unsubstituted alkyl group or a substituted or unsubstituted aralkyl group, X₁, X₂ and X₃ each independently represents an oxygen atom or a sulfur atom, G represents an ethylene group,

A represents a C₂-C₁₀ alkylene group, a C₄-C₁₀ alkenylene group or a C₄-C₁₀ alkynylene group, all of which may be branched or linear and may be substituted by one or more halogen atoms.

Y represents CH, or a nitrogen atom and when Y represents CH, m stands for 0 or 1, n stands for 1 or 2, B represents a carbonyl group
but, when Y represents a nitrogen atom, m stands fro 0 or 1, n stands for 2 or 3, B represents a group -CHR₇- in which R₇ represents an alkyl group, a C₁-C₁₄ aryl group, which may be unsubstituted or substituted by one or more halogen atoms, C₁-C₄-alkyl groups and/or C₁-C₄ alkoxy groups.

E₁ and E₂ each independently represents a hydrogen atom or a C₁-C₄ alkyl group and,

D represents a C₆₋₂₈ aromatic hydrocarbon group or a aromatic heterocyclic group, which is monocyclic or bicyclic and contains three or less oxygen, sulfur and/or nitrogen atoms, whereby the aromatic hydrocarbon or aromatic heterocyclic group may be substituted with halogen atoms, C₁₋₄ alkyl groups, C₁₋₄ alkoxyl groups, C₆₋₁₄ aryl groups, C₇₋₂₂ aralkyl groups, C₇₋₂₂ aralkyloxy groups, cyano groups; nitro groups; carboxyl groups; alkoxycarbonyl groups having a C₁₋₆ alcohol moiety; lower alkylsulfonylamino groups having a C₁₋₄ alkyl moiety; carbamoyl groups; and hydroxyl groups; wherein the substituents for the C₁₋₄ alkyl groups, the C₇₋₂₂ aralkyl groups and C₆₋₁₄ aryl groups are halogen atoms, C₁₋₄ alkyl groups and/or C₁₋₄ alkoxy groups, or a salt thereof, with the proviso that when
E₁ and E₂ represent hydrogen
n has a value of 2
Y is CH
B is methylene
m has a value of 1
Z is carbonyl,
A is an unsubstituted C₂ or C₃ alkylene bridge
D does not represent 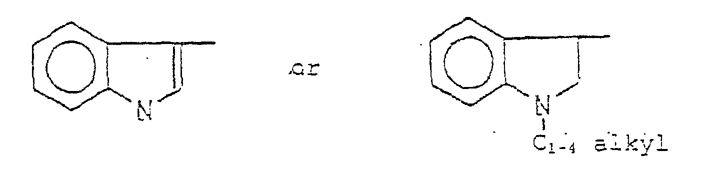
wherein the benzyl ring is unsubstituted or substituted with halogen.

Another object of the present invention is to provide a preparation process of the benzothiazine derivative (I) or its salt.

A further object of the present invention is to provide a serotonin-2 receptor antagonist as a treating agent for circulatory diseases, said antagonist containing the benzothiazine derivative (I) or its pharmaceutically-acceptable salt as an effective ingredient.

### Best Modes for Carrying out the Invention

In the benzothiazine derivatives (I) of the present invention, R₁ means branched or linear C₁₋₄ alkyl groups such as methyl and ethyl which may be substituted by one or more of halogen atoms such as fluorine, chlorine and bromine and/or C₁₋₄ alkoxy groups such as methoxy and ethoxy and C₇₋₂₂ aralkyl groups such as benzyl and phenethyl, which may be substituted by one or more of halogen atoms such as fluorine, chlorine and bromine; C₁₋₄ alkyl groups such as methyl and ethyl; and/or C₁₋₄ alkoxy groups such as methoxy and ethoxy.

R₂ means branched or linear C₁₋₄ alkyl groups such as methyl and ethyl which may be substituted by one or more of halogen atoms such as fluorine, chlorine and bromine and/or C₁₋₄ alkoxy groups such as methoxy and ethoxy C₆₋₁₄ aryl groups such as phenyl and naphthyl and C₇₋₂₂ aralkyl groups such as benzyl and phenethyl, each of which may be substituted by one or more of halogen atoms such as fluorine, chlorine and bromine;
C₁₋₄ alkyl groups such as methyl and ethyl; and/or C₁₋₄ alkoxy groups such as methoxy and ethoxy. In this case, preferred examples of group R₂X₁ - include methoxy, methylthio, ethoxy and ethylthio groups.

Preferred examples of the group: 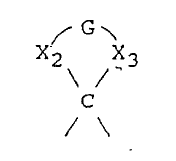 includes groups represented by the following formulas: 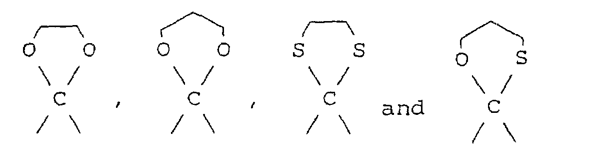 in which one or more of the hydrogen atoms may be substituted by a corresponding number of halogen atoms such as fluorine, chlorine and bromine; alkyl groups, preferably C₁₋₄ alkyl groups such as methyl and ethyl; aryl groups, preferably C₆₋₁₄ aryl groups such as phenyl and naphthyl; aralkyl groups, preferably C₇₋₂₂ aralkyl groups such as benzyl and phenethyl; and/or alkylidene groups, preferably C₁₋₄ alkylidene groups such as methylidene and ethylidene.

R₃ of group NOR₃ means a hydrogen atom, branched or linear C₁₋₄ alkyl groups such as methyl and ethyl, which may be substituted by one or more of halogen atoms such as fluorine, chlorine and bromine and/or C₁₋₄ alkoxy groups such as methoxy and ethoxy, C₆₋₁₄ aryl groups such as phenyl and naphthyl and C₇₋₂₂ aralkyl groups such as benzyl and phenethyl. The exemplified groups may be substituted by one or more of halogen atoms such as fluorine, chlorine and bromine; C₁₋₄ alkyl groups such as methyl and ethyl; and/or preferably, C₁₋₄ alkoxy groups such as methoxy and ethoxy.

R₄ means a hydrogen atom, branched or linear C₁₋₄ alkyl groups such as methyl and ethyl and C₇₋₂₂ aralkyl groups such as benzyl and phenethyl. Each of the exemplified groups may be substituted by one or more of halogen atoms such as fluorine, chlorine and bromine; C₁₋₄ alkyl groups such as methyl and ethyl; and/or C₁₋₄ alkoxy groups such as methoxy and ethoxy.

Preferred examples of group Z are

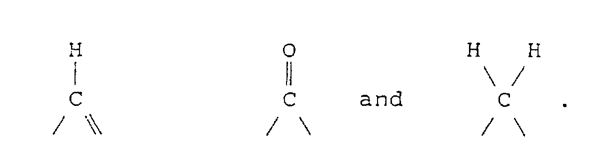

Specifically preferred examples of group Z are 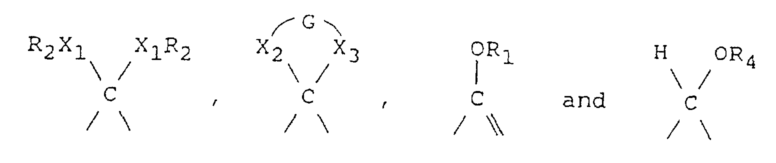 wherein G, R₁, R₂, R₄, X₁, X₂ and X₃ have the same meanings as defined above.

A means branched or linear C₂₋₁₀ alkylene groups such as ethylene, trimethylene, tetramethylene, pentamethylene and octamethylene, branched or linear C₄₋₁₀ alkenylene groups such as 2-butenylene and 3-pentenylene, and branched or linear C₄₋₁₀ alkynylene groups such as 2-butynylene and 3-pentynylene. Each of the exemplified group may be substituted by one or more of halogen atoms such as fluorine, chlorine and bromine. Among them, ethylene, trimethylene and tetramethylene groups are particularly preferred.

The following group: 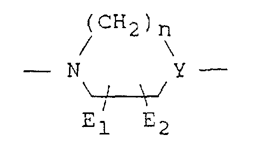 wherein E₁, E₂, Y and n have the same meanings as defined above, is a heterocyclic group led by a pyrrolidine, piperidine, piperazine or homopiperazine group, in which two or less hydrogen atoms on the ring may be substituted by C₁₋₄ alkyl groups such as methyl or ethyl.
When the group of the above formula is a heterocyclic group led by a pyrrolidine or piperidine group, preferably a piperidine group, m stands for 0 or 1, B represents a carbonyl group.

When the heterocyclic group is a group led by a piperazine or homopiperazine group, preferably a piperazine group, m stands for 0 or 1 (preferably 0), B represents a carbonyl group, or a group -CHR₇- (in which R₇ represents a C₆₋₁₄ aryl group such as phenyl and naphthyl).

Further, R₇ may be substituted, by one or more of halogen atoms such as fluorine, chlorine and bromine, C₁₋₄ alkyl groups such as methyl and ethyl, and/or, preferably C₁₋₄ alkoxy groups such as methoxy and ethoxy.

Among those exemplified above as B, preferred are substituted or unsubstituted phenylmethylene groups.

D means C₆₋₂₈ aromatic hydrocarbon groups such as a phenyl group in which one or more of the hydrogen atoms may be substituted and a naphthyl group in which one or more of the hydrogen atoms may be substituted. Other preferred examples of D include aromatic heterocyclic groups, preferably those each of which is monocyclic or dicyclic and contains three or less oxygen, sulfur and/or nitrogen atoms - such as pyridyl, pyrimidyl, benzisothiazolyl, benzisoxazolyl, indazolyl and indolyl groups in which one or more of hydrogen atoms may be substituted.

Substituents for the above aromatic hydrocarbon or aromatic heterocyclic group are halogen atoms such as fluorine, chlorine and bromine;
alkyl groups, preferably C₁₋₄ alkyl groups such as methyl and ethyl; alkoxyl groups, preferably C₁₋₄ alkoxyl groups such as methoxy and ethoxy; aryl groups, preferably C₆₋₁₄ aryl groups such as phenyl and naphthyl; aralkyl groups, preferably C₇₋₂₂ aralkyl groups, such as benzyl and phenethyl; aralkyloxy groups, preferably C₇₋₂₂ aralkyloxy groups such as benzyloxy; cyano groups; nitro groups; carboxyl groups; alkoxycarbonyl groups (having preferably a C₁₋₆ alcohol moiety); lower alkylsulfonylamino groups (having preferably a C₁₋₄ alkyl moiety); carbamoyl groups; and hydroxyl groups.

Preferred examples of group D among these examples include phenyl groups which have been unsubstituted or substituted by one or more of halogen atoms, alkoxy groups and/or hydroxyl groups; benzisothiazolyl groups which have been unsubstituted or substituted by one or more halogen atoms; benzisoxazolyl groups which have been unsubstituted or substituted by one or more halogen atoms; and indazolyl groups which have been unsubstituted or substituted by one or more halogen atoms. Particularly preferred are phenyl groups which have been unsubstituted or substituted by one or more of fluorine atoms, methoxy groups and/or hydroxyl groups.

Many of the compounds (I) according to the present invention have isomers. It is to be noted that these isomers and mixtures thereof are all embraced by the present invention.

Various processes can be employed for the preparation of the benzothiazine derivatives (I) according to the present invention. It is however preferred to prepare the benzothiazine derivatives, for example, by any one of the following processes.

### Process 1:

Among the benzothiazine derivatives (I), each of the compounds (Ib) in which Z is represented by a group: 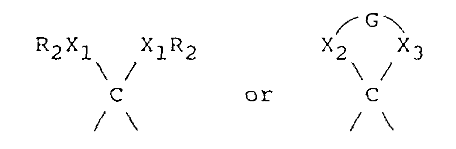 can be synthesized in accordance with any of the processes shown by the following schemes.

(a) The intended compound can be obtained, in accordance with the following reaction scheme, by reacting the compound represented by formula (XXII) with the compound represented by formula (III) to convert the former compound into the compound represented by formula (XXIII) and then reacting the compound (XXIII) with a nitrogen-containing compound represented by formula (V) or a salt thereof. 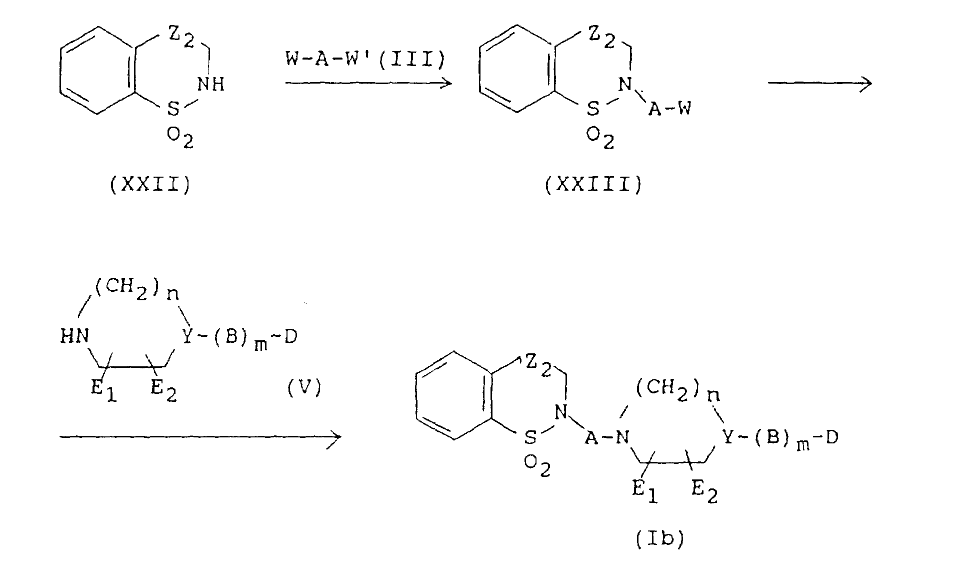 wherein A, B, D, E₁, E₂, Y, m and n have the same meanings as defined above; Z₂ represents one of the following groups: 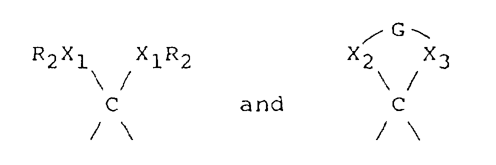 in which G, R₂, X₁, X₂ and X₃ have the same meanings as defined above; and W and W' may be the same or different and individually represents a substituent easily replaceable with an amino group.

In the above reaction, the conversion from the compound (XXII) to the compound (XXIII) can be effected by treating the compound (XXII) with an organic or inorganic base and then causing the compound (III) to act on the compound (XXII), or by causing the compound (III) to act on the compound (XXII) in the presence of such a base.

Examples of group W or W' of the compound (III), which is an eliminative substituent and easily replaceable with an amino group, include halogen atoms such as chlorine and bromine, alkylsulfonyloxy groups such as methanesulfonyloxy and arylsulfonyloxy groups such as p-toluenesulfonyloxy.

Exemplary organic or inorganic bases include sodium hydride, potassium hydride, sodium carbonate, potassium carbonate, triethylamine and potassium t-butoxide. Further, illustrative solvents useful for the above reaction include tetrahydrofuran, dioxane, dimethylformamide, dimethylsulfoxide, acetonitrile, N-methylpyrrolidone, acetone, 2-butanone and toluene. The reaction is conducted at -78°C to reflux temperature.

To prepare the compound (Ib) by reacting the compound (XXIII) with the nitrogen-containing compound (V), it is only necessary to react the nitrogen-containing compound (V) or an organic acid or inorganic acid salt thereof with the compound (XXIII), optionally together with an organic base such as triethylamine, pyridine, collidine, 1,8-diazabicyclo[5.4.0]undec-7-en (DBU) or potassium t-butoxide or an inorganic base such as potassium carbonate, sodium carbonate, sodium hydrogencarbonate, sodium hydroxide or sodium hydride, optionally after adding an alkali iodide such as potassium iodide or sodium iodide, at 0°C to 150°C in the solvent exemplified above or a solvent such as methanol, ethanol, propanol or butanol.

Examples of the nitrogen-containing compound (V) include 1-phenylpiperazine, 1-(2-fluorophenyl)piperazine, 1-(3-fluorophenyl)piperazine, 1-(4-fluorophenyl)-piperazine, 1-(4-hydroxyphenyl)piperazine, 1-(2-chlorophenyl)piperazine, 1-(3-chlorophenyl)piperazine, 1-(4-chlorophenyl)piperazine, 1-(2-methoxyphenyl)piperazine, 1-(3-methoxyphenyl)piperazine, 1-(4-methoxyphenyl)-piperazine, 1-(4-methanesulfonamidophenyl)piperazine, 1-(4-cyanophenyl)piperazine, 1-(4-carbamoylphenyl)-piperazine, 1-(4-methoxycarbonylphenyl)piperazine, 1-(2-pyridyl)piperazine, 1-(2-pyrimidyl)piperazine, 1-benzylpiperazine, 1-diphenylmethylpiperazine, 1-cinnamylpiperazine, 1-benzoylpiperazine, 1-(4-benzyloxybenzoyl)piperazine, 1-(4-hydroxybenzoyl)piperazine, 1-(2-furoyl)piperazine, 1-(1,2-benzisoxazol-3-yl)-piperazine, 1-(1,2-benzisothiazol-3-yl)piperazine, 4-phenylpiperidine, 4-benzylpiperidine, α,α-bis(4-fluorophenyl)-4-piperidinemethanol, 4-(4-fluorobenzoyl)piperidine, 4-benzoylpiperidine, 4-(4-methoxybenzoyl)piperidine, 4-(4-chlorobenzoyl)piperidine, 3-(4-fluorobenzoyl)piperidine, 4-(6-fluoro-1,2-benzisoxazol-3-yl)piperidine, 4-(6-fluoro-1,2-benzisothiazol-3-yl)piperidine, 4-(6-fluoro-1H-indazol-3-yl)-piperidine, 3-benzoylpyrrolidine, 3-(4-fluorobenzoyl)-pyrrolidine, 4-(4-fluorophenoxy)piperidine, 4-[(4-fluorophenyl)thio]piperidine, 4-[(4-fluorophenyl)-sulfinyl]piperidine, 4-[(4-fluorophenyl)sulfonyl]-piperidine, 4-[bis(4-fluorophenyl)methylene]piperidine and 4-(4-fluorobenzoyl)piperidine ethylene acetal. They are all either known compounds or compounds which can be readily prepared by a known process or a process similar to the known process.

Incidentally, among the compounds (XXII) employed as starting materials in the above reaction, the compounds except those in which Z₂ is a group represented by the following formula: 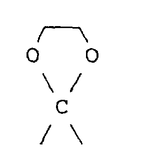 are novel compounds. These novel compounds can each be prepared from a known compound (XV) by any one of the various processes. For example, it is possible to select a suitable method among those described in "Protective Groups in Organic Synthesis" (written by T.W. Greene; John Wiley & Sons, Inc.) and the like. The typical example includes a process in which R₂X₁H or HX₂-G-X₃H is, as described below, caused to act on the compound (XV) in the presence of an acid. 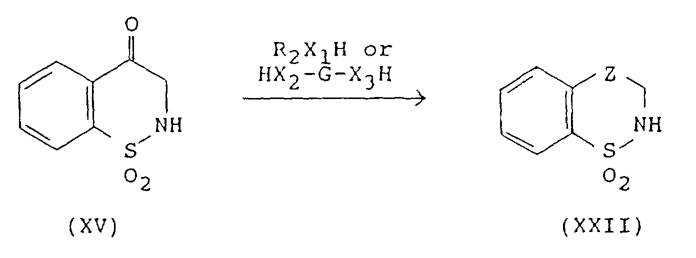 wherein G, R₂, X₁, X₂, X₃ and Z₂ have the same meanings as defined above.

(b) The intended compound can be obtained by causing the nitrogen-containing compound (VI) or a salt thereof to act on the compound represented by the formula (XXII) in accordance with the following reaction scheme: 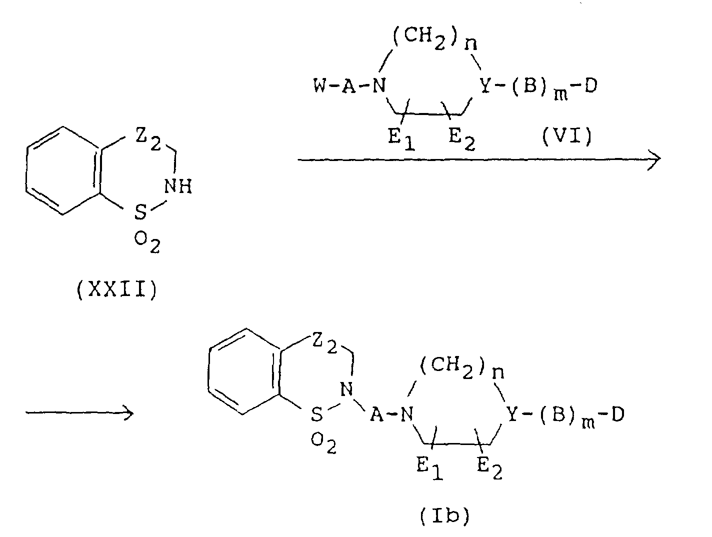 wherein A, B, D, E₁, E₂, W, Y, Z₂, m and n have the same meanings as defined above.

The conversion from the compound (XXII) to the compound (Ib) can be conducted by treating the compound (XXII) with an organic or inorganic base and then causing the compound (VI) to act on the resulting compound, or causing the compound (VI) to act on the compound (XXII) in the presence of the inorganic or organic base. The conversion from the compound can be effected under the conditions similar to those shown in the conversion from the compound (XXII) to the compound (XXIII) in Process 1(a). In this case, it is also possible to add an alkali iodide such as potassium iodide or sodium iodide at need. Incidentally, the compound (VI) can be synthesized by reacting the compound (V) with the compound (III) in a manner known *per se* in the art.

### Process 2:

Among the benzothiazine derivatives (I), each of compounds (Ic) in which Z is represented by a group: 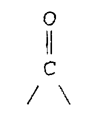 can be synthesized in any of the following processes.

(a) The intended compound can be obtained, in accordance with the following scheme, by converting the compound (XV) or (XXIII) to the compound (VIII) and then reacting the compound (VIII) with the compound represented by the formula (V): 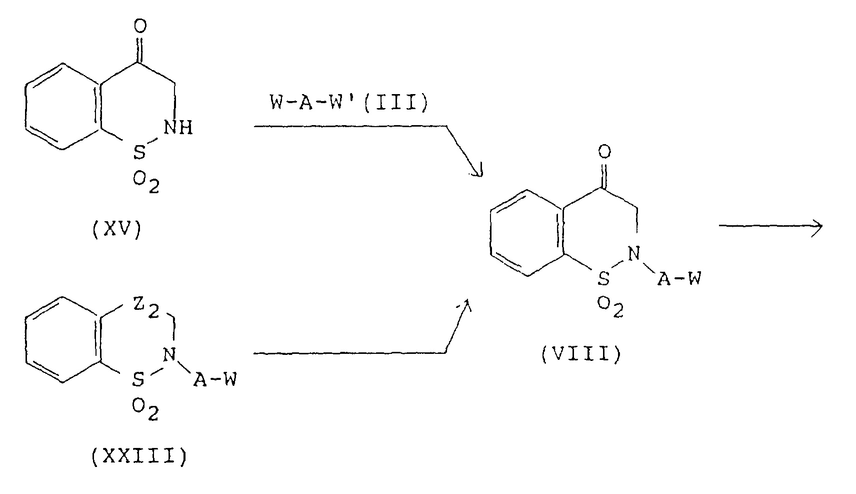 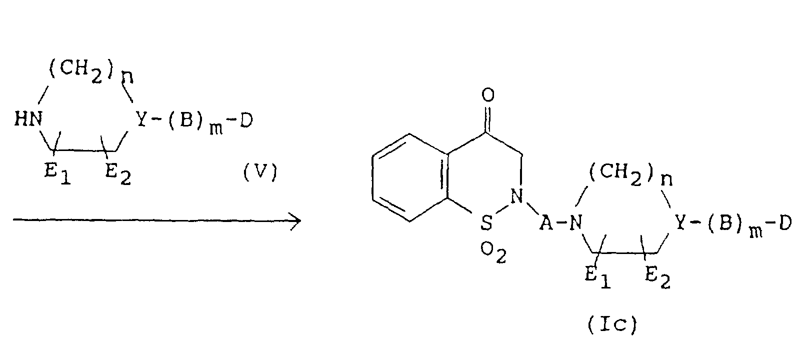 wherein A, B, D, E₁, E₂, W, W', Y, Z₂, m and n have the same meanings as defined above.

The conversion from the compound (XV) to the compound (VIII) can be effected under the conditions similar to those shown in the conversion from the compound (XXII) to the compound (XXIII) in Process 1(a). Alternatively, the conversion from the compound (XXIII) to the compound (VIII) can be effected employing a method described in "Protective Groups in Organic Synthesis" (written by T.W. Greene; John Wiley & Sons, Inc.) and the like. For instance, the conversion to the intended compound (VIII) can be conducted by acid treatment of the compound (XXIII) when in Z₂, X₁ represents an oxygen atom or X₂ and X₃ both represent an oxygen atom, or by the treatment with mercury (II) chloride when also in Z₂, X₁ represents a sulfur atom or X₂ and X₃ both represent a sulfur atom.

The conversion from the compound (VIII) to the compound (Ic) can be effected under the conditions similar to those shown in the conversion from the compound (XXIII) to the compound (Ib) in Process 1(a).

(b) The intended compound can be obtained by the replacement of the group Z₂ of the compound (Ib) with a carbonyl group in accordance with the following reaction scheme. 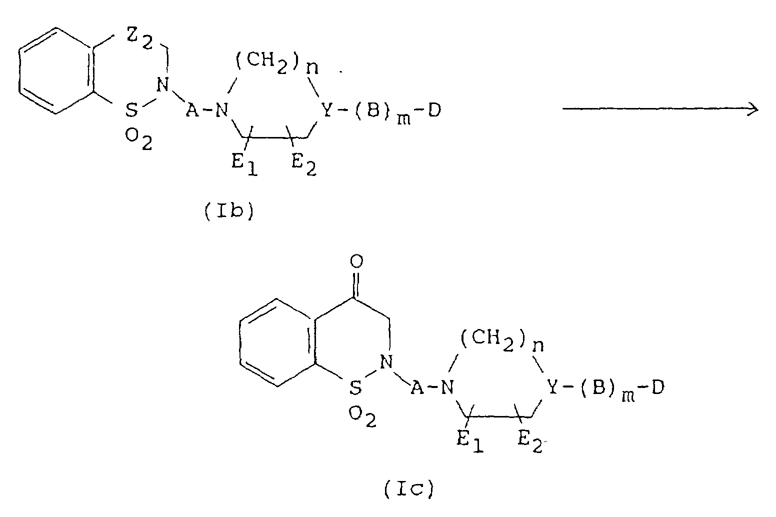 wherein A, B, D, E₁, E₂, Y, Z₂, m and n have the same meanings as defined above.

The conversion from the compound (Ib) to the compound (Ic) can be effected under the conditions similar to those shown in the conversion from the compound (XXIII) to the compound (VIII) in Process 2(a).

### Process 3:

Among the benzothiazine derivatives (I), each of the compounds (Ig) and (Ie) in which Z is represented by a group: 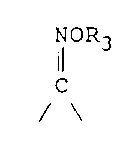 can be synthesized in accordance with any of the following processes. Where there is a group reactive to a hydroxylamine or a derivative thereof (VII) or a salt of the hydroxylamine or the derivative in a nitrogen-containing compound (V), it is desired to choose process (a).

(a) Each compound (Ig) can be obtained, in accordance with the following reaction scheme, by causing a hydroxylamine or a derivative thereof represented by the formula (VII) or a salt of the hydroxylamine or the derivative to act on the compound represented by the formula (VIII) and then causing the nitrogen-containing compound (V) to act further. 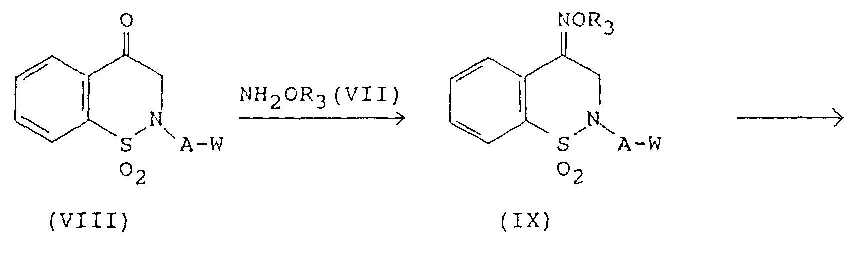 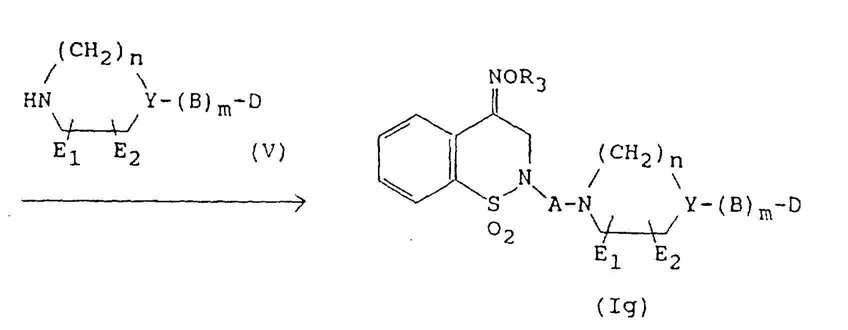 wherein A, B, D, E₁, E₂, R₃, W, Y, m and n have the same meanings as defined above.

The reaction between the compound (VIII) and the hydroxylamine or its derivative (VII) can be practiced, if necessary, in the presence of an organic base such as pyridine, triethylamine, collidine, DBU or sodium acetate or an inorganic base such as potassium carbonate or sodium hydroxide. The hydroxylamine or its derivative (VII) may also be used in the form of an organic acid salt or an inorganic acid salt.

The reaction is conducted at 0°C to reflux temperature, preferably 0°C to 100°C optionally in a suitable solvent such as methanol, ethanol, propanol, tetrahydrofuran, dimethylformamide or dimethylsulfoxide.

The conversion from the resulting compound (IX) to the compound (Ig) can be effected under the conditions similar to those shown in the conversion from the compound (XXIII) to the compound (Ib) in Process 1(a).

(b) Each compound (Ie) can be obtained, in accordance with the following reaction scheme, by causing a hydroxylamine or a derivative thereof (VII) or a salt of the hydroxylamine or the derivative to act on the compound (Id): 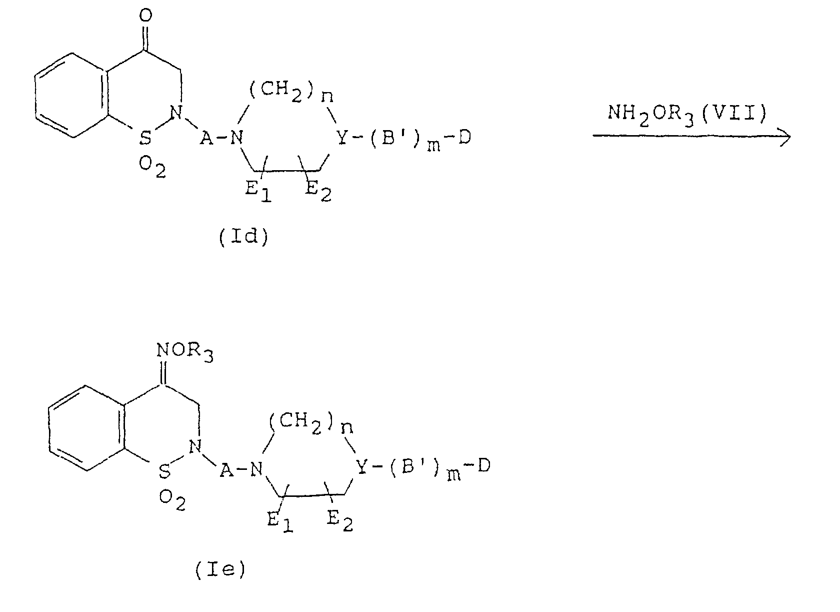 B' has the same meaning as B and A, D, E₁, E₂, R₃, Y, m and n have the same meanings as defined above.

The conversion from the compound (Id) to the compound (Ie) can be effected under the conditions similar to those shown in the conversion from the compound (VIII) to the compound (IX) in Process 3(a).

### Process 4:

Among the benzothiazine derivatives (I), each of compounds (Ih) and (If) in which Z is represented by a group: 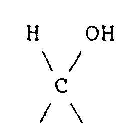 can be synthesized by any one of the following processes.

Incidentally, it is desired to select process (a) when there is a group reactive with a reducing agent in a nitrogen-containing compound (V).

(a) Each compound (In) can be obtained by reducing the compound represented by the formula (VIII) to obtain the compound (X) and then causing the nitrogen-containing compound (V) to act on the resulting compound. 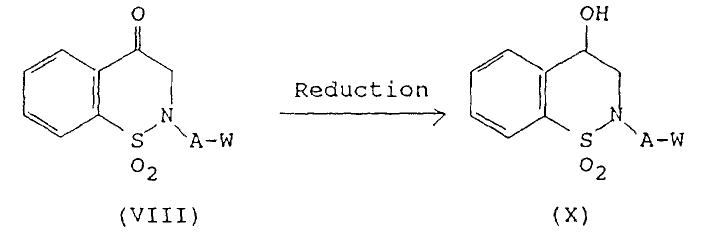 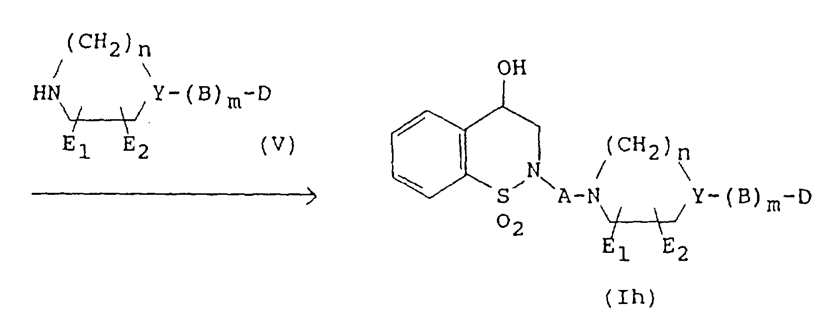 wherein A, B, D, E₁, E₂, W, Y, m and n have the same meanings as defined above.

The conversion from the compound (VIII) to the compound (X) can be effected by treating the compound represented by the formula (VIII) with a reducing agent such as sodium borohydride, potassium borohydride, sodium cyanoborohydride or tri-n-tin hydride in an ordinarily-employed solvent at -78°C to reflux temperature, preferably -20°C to room temperature.

Further, the conversion from the compound (X) to the compound (Ih) can be effected under conditions similar to those shown in the conversion from the compound (XXIII) to the compound (Ib) in Process 1(a).

(b) Each compound (If) can be obtained by reducing the compound (Id) in accordance with the following reaction scheme: 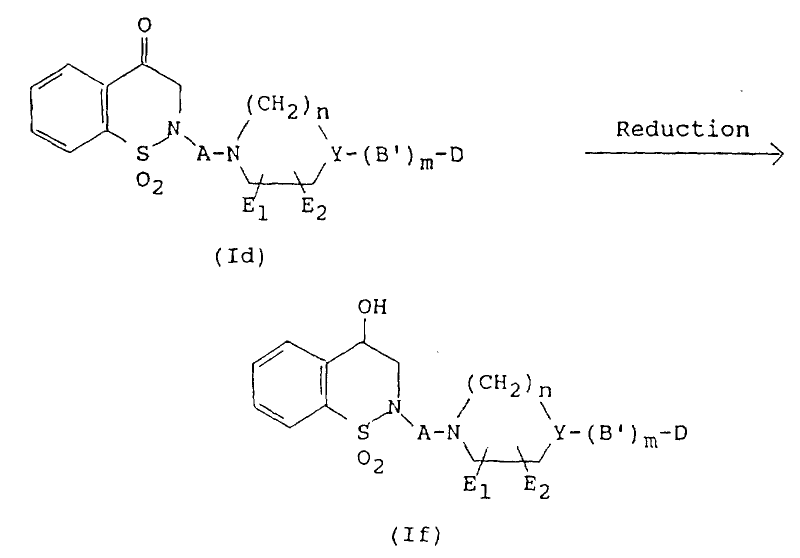 wherein A, B', D, E₁, E₂, Y, m and n have the same meanings as defined above.

The conversion from the compound (Id) to the compound (If) can be effected under conditions similar to those shown in the conversion from the compound (VIII) to the compound (X) in accordance with Process 4(a).

### Process 5:

Among the benzothiazine derivatives (I), each compound (I1) in which Z is represented by a group: 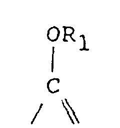 can be synthesized in accordance with the process which will be described hereinafter.

The intended compound can be obtained, in accordance with the following reaction scheme, by reacting the compound represented by the formula (XV) with the compound represented by the formula (XVI) to obtain the compound represented by the formula (XVII), reacting the resulting compound with the compound represented by the formula (III) to obtain the compound represented by the formula (XXIV), and then causing a nitrogen-containing compound represented by the formula (V) to act on the compound (XXIV). 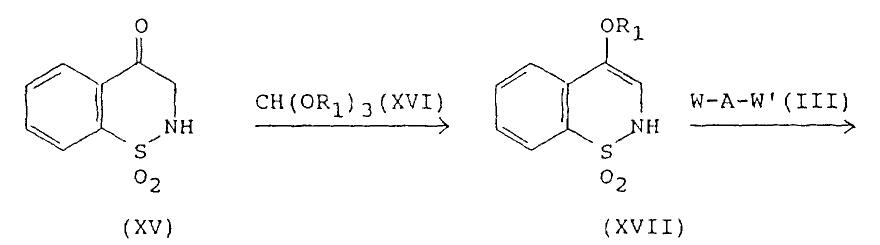 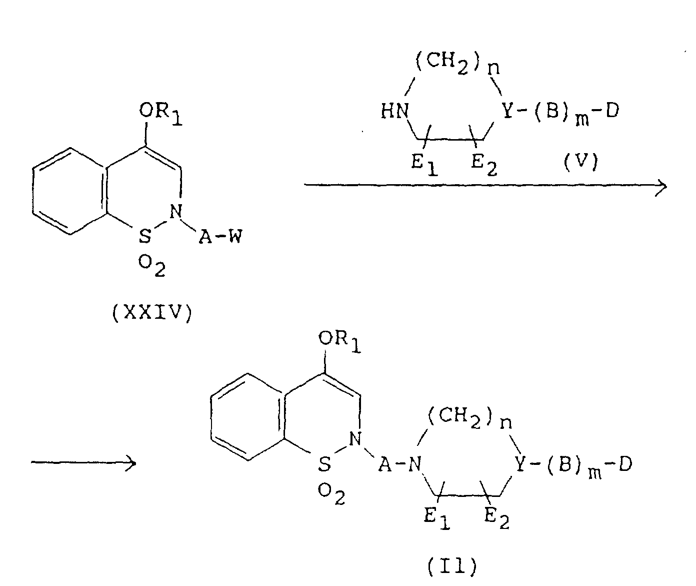 wherein A, B, D, E₁, E₂, R₁, W, W', Y, m and n have the same meanings as defined above.

In the above reaction, the conversion from the compound (XV) to the compound (XVII) can be effected by causing the compound (XVI) to act on the compound (XV) in the presence of p-toluenesulfonic acid, boron trifluoride·ether complex, Amberlite 15 or the like.

Examples of the solvent usable in the above reaction may include methanol, ethanol, propanol and butanol. The reaction is conducted at -78°C to reflux temperature.

The conversion from the compound (XVII) to the compound (I1) can be effected under conditions similar to those shown in the conversion from the compound (XXII) to the compound (Ib) in Process 1(a).

### Process 6:

Among the benzothiazine derivatives (I), each compound (Ii) in which Z is represented by a group: 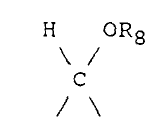 can be synthesized in accordance with the process which will be described hereinafter.

The compound represented by the formula (Ii) can be obtained, in accordance with the reaction scheme shown below, (1) by reducing the compound represented by the formula (XXV) to the compound represented by the formula (XX) and reacting the resulting compound with the compound represented by the formula (III) to obtain the compound (XII), or (2) by reacting the compound represented by the formula (X) with the compound represented by the formula (XI) to obtain the compound (XII), and then reacting the resulting compound (XII) with a nitrogen-containing compound represented by the formula (V).

In this case, it is desired to select a suitable process from the processes (1) and (2) according to the kind of group R₈. 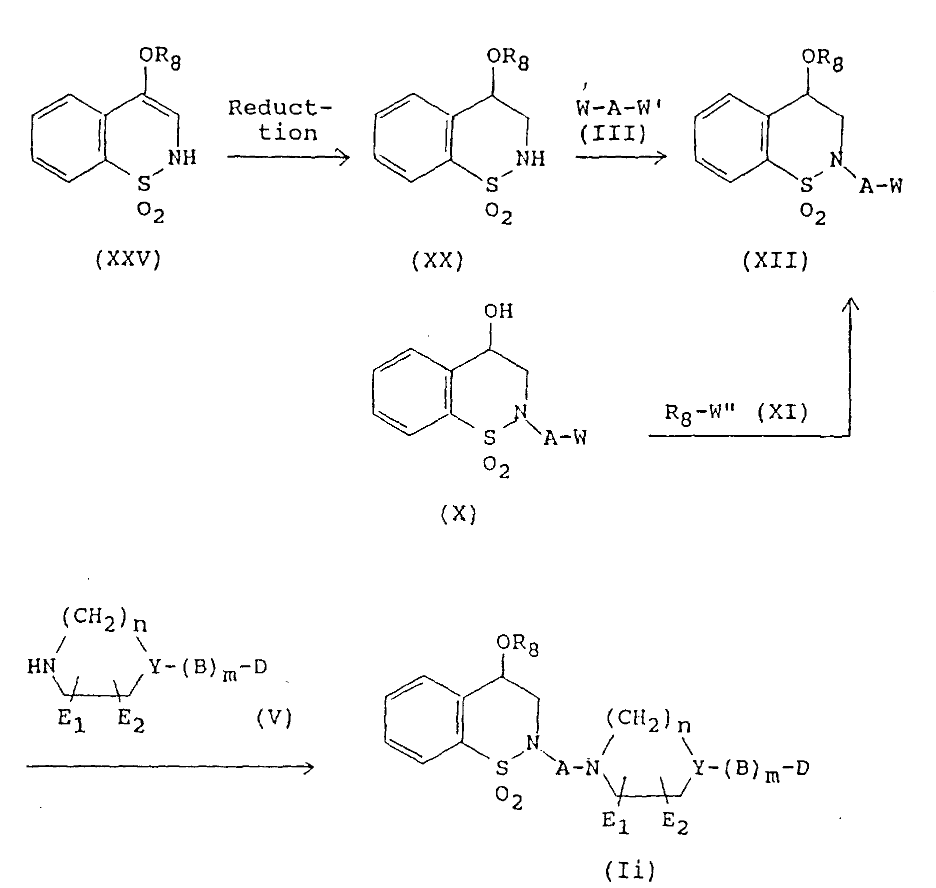 wherein A, B, D, E₁, E₂, R₈, W, W', W", Y, m and n have the same meanings as defined above.

In the above reaction, the conversion from the compound (XXV) to the compound (XX) can be conducted by treating, in the presence of a catalyst such as palladium-carbon or platinum, the compound (XXV) with hydrogen gas in an ordinarily-employed solvent at -78°C to reflux temperature, preferably at room temperature. The conversion from the compound (XX) to the compound (XII) can be effected under conditions similar to those shown in the conversion from the compound (XXII) to the compound (XXIII) in Process 1(a).

The conversion from the compound (X) to the compound (XII) can be conducted by treating the compound (X) with an inorganic or organic base and then, causing the compound (XI) to act on the resulting compound, or causing the compound (XI) to act on the compound (X) in the presence of such a base.

Examples of group W" of the compound (XI), which is an eliminative substituent, include halogen atoms such as chlorine and bromine, alkylsulfonyloxy groups such as methanesulfonyloxy and arylsulfonyloxy groups such as p-toluenesulfonyloxy.

Further, exemplary inorganic or organic bases usable in the above reaction include sodium hydride, sodium bis(trimethylsilyl)amide, lithium diisopropylamide and potassium t-butoxide. Illustrative solvents usable in the present reaction include, tetrahydrofuran, dioxane, dimethylformamide, dimethylsulfoxide, N-methylpyrrolidone and toluene. The reaction may be conducted at -78°C to reflux temperature.

The conversion from the compound (XII) to the compound (Ii) can be effected under conditions similar to those shown in the conversion from the compound (XXIII) to the compound (Ib) in Process 1(a).

### Process 7:

Among the benzothiazine derivatives (I), each compound (Ij) in which Z is represented by a group: 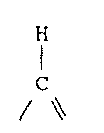 can be synthesized in accordance with the following process.

The compound represented by the formula (Ij) can be obtained, in accordance with the following reaction scheme, by subjecting the compound represented by formula (X) to dehydration to convert it into the compound represented by formula (XIII) and then causing a nitrogen-containing compound represented by formula (V) to act on the resulting compound. 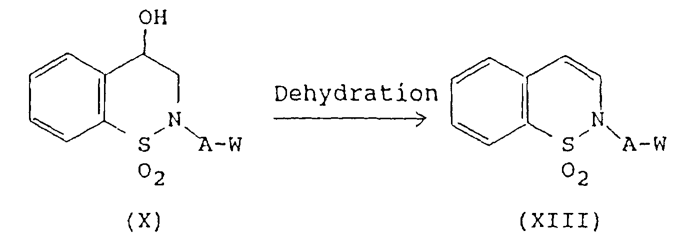 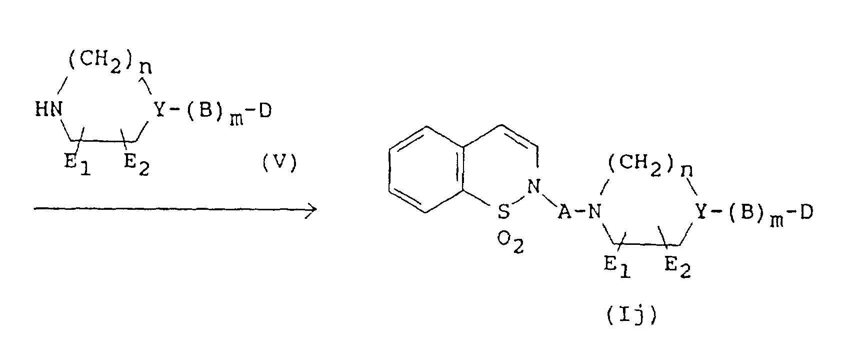 wherein A, B, D, E₁, E₂, W, Y, m and n have the same meanings as defined above.

In the above reaction, the conversion from the compound (X) to the compound (XIII) can be effected by causing methanesulfonyl chloride or p-toluenesulfonyl chloride and a base such as triethylamine, pyridine or collidine to act on the compound (X) in a solvent such as dichloromethane, chloroform or toluene and then treating the resulting product with the base described above or silica gel at room temperature to reflux temperature.

The conversion from the compound (XIII) to the compound (Ij) can be effected under conditions similar to those shown in the conversion from the compound (XXIII) to the compound (Ib) in Process 1(a).

### Process 8:

Among the benzothiazine derivatives (I), each compound (Ik) in which Z is represented by a group: 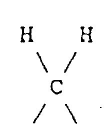 can be synthesized in accordance with the following process.

The compound represented by the formula (Ik) can be obtained, in accordance with the following reaction scheme, by reducing the compound represented by formula (XIII) to convert it into the compound represented by formula (XIV) and then reacting the resulting compound with a nitrogen-containing compound represented by formula (V). 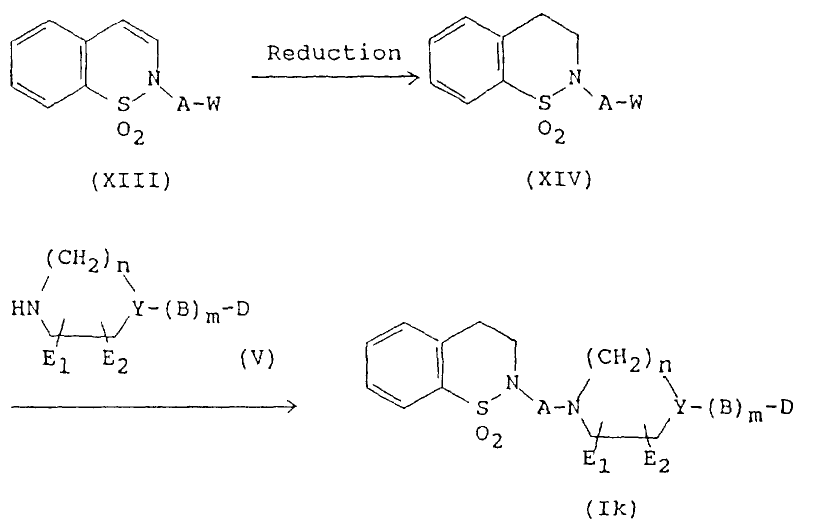 wherein A, B, D, E₁, E₂, W, Y, m and n have the same meanings as defined above.

In the above reaction, the conversion from the compound (XIII) to the compound (XIV) can be effected under conditions similar to those shown in the conversion from the compound (XXV) to the compound (XX) in Process 6.

The conversion from the compound (XIV) to the compound (Ik) can be effected under conditions similar to those shown in the conversion from the compound (XXIII) to the compound (Ib) in Process 1(a).

If necessary, the compounds (I) of the present invention obtained according to the above-described processes can each be reacted with one of various acids to convert the compound to its salt. Then, the resulting salt can be purified by a method such as recrystallization or column chromatography.

Exemplary acids usable to convert the benzothiazine derivatives (I) to their salts include inorganic acids such as hydrochloric acid, nitric acid, sulfuric acid, phosphoric acid and hydrobromic acid; and organic acids such as maleic acid, fumaric acid, tartaric acid, lactic acid, citric acid, acetic acid, methanesulfonic acid, p-toluenesulfonic acid, adipic acid, palmitic acid and tannic acid.

As will be demonstrated later by test, the benzothiazine derivatives (I) and their salts according to the present invention, which can be obtained as described above, have a strong serotonin-2 blocking action and in addition, they have excellent selectivity of the serotonin-2 blocking action against α₁ blocking action. Further, as a result of a toxicity test, they have been found to feature high safety. The compounds according to the present invention can therefore be used as therapeutics for circulatory diseases such as ischemic heart diseases, cerebrovascular disturbance and peripheral circulatory disturbance.

When the benzothiazine derivative (I) according to this invention are used as drugs, they can be administered in an effective dose as they are. As an alternative, they can also be formulated into various preparation forms by known methods and then administered.

Exemplary preparation forms as drugs include orally administrable preparation forms such as tablets, powders, granules, capsules and syrups as well as parenterally administrable preparation forms such as injections and suppositories. Whichever preparation form is used, a known liquid or solid extender or carrier usable for the formulation of the preparation form can be employed.

Examples of such extender or carrier include polyvinylpyrrolidone, arabic gum, gelatin, sorbit, cyclodextrin, tragacanth gum, magnesium stearate, talc, polyethylene glycol, polyvinyl alcohol, silica, lactose, crystalline cellulose, sugar, starch, calcium phosphate, vegetable oil, carboxymethylcellulose, sodium laurylsulfate, water, ethanol, glycerin, mannitol and syrup, When the compounds (I) according to the present invention are used as drugs, their dose varies depending on the administration purpose, the age, body weight and conditions of the patient to be administered, etc. In oral administration, the daily dose may generally be about 0.01-1,000 mg.

The present invention will next be described in further detail by the following examples and tests. But the present invention is not limited to the following examples and tests.

### Example 1

### Synthesis of 3,4-dihydro-2H-1,2-benzothiazin-4-one 1,1-dioxide trimethylene acetal (Compound No. 1).

A mixture of 2.96 g (15 mmol) of 3,4-dihydro-2H-1,2-benzothiazin-4-one 1,1-dioxide, 5.71 g (75 mmol) of trimethylene glycol, 285 mg (1.5 mmol) of p-toluenesulfonic acid monohydrate and 75 mℓ of toluene was refluxed for 30 hours in a container equipped with a Dean & Stark water separator.

The reaction mixture was cooled and then, 100 mℓ of a 0.02N aqueous solution of sodium hydroxide and ethyl acetate were added to the reaction mixture in this order, followed by fractionation. The resulting organic layer was washed with water and a saturated brine, dried over anhydrous sodium sulfate and then, concentrated under reduced pressure. The oil so obtained was purified by chromatography on a silica gel column in which "No. 9385" (product of Merck & Co., the same silica gel was also used in the subsequent examples) was used as silica gel (eluent: ethyl acetate: hexane = 1:1), whereby 1.01 g of the title compound were obtained (yield: 26%).

### Example 2

### Synthesis of 3,4-dihydro-2H-1,2-benzothiazine-4-spiro-2'-[(4'R,5'R)-dimethyl-1',3'-dioxolan] 1,1-dioxide (Compound No. 2)

A solution of 410 mg (2.08 mmol) of 3,4-dihydro-2H-1,2-benzothiazin-4-one 1,1-dioxide, 517 mg (5.70 mmol) of (2R,3R)-2,3-butanediol and 38 mg (0.2 mmol) of p-toluenesulfonic acid monohydrate in 10 mℓ of benzene was refluxed for 20 hours in a container equipped with a Dean & Stark water separator.

Ethyl acetate was added to the reaction mixture. The resulting organic layer was washed with a saturated aqueous solution of sodium bicarbonate, water and saturated brine, dried over anhydrous magnesium sulfate and then, concentrated under reduced pressure. The residue was purified by chromatography on a silica gel column (eluent: methylene chloride:ethyl acetate = 30:1), whereby 545 mg of the title compound were obtained (yield: 97%).

### Example 3

### Synthesis of 3,4-dihydro-2H-1,2-benzothiazine-4-spiro-2'-(1',3'-oxathiolan) 1,1-dioxide (Compound No. 3)

Under cooling and stirring, 615 µℓ (5 mmol) of boron trifluoride ethyl ether complex were added to a solution of 986 mg (5 mmol) of 3,4-dihydro-2H-1,2-benzothiazin-4-one 1,1-dioxide and 586 mg (7.5 mmol) of β-mercaptoethanol in 20 mℓ of methylene chloride. The reaction mixture was stirred for 27 hours at room temperature. An aqueous solution containing 691 mg (5 mmol) of potassium carbonate were thereafter added to the resulting mixture, followed by extraction with methylene chloride.

The resulting organic layer was washed with water and saturated brine, dried over anhydrous sodium sulfate and then, concentrated under reduced pressure. The residue was purified by chromatography on a silica gel column (eluent: chloroform), whereby 1.00 g of the title compound was obtained (yield 78%).

### Example 4

### Synthesis of 3,4-dihydro-2H-1,2-benzothiazine-4-spiro-2'-(1',3'-dithiolan) 1,1-dioxide (Compound No. 4)

Under ice cooling and stirring, 250 µℓ (2 mmol) of boron trifluoride ethyl ether complex were added to a solution of 1.97 g (10 mmol) of 3,4-dihydro-2H-1,2-benzothiazin-4-one 1,1-dioxide and 1.51 g (16 mmol) of 1,2-ethanedithiol in 38 mℓ of methylene chloride. The reaction mixture was then stirred at room temperature.

Twenty eight hours later, another 250 µℓ (2 mmol) of boron trifluoride ethyl ether complex were added to the reaction mixture, followed by stirring for further 66 hours. The resulting reaction mixture was post-treated as in Example 3. The crude product so obtained was washed with chloroform, whereby 1.88 g of the title compound were obtained (yield: 69%).

### Example 5

### Synthesis of 3,4-dihydro-2H-1,2-benzothiazine-4-spiro-2'-(1',3'-dithian) 1,1-dioxide (Compound No. 5)

Under cooling and stirring, 710 mg (5 mmol) of boron trifluoride ethyl ether complex were added to a suspension of 1.97 g (10 mmol) of 3,4-dihydro-2H-1,2-benzothiazin-4-one 1,1-dioxide and 1.62 g (15 mmol) of 1,3-propanedithiol in 50 mℓ of methylene chloride. The reaction mixture was stirred for 24 hours at room temperature. The crystals so precipitated were collected by filtration, followed by washing with methylene chloride.

The filtrate and the wash liquid were combined together, followed by washing with 100 mℓ of a 1% aqueous solution of potassium carbonate, water and saturated brine, drying over anhydrous sodium sulfate and concentration under reduced pressure. The crude crystals so obtained and the crystals collected by filtration were combined together and recrystallized from acetonitrile, whereby 2.55 g of the title compound were obtained (yield: 89%).

### Example 6

### Synthesis of 4,4-bis(ethylthio)-3,4-dihydro-2H-1,2-benzothiazine 1,1-dioxide (Compound No. 6)

Under cooling and stirring, 50 µℓ (0.4 mmol) of boron trifluoride ethyl ether complex were added to a solution of 197 mg (1 mmol) of 3,4-dihydro-2H-1,2-benzothiazin-4-one 1,1-dioxide and 223 µℓ (3 mmol) of ethanethiol in 5 mℓ of methylene chloride. The reaction mixture was stirred for 1 hour at room temperature. The post-treatment and purification were conducted as in Example 3, whereby 285 mg of the title compound were obtained (yield: 94%).

### Example 7

### Synthesis of 4,4-dimethoxy-3,4-dihydro-2H-1,2-benzothiazine 1,1-dioxide (Compound No. 7)

Under cooling and stirring, 0.75 mℓ (6 mmol) of boron trifluoride ethyl ether complex was added to a solution of 985 mg (5 mmol) of 3,4-dihydro-2H-1,2-benzothiazin-4-one and 10 mℓ (91.4 mmol) of methyl orthoformate in 10 mℓ of methanol, followed by stirring at room temperature. After 85 hours, 0.75 mℓ (6 mmol) of boron trifluoride ethyl ether complex was added further to the reaction mixture, followed by stirring for 20 hours.

Under ice cooling, the reaction mixture was added with 50 mℓ of a saturated aqueous solution of sodium bicarbonate and extracted with ethyl acetate. The organic layer was washed with saturated brine, dried over anhydrous magnesium sulfate and then concentrated under reduced pressure. The residue was purified by chromatography on a silica gel column (eluent: ethyl acetate: methylene chloride = 1:20), whereby 83 mg of the title compound were obtained (yield: 6.8%).

### Example 8

### Synthesis of 4-methoxy-2H-1,2-benzothiazine 1,1-dioxide (Compound No. 8)

A mixture of 1.97 g (10 mmol) of 3,4-dihydro-2H-1,2-benzothiazin-4-one 1,1-dioxide, 500 mg (2.6 mmol) of p-toluenesulfonic acid monohydrate, 20 mℓ of methyl orthoformate and 20 mℓ of methanol was refluxed for 3 hours. The solvent was distilled off and the residue was purified by chromatography on a silica gel column (eluent: methylene chloride:ethyl acetate = 40:1), whereby 2.06 g of the title compound were obtained (yield: 97%).

### Example 9

### Synthesis of 4-methoxy-3,4-dihydro-2H-1,2-benzothiazine 1,1-dioxide (Compound No. 9)

A suspension of 1.50 g (7.1 mmol) of Compound No. 8 and 300 mg of 10% palladium-carbon in 70 mℓ of ethanol was stirred vigorously for 24 hours under hydrogen gas atmosphere. The reaction mixture was filtered and the filtrate was then concentrated under reduced pressure. The residue was purified by chromatography on a silica gel column (eluent: methylene chloride:ethyl acetate = 10:1), whereby 1.50 g of the title compound were obtained (yield: 99%).

### Example 10

### Synthesis of 2-(2-chloroethyl)-3,4-dihydro-2H-1,2-benzothiazin-4-one 1,1-dioxide ethylene acetal (Compound No. 10)

To a suspension of 440 mg (11 mmol) of 60% sodium hydride in 20 mℓ of DMF, a solution of 2.41 g (10 mmol) of 3,4-dihydro-2H-1,2-benzothiazin-4-one 1,1-dioxide ethylene acetal in 10 mℓ of DMF was added under ice cooling and stirring. The reaction mixture was stirred for one hour at 0°C and then for further one hour at room temperature. Under ice cooling, a solution of 2.86 g (20 mmol) of 1-bromo-2-chloroethane in 10 mℓ of DMF was added to the reaction mixture, followed by stirring for 16 hours at room temperature.

The reaction mixture was concentrated under reduced pressure. To the residue, a 3:1 v/v mixed solvent of ethyl acetate and benzene was added. The organic layer was washed with a 5% aqueous solution of citric acid, water (twice) and a saturated brine, dried over anhydrous sodium sulfate and then, concentrated under reduced pressure. The residue was purified by chromatography on a silica gel column (eluent: ethyl acetate:hexane = 1:1), whereby 2.55 g of the title compound were obtained (yield: 84%).

### Example 11

### Synthesis of 2-(3-chloropropyl)-3,4-dihydro-2H-1,2-benzothiazin-4-one 1,1-dioxide ethylene acetal (Compound No. 11)

To a suspension of 2.20 g (50 mmol) of 60% sodium hydride in 100 mℓ of DMF, a solution of 12.06 g (50 mmol) of 3,4-dihydro-2H-1,2-benzothiazin-4-one 1,1-dioxide ethylene acetal in 50 mℓ of DMF was added under ice cooling and stirring. The reaction mixture was stirred for 30 minutes at 0°C and then for further one hour at room temperature. Under ice cooling, a solution of 16.95 g (150 mmol) of 1,3-dichloropropane in 50 mℓ of DMF was added to the reaction mixture, followed by stirring for 17 hours at room temperature. The post-treatment and purification were conducted as in Example 10, whereby 13.41 g of the title compound were obtained (yield: 84%).

### Example 12

### Synthesis of 2-(4-chlorobutyl)-3,4-dihydro-2H-1,2-benzothiazin-4-one 1,1-dioxide ethylene acetal (Compound No. 12)

In a similar manner to Example 10 except that 1-bromo-2-chloroethane was replaced by the equimolar amount of 1,4-dichlorobutane, the reaction, post-treatment and purification were conducted, whereby 2.53 g of the title compound were obtained (yield: 76%).

### Example 13

### Synthesis of 2-(2-chloroethyl)-3,4-dihydro-2H-1,2-benzothiazine-4-spiro-2'-(1',3'-dithiolan) 1,1-dioxide (Compound No. 13)

As in Example 10, the reaction and post treatments were conducted using 547 mg (2 mmol) of Compound No. 4, 88 mg (2.2 mmol) of 60% sodium hydride, 574 mg (4 mmol) of 1-bromo-2-chloroethane and 12 mℓ of DMF.

The crude crystals so obtained were recrystallized from ethyl acetate-hexane, whereby 533 mg of the title compound were obtained (yield: 79%).

### Example 14

### Synthesis of 2-(3-chloropropyl)-3,4-dihydro-2H-1,2-benzothiazine-4-spiro-2'-(1',3'-dithiolan) 1,1-dioxide (Compound No. 14)

As in Example 13, the reaction, post-treatment and purification were conducted employing 10.94 g (40 mmol) of Compound No. 4, 1.76 g (44 mmol) of 60% sodium hydride, 18.89 g (120 mmol) of 1-bromo-3-chloropropane and 160 mℓ of DMF, whereby 13.17 g of the title compound were obtained (yield: 87%).

### Example 15

### Synthesis of 2-(2-chloroethyl)-3,4-dihydro-2H-1,2-benzothiazine-4-spiro-2'-(1',3'-dithian) 1,1-dioxide (Compound No. 15)

To a suspension of 66 mg (1.65 mmol) of 60% sodium hydride in 10 mℓ of DMF, a solution of 431 mg (1.5 mmol) of Compound No. 5 in 10 mℓ of DMF was added under ice cooling and stirring. The reaction mixture was stirred for 1.5 hours at room temperature and then for further 0.5 hour at 50 °C. Under ice cooling, a solution of 430 mg (3 mmol) of 1-bromo-2-chloroethane in 10 mℓ of DMF was added to the reaction mixture, followed by stirring for 16 hours at room temperature.

The reaction mixture was concentrated under reduced pressure. To the residue, 50 mℓ of a half-saturated aqueous solution of potassium carbonate were added, followed by extraction with chloroform. The organic layer was washed with saturated brine, dried over anhydrous sodium sulfate and then, concentrated under reduced pressure. The residue was subjected to chromatography on a silica gel column (eluent: chloroform). The crude crystals so obtained were recrystallized from ethyl acetate-hexane, whereby 403 mg of the title compound were obtained (yield: 77%).

### Example 16

### Synthesis of 2-(3-chloropropyl)-3,4-dihydro-2H-1,2-benzothiazine-4-spiro-2'-(1',3'-dithian) 1,1-dioxide (Compound No. 16)

As in Example 15, the reaction and post-treatment were conducted using 1.15 g (4 mmol) of Compound No. 5, 176 mg (4.4 mmol) of 60% sodium hydride, 1.36 g (12 mmol) of 1,3-dichloropropane and 50 mℓ of DMF.

The residue was subjected to chromatography on a silica gel column (eluent: ethyl acetate:hexane = 1:2). The crude products so obtained were recrystallized from ethyl acetate-hexane, whereby 1.00 g of the title compound were obtained (yield: 69%).

### Example 17

### Synthesis of 2-(3-bromopropyl)-4,4-dimethoxy-3,4-dihydro-2H-1,2-benzothiazine 1,1-dioxide (Compound No. 17)

A suspension of 83 mg (0.34 mmol) of Compound No. 7, 683 mg (3.4 mmol) of 1,3-dibromopropane and 97 mg (0.70 mmol) of potassium carbonate in 5 mℓ of acetone was refluxed for 4 hours. The reaction mixture was filtered and the filtrate was then concentrated under reduced pressure. The oil so obtained was purified by chromatography on a silica gel column (eluent: ethyl acetate:hexane = 1:3), whereby 112 mg of the title compound were obtained (yield: 90%).

### Example 18

### Synthesis of 2-(3-bromopropyl)-4-methoxy-2H-1,2-benzothiazine 1,1-dioxide (Compound No. 18)

A suspension of 527 mg (2.5 mmol) of Compound No. 8, 2.51 g (12.5 mmol) of 1,3-dibromopropane and 690 mg (5 mmol) of potassium carbonate in 37.5 mℓ of acetone was refluxed for 2 hours. The reaction mixture was filtered and the filtrate was then concentrated under reduced pressure. The residue was purified by chromatography on a silica gel column (eluent: ethyl acetate:hexane = 1:4), whereby 706 mg of the title compound were obtained (yield: 85%).

### Example 19

### Synthesis of 2-(3-bromopropyl)-4-methoxy-3,4-dihydro-2H-1,2-benzothiazine 1,1-dioxide (Compound No. 19)

A suspension of 213 mg (1 mmol) of Compound No. 9, 1.00 g (5 mmol) of 1,3-dibromopropane and potassium carbonate in 10 mℓ of acetone was refluxed for 4 hours. The reaction mixture was filtered and the filtrate was then concentrated under reduced pressure. The residue was purified by chromatography on a silica gel column (eluent: ethyl acetate:hexane = 1:3), whereby 294 mg of the title compound were obtained (yield: 88%).

### Example 20

### Synthesis of 2-(3-chloropropyl)-4-methoxy-3,4-dihydro-2H-1,2-benzothiazine 1,1-dioxide (Compound No. 20)

A suspension of 852 mg (4 mmol) of Compound No. 9, 942 mg (6 mmol) of 1-bromo-3-chloropropane and 1.10 g (8 mmol) of potassium carbonate in 40 mℓ of acetone was refluxed for 10 hours. The reaction mixture was filtered and the filtrate was then concentrated under reduced pressure. The residue was purified by chromatography on a silica gel column (eluent: ethyl acetate:hexane = 1:2), whereby 1.11 g of the title compound were obtained (yield: 95%).

### Example 21

### Synthesis of 2-(3-chloropropyl)-3,4-dihydro-2H-1,2-benzothiazin-4-one 1,1-dioxide (Compound No. 21)

A mixture of 3.18 g (10 mmol) of Compound No. 11, 40 mℓ of 3N hydrochloric acid and 40 mℓ of methanol was refluxed for 5 hours. The reaction mixture was concentrated under reduced pressure. To the residue, water was added, followed by extraction with methylene chloride. The organic layer was washed with a saturated brine, dried over anhydrous sodium sulfate and then, concentrated under reduced pressure. The residue was purified by chromatography on a silica gel column (eluent: ethyl acetate:hexane = 3:2), whereby 2.66 g of the title compound were obtained (yield: 97%).

### (Another process)

In a similar manner to Example 10 except that 3,4-dihydro-2H-1,2-benzothiazin-4-one 1,1-dioxide ethylene acetal and 1-bromo-2-chloroethane were replaced by 1.97 g (10 mmol) of 3,4-dihydro-2H-1,2-benzothiazin-4-one and 4.72 g (30 mmol) of 1-bromo-3-chloropropane, respectively, the reaction, post-treatment and purification were conducted, whereby 721 mg of the title compound were obtained (yield: 26%).

### Example 22

### Synthesis of 2-(3-chloropropyl)-4-hydroxyimino-3,4-dihydro-2H-1,2-benzothiazine 1,1-dioxide (Compound No. 22)

A mixture of 1.64 g (6 mmol) of Compound No. 21, 738 mg (9 mmol) of sodium acetate, 625 mg (9 mmol) of hydroxylamine hydrochloride and 50 mℓ of methanol was refluxed for 40 hours.

The reaction mixture was concentrated under reduced pressure. To the residue, a half-saturated aqueous solution of potassium carbonate was added, followed by extraction with chloroform. The organic layer was washed with a saturated brine, dried over anhydrous sodium sulfate and then, concentrated under reduced pressure. The residue was purified by chromatography on a silica gel column (eluent: ethyl acetate:hexane = 1:2), whereby 1.51 g of the title compound were obtained (yield: 87%).

### Example 23

### Synthesis of 2-(3-chloropropyl)-4-hydroxy-3,4-dihydro-2H-1,2-benzothiazine 1,1-dioxide (Compound No. 23)

Under ice cooling and stirring, 378 mg (10 mmol) of sodium borohydride were added in portions to a solution of 1.09 g (4 mmol) of Compound No. 21 in 40 mℓ of ethanol. The reaction mixture was stirred for 2 hours at 0°C and then for 18 hours at room temperature.

The reaction mixture was concentrated under reduced pressure. To the residue, water was added, followed by extraction with methylene chloride. The organic layer was washed with a saturated brine, dried over anhydrous sodium sulfate and then, concentrated under reduced pressure. The residue was purified by chromatography on a silica gel column (eluent: ethyl acetate:hexane = 1:1), whereby 1.03 g of the title compound were obtained (yield: 93%).

### (Another process)

A mixture of 398 mg (2 mmol) of 4-hydroxy-3,4-dihydro-2H-1,2-benzothiazine 1,1-dioxide, 471 mg (3 mmol) of 1-bromo-3-chloropropane, 552 mg (4 mmol) of potassium carbonate and 20 mℓ of acetone was refluxed for 10 hours. The reaction mixture was filtered and the filtrate was then concentrated under reduced pressure. The residue was purified by chromatography on a silica gel column (eluent: methylene chloride:ethyl acetate = 20:1), whereby 514 mg of the title compound were obtained (yield: 93%).

### Example 24

### Synthesis of 2-(3-chloropropyl)-4-ethoxy-3,4-dihydro-2H-1,2-benzothiazine 1,1-dioxide (Compound No. 24)

Under ice cooling and stirring, 23 mg (0.57 mmol) of 60% sodium hydride and 0.08 mℓ (0.94 mmol) of ethyl iodide were successively added to a solution of 130 mg (0.47 mmol) of Compound No. 23 in 2 mℓ of DMF. The reaction mixture was stirred for one hour under ice cooling and then, for further one hour at room temperature.

Ethyl acetate was added to the reaction mixture. The resulting mixture was washed with an aqueous solution of 10% citric acid (twice), water and a saturated brine, dried over anhydrous sodium sulfate and then, concentrated under reduced pressure. The residue was purified by chromatography on a silica gel column (eluent: ethyl acetate:hexane = 1:3), whereby 125 mg of the title compound were obtained (yield: 87%).

### Example 25

### Synthesis of 4-benzyloxy-2-(3-chloropropyl)-3,4-dihydro-2H-1,2-benzothiazine 1,1-dioxide (Compound No. 25)

Under ice cooling, 24 mg (0.60 mmol) of 60% sodium hydride and 0.12 mℓ (1.0 mmol) of benzyl bromide were added successively to a solution of 138 mg (0.50 mmol) of Compound No. 23 in 2 mℓ of DMF. The reaction mixture was stirred for one hour under ice cooling and for further one hour at room temperature. The reaction mixture was post-treated as in Example 24. The residue was purified by chromatography on a silica gel column (eluent: ethyl acetate:hexane = 1:4), whereby 139 mg of the title compound were obtained (yield: 76%).

### Example 26

### Synthesis of 2-(3-chloropropyl)-2H-1,2-benzothiazine 1,1-dioxide (Compound No. 26)

Under ice cooling and stirring, 1.2 mℓ (15 mmol) of methanesulfonyl chloride were added to a solution of 1.44 g (5.2 mmol) of Compound No. 23, 3.03 g (30 mmol) of triethylamine in 52 mℓ of methylene chloride, followed by stirring at 0°C for one hour.

Ethyl acetate was added to the reaction mixture. The organic layer was washed with 0.5N hydrochloric acid, water, a saturated aqueous solution of sodium bicarbonate and saturated brine, dried over anhydrous sodium sulfate and then concentrated under reduced pressure. The residue was dissolved in 100 mℓ of methylene chloride and 50 mℓ of ethyl acetate, followed by the addition of 50 g of silica gel. After the resulting mixture was stirred at room temperature for 17 hours, the reaction mixture was filtered. The solid matter so obtained was extracted with ethyl acetate. The filtrate and the extract were combined together, followed by washing with a saturated aqueous solution of sodium bicarbonate and saturated brine, drying over anhydrous sodium sulfate and concentration under reduced pressure. The residue was purified by chromatography on a silica gel column (eluent: methylene chloride:ethyl acetate = 20:1), whereby 937 mg of the title compound were obtained (yield: 70%).

### Example 27

### Synthesis of 2-(3-chloropropyl)-3,4-dihydro-2H-1,2-benzothiazine 1,1-dioxide (Compound No. 27)

To a solution of 82 mg (0.32 mmol) of Compound No. 26 and three drops of acetic acid in 10 mℓ of ethanol, 20 mg of 10% palladium-carbon were added, followed by stirring under a hydrogen gas stream for 72 hours. The reaction mixture was filtered. The filtrate was concentrated under reduced pressure. The residue so obtained was purified by chromatography on a silica gel column (eluent: ethyl acetate:hexane = 1:3), whereby 73 mg of the title compound were obtained (yield: 88%).

### Example 28

### Synthesis of 2-[3-(4-phenylpiperazin-1-yl)propyl]-3,4-dihydro-2H-1,2-benzothiazin-4-one 1,1-dioxide ethylene acetal (Compound No. 28)

A suspension of 1.27 g (4 mmol) of Compound No. 11, 2.60 g (16 mmol) of 1-phenylpiperazine and 6.00 g (40 mmol) of sodium iodide in 95 mℓ of DMF was stirred at 80°C for 16 hours.

To the reaction mixture, 400 mℓ of a 3:1 v/v mixed solvent of ethyl acetate and benzene were added. The organic layer was washed with a half-saturated aqueous solution of potassium carbonate, water and a saturated brine, dried over anhydrous sodium sulfate and then, concentrated under reduced pressure. The residue was subjected to chromatography on a silica gel column (eluent: ethyl acetate). The crude crystals so obtained were recrystallized from 2-propanol-isopropyl ether, whereby 1.57 g of the title compound were obtained (yield: 88%).

### Example 29

### Synthesis of 2-[2-[4-(4-fluorophenyl)piperazin-1-yl]ethyl]-3,4-dihydro-2H-1,2-benzothiazin-4-one 1,1-dioxide ethylene acetal (Compound No. 29)

A suspension of 607 mg (2 mmol) of Compound No. 10, 721 mg (4 mmol) of 1-(4-fluorophenyl)piperazine and 600 mg (4 mmol) of sodium iodide in 30 mℓ of acetonitrile was refluxed for 16 hours.

The reaction mixture was concentrated under reduced pressure. To the residue, ethyl acetate was added. The organic layer was washed with a half-saturated aqueous solution of potassium carbonate, water and a saturated brine, dried over anhydrous sodium sulfate and then, concentrated under reduced pressure. The residue was purified by chromatography on a silica gel column (eluent: ethyl acetate:hexane = 1:1 → ethyl acetate), whereby 443 mg of the title compound were obtained (yield: 49%).

### Example 30

### Synthesis of 2-[4-[4-(4-fluorophenyl)piperazin-1-yl]butyl]-3,4-dihydro-2H-1,2-benzothiazin-4-one 1,1-dioxide ethylene acetal (Compound No. 30)

In a similar manner to Example 29 except that Compound No. 10 was replaced by 663 mg (2 mmol) of Compound No. 12, the reaction, post-treatment and purification were conducted, whereby 697 mg of the title compound were obtained (yield: 73%).

### Example 31

### Synthesis of 2-[3-(4-diphenylmethylpiperazin-1-yl)propyl]-3,4-dihydro-2H-1,2-benzothiazin-4-one 1,1-dioxide ethylene acetal (Compound No. 31)

A suspension of 636 mg (2 mmol) of Compound No. 11, 606 mg (2.4 mmol) of 1-diphenylmethylpiperazine and 332 mg (2.4 mmol) of potassium carbonate in 30 mℓ of acetonitrile was refluxed for 40 hours. The reaction mixture was post-treated as in Example 22. The residue was subjected to chromatography on a silica gel column (eluent: methanol:chloroform = 3:97). The crude crystals so obtained were recrystallized from ethanol, whereby 760 mg of the title compound were obtained (yield: 71%).

### Example 32

### Synthesis of 2-[3-(4-phenylpiperidino)propyl]-3,4-dihydro-2H-1,2-benzothiazin-4-one 1,1-dioxide ethylene acetal (Compound No. 32)

A suspension of 636 mg (2 mmol) of Compound No. 11, 484 mg (3 mmol) of 4-phenylpiperidine, 415 mg (3 mmol) of potassium carbonate and 600 mg (4 mmol) of sodium iodide in 30 mℓ of acetonitrile was refluxed for 13 hours.

The post-treatment and purification were conducted as in Example 29, whereby 693 mg of the title compound were obtained (yield: 78%).

### Example 33

### Synthesis of 2-[3-[4-(3-methoxyphenyl)piperazin-1-yl]propyl]-3,4-dihydro-2H-1,2-benzothiazin-4-one 1,1-dioxide ethylene acetal (Compound No. 33)

A suspension of 318 mg (1 mmol) of Compound No. 11, 288 mg (1.5 mmol) of 1-(3-methoxyphenyl)piperazine, 207 mg (1.5 mmol) of potassium carbonate and 300 mg (2 mmol) of sodium iodide in 15 mℓ of acetonitrile was refluxed for 17 hours. The reaction mixture was post-treated as in Example 31. The residue was subjected to chromatography on a silica gel column (eluent: methanol:chloroform = 3:97). The crude crystals so obtained were recrystallized from ethyl acetate-hexane, whereby 326 mg of the title compound were obtained (yield: 69%).

### Example 34

### Synthesis of 2-[3-[4-(4-methoxyphenyl)piperazin-1-yl]propyl]-3,4-dihydro-2H-1,2-benzothiazin-4-one 1,1-dioxide ethylene acetal (Compound 34)

A suspension of 318 mg (1 mmol) of Compound No. 11, 264 mg (1 mmol) of 1-(4-methoxyphenyl)piperazine dihydrochloride, 420 mg (5 mmol) of sodium bicarbonate and 300 mg (2 mmol) of sodium iodide in 15 mℓ of acetonitrile was refluxed for 20 hours. The reaction mixture was post-treated as in Example 31. The residue was subjected to chromatography on a silica gel column (eluent: methanol:chloroform = 1:99), whereby 398 mg of the title compound were obtained (yield: 84%).

### Example 35

### Synthesis of 2-[3-[4-(2-fluorophenyl)piperazin-1-yl]propyl]-3,4-dihydro-2H-1,2-benzothiazin-4-one 1,1-dioxide ethylene acetal (Compound 35)

A suspension of 318 mg (1 mmol) of Compound No. 11, 217 mg (1 mmol) of 1-(2-fluorophenyl)piperazine hydrochloride, 336 mg (4 mmol) of sodium bicarbonate and 300 mg (2 mmol) of sodium iodide in 15 mℓ of acetonitrile was refluxed for 18 hours. The reaction mixture was post-treated as in Example 31. The residue was subjected to chromatography on a silica gel column (eluent: methanol:chloroform = 1:99). The crude crystals so obtained were recrystallized from ethyl acetate-hexane-ethyl ether, whereby 233 mg of the title compound were obtained (yield: 50%).

### Example 36

### Synthesis of 2-[3-[4-(3-fluorophenyl)piperazin-1-yl]propyl]-3,4-dihydro-2H-1,2-benzothiazin-4-one 1,1-dioxide ethylene acetal (Compound No. 36)

In a similar manner to Example 35 except that 1-(2-fluorophenyl)piperazine hydrochloride was replaced by 261 mg (1 mmol) of 1-(3-fluorophenyl)piperazine hydrobromide, the reaction and post-treatment were conducted. The residue was purified by chromatography on a silica gel column (eluent: chloroform), whereby 369 mg of the title compound were obtained (yield: 80%).

### Example 37

### Synthesis of 2-[3-[4-(4-hydroxyphenyl)piperazin-1-yl]propyl]-3,4-dihydro-2H-1,2-benzothiazin-4-one 1,1-dioxide ethylene acetal (Compound No. 37)

In a similar manner to Example 35 except that 1-(2-fluorophenyl)piperazine hydrochloride was replaced by 259 mg (1 mmol) of 1-(4-hydroxyphenyl)piperazine hydrobromide, the reaction and post-treatment were conducted. The residue was purified by chromatography on a silica gel column (eluent: methanol:chloroform = 3:97), whereby 286 mg of the title compound were obtained (yield: 62%).

### Example 38

### Synthesis of 2-[3-[4-(4-chlorophenyl)piperazin-1-yl]propyl]-3,4-dihydro-2H-1,2-benzothiazin-4-one 1,1-dioxide ethylene acetal (Compound No. 38)

In a similar manner to Example 35 except that 1-(2-fluorophenyl)piperazine hydrochloride was replaced by 233 mg (1 mmol) of 1-(4-chlorophenyl)piperazine hydrochloride, the reaction and post-treatment were conducted. The residue was purified by chromatography on a silica gel column (eluent: methanol:chloroform = 1:99), whereby 434 mg of the title compound were obtained (yield: 90%).

### Example 39

### Synthesis of 2-[3-[4-(4-methanesulfonamidophenyl)-piperazin-1-yl]propyl]-3,4-dihydro-2H-1,2-benzothiazin-4-one 1,1-dioxide ethylene acetal (Compound 39)

A suspension of 159 mg (0.5 mmol) of Compound No. 11, 146 mg (0.5 mmol) of 4-(4-methanesulfonamidophenyl)piperazine hydrochloride, 168 mg (2 mmol) of sodium bicarbonate and 150 mg (1 mmol) of sodium iodide in 15 mℓ of acetonitrile was refluxed for 16 hours. The reaction mixture was post-treated in a similar manner to Example 22. The residue was purified by chromatography on a silica gel column (eluent: methanol:chloroform = 1:99 → 2:98 → 3:97), whereby 133 mg of the title compound were obtained (yield: 50%).

### Example 40

### Synthesis of 2-[3-[4-(4-fluorobenzoyl)piperidino]-propyl]-3,4-dihydro-2H-1,2-benzothiazine-4-spiro-2'-(1',3'-dioxolan) 1,1-dioxide (Compound No. 40)

A suspension of 795 mg (2.5 mmol) of Compound No. 11, 609 mg (2.5 mmol) of 4-(4-fluorobenzoyl)piperidine hydrochloride, 804 mg (10 mmol) of sodium bicarbonate and 750 mg (5 mmol) of sodium iodide in 25 mℓ of acetonitrile was refluxed for 26 hours. The post-treatment and purification were conducted as in Example 31, whereby 1.02 g of the title compound were obtained (yield: 84%).

### Example 41

### Synthesis of 2-[3-[4-(2,4-difluorobenzoyl)-piperidino]propyl]-3,4-dihydro-2H-1,2-benzothiazin-4-one 1,1-dioxide ethylene acetal (Compound No. 41)

In a similar manner to Example 39 except that 4-(4-methanesulfonamidophenyl)piperazine hydrochloride was replaced by 157 mg (0.6 mmol) of 4-(2,4-difluorobenzoyl)piperidine hydrochloride, the reaction and the post treatments were conducted. The residue was purified by chromatography on a silica gel column (eluent: methylene chloride:methanol = 15:1), whereby 171 mg of the title compound were obtained (yield: 68%).

### Example 42

### Synthesis of 2-[2-[4-(6-fluoro-1,2-benzisoxazol-3-yl)piperidino]ethyl]-3,4-dihydro-2H-1,2-benzothiazin-4-one 1,1-dioxide ethylene acetal (Compound No. 42)

A suspension of 304 mg (1 mmol) of Compound No. 10, 264 mg (1.2 mmol) of 4-(6-fluoro-1,2-benzisoxazol-3-yl)piperidine, 168 mg (2 mmol) of sodium bicarbonate and 300 mg (2 mmol) of sodium iodide in 20 mℓ of acetonitrile was refluxed for 18 hours.

The reaction mixture was post-treated as in Example 22. The residue was purified by chromatography on a silica gel column (eluent: methylene chloride: methanol = 30: 1), whereby 270 mg of the title compound were obtained (yield: 55%).

### Example 43

### Synthesis of 2-[3-[4-(6-fluoro-1,2-benzisoxazol-3-yl)piperidino]propyl] -3,4-dihydro-2H-1,2-benzothiazin-4-one 1,1-dioxide ethylene acetal (Compound No. 43)

A suspension of 95 mg (0.3 mmol) of Compound No. 11, 72 mg (0.3 mmol) of 4-(6-fluoro-1,2-benzisoxazol-3-yl)piperidine, 101 mg (1.2 mmol) of sodium bicarbonate and 90 mg (0.6 mmol) of sodium iodide in 7.5 mℓ of acetonitrile was refluxed for 22 hours. The reaction mixture was post-treated and purified as in Example 42, whereby 128 mg of the title compound were obtained (yield: 85%).

### Example 44

### Synthesis of 2-[3-[4-(1,2-benzisothiazol-3-yl]-piperazin-1-yl]propyl]-3,4-dihydro-2H-1,2-benzothiazin-4-one 1,1-dioxide ethylene acetal (Compound No. 44)

A suspension of 318 mg (1 mmol) of Compound No. 11, 329 mg (1.5 mmol) of 1-(1,2-benzisothiazol-3-yl)piperazine, 207 mg (1.5 mmol) of potassium carbonate and 300 mg (2 mmol) of sodium iodide in 15 mℓ of acetonitrile was refluxed for 15 ours. The reaction mixture was post-treated as in Example 31. The residue was purified by chromatography on a silica gel column (eluent: methanol:chloroform = 1:99), whereby 501 mg of the title compound were obtained (yield: 100%).

### Example 45

### Synthesis of 2-[3-[4-(6-fluoro-1H-indazol-3-yl)piperidino]propyl]-3,4-dihydro-2H-1,2-benzothiazin-4-one 1,1-dioxide ethylene acetal (Compound No. 45)

A suspension of 159 mg (0.5 mmol) of Compound No. 11, 164 mg (0.75 mmol) of 4-(6-fluoro-1H-indazol-3-yl)piperidin, 104 mg (0.75 mmol) of potassium carbonate and 150 mg (1 mmol) of sodium iodide in 10 mℓ of acetonitrile was refluxed for 15 hours. The reaction mixture was post-treated as in Example 22. The residue was subjected to chromatography on a silica gel column (eluent: methanol:chloroform = 1:19). The crude crystals so obtained were recrystallized from chloroformethyl ether, whereby 204 mg of the title compound were obtained (yield: 82%).

### Example 46

### Synthesis of 2-[3-[4-(2-pyridyl)piperazin-1-yl]-propyl]-3,4-dihydro-2H-1,2-benzothiazin-4-one 1,1-dioxide ethylene acetal (Compound No. 46)

In a similar manner to Example 44 except that 1-(1,2-benzisothiazol-3-yl)piperazine was replaced by 245 mg (1.5 mmol) of 1-(2-pyridyl)piperazine, the reaction and post-treatment were conducted. The residue so obtained was purified by chromatography on a silica gel column (eluent: methanol:chloroform = 2:98), whereby 336 mg of the title compound were obtained (yield: 76%).

### Example 47

### Synthesis of 2-[3-[4-(2-pyrimidyl)piperazin-1-yl]propyl]-3,4-dihydro-2H-1,2-benzothiazin-4-one 1,1-dioxide ethylene acetal (Compound No. 47)

In a similar manner to Example 34 except that 1-(4-methoxyphenyl)piperazine dihydrochloride was replaced by 237 mg (1 mmol) of 1-(2-pyrimidyl)piperazine dihydrochloride, the reaction and post-treatment were conducted. The residue so obtained was purified by chromatography on a silica gel column (eluent: methanol:chloroform = 2:98), whereby 390 mg of the title compound were obtained (yield: 88%).

### Example 48

### Synthesis of 2-[3-[4-(4-fluorobenzoyl)piperidino]-propyl]-3,4-dihydro-2H-1,2-benzothiazine-4-spiro-2'-(1',3'-dithiolan) 1,1-dioxide (Compound No. 48)

A suspension of 350 mg (1 mmol) of Compound No. 14, 244 mg (1 mmol) of 4-(4-fluorobenzoyl)piperidine hydrochloride, 336 mg (4 mmol) of sodium bicarbonate and 300 mg (2 mmol) of sodium iodide in 15 mℓ of acetonitrile was refluxed for 17 hours. The reaction mixture was post-treated as in Example 22. The residue was purified by chromatography on a silica gel column (eluent: methylene chloride:methanol = 97:3), whereby 450 mg of the title compound were obtained (yield: 86%).

### Example 49

### Synthesis of 2-[3-(4-(6-fluoro-1H-indazol-3-yl)piperidino]propyl]-3,4-dihydro-2H-1,2-benzothiazine-4-spiro-2'-(1',3'-dithiolan) 1,1-dioxide (Compound No. 49)

In a similar manner to Example 48 except that 4-(4-fluorobenzoyl)piperidine hydrochloride was replaced by 219 mg (1 mmol) of 4-(6-fluoro-1H-indazol-3-yl)piperidine, the reaction, post-treatment and purification were conducted, whereby 240 mg of the title compound were obtained (yield: 45%).

### Example 50

### Synthesis of 2-[3-[4-(4-hydroxyphenyl)piperazin-1-yl]propyl]-3,4-dihydro-2H-1,2-benzothiazine-4-spiro-2'-(1',3'-dithiolan) 1,1-dioxide (Compound No. 50)

In a similar manner to Example 48 except that 4-(4-fluorobenzoyl)piperidine hydrochloride was replaced by 259 mg (1 mmol) of 1-(4-hydroxyphenyl)piperazine hydrobromide, the reaction and post-treatment were conducted. The residue was subjected to chromatography on a silica gel column (eluent: methylene chloride: methanol = 97:3 → 19:1). The crude crystals so obtained were recrystallized from acetonitrile-isopropyl ether, whereby 270 mg of the title compound were obtained (yield: 55%).

### Example 51

### Synthesis of 2-[2-[4-(4-fluorophenyl)piperazin-1-yl]ethyl]-3,4-dihydro-2H-1,2-benzothiazine-4-spiro-2'-(1',3'-dithiolan) 1,1-dioxide (Compound No. 51)

A suspension of 336 mg (1 mmol) of Compound No. 13, 276 mg (1.5 mmol) of 1-(4-fluorophenyl)piperazine, 504 mg (6 mmol) of sodium bicarbonate and 300 mg (2 mmol) of sodium iodide in 15 mℓ of acetonitrile was refluxed for 44 hours. The reaction mixture was post-treated as in Example 31. The residue was subjected to chromatography on a silica gel column (eluent: ethyl acetate:hexane = 1:1). The crude crystals so obtained were recrystallized from ethyl acetate-hexane, whereby 315 mg of the title compound were obtained (yield: 66%).

### Example 52

### Synthesis of 2-[2-[4-(4-hydroxyphenyl)piperazin-1-yl]ethyl]-3,4-dihydro-2H-1,2-benzothiazine-4-spiro-2'-(1',3'-dithiolan) 1,1-dioxide (Compound No. 52)

A suspension of 168 mg (0.5 mmol) of Compound No. 13, 130 mg (0.5 mmol) of 1-(4-hydroxyphenyl)piperazine hydrobromide, 168 mg (2 mmol) of sodium bicarbonate and 150 mg (1 mmol) of sodium iodide in 15 mℓ of acetonitrile was refluxed for 20 hours. The reaction mixture was filtered and the filtrate was concentrated under reduced pressure. The residue was subjected to chromatography on a silica gel column (eluent: methanol:chloroform = 3:97). The crude crystals so obtained were recrystallized from ethyl acetate-hexane, whereby 78 mg of the title compound were obtained (yield: 33%).

### Example 53

### Synthesis of 2-[3-[4-(4-hydroxyphenyl)piperazin-1-yl]propyl]-3,4-dihydro-2H-1,2-benzothiazine-4-spiro-2'-(1',3'-dithian) 1,1-dioxide (Compound No. 53)

As in Example 52, the reaction and post-treatment were conducted employing 364 mg (1 mmol) of Compound No. 16, 259 mg (1 mmol) of 1-(4-hydroxyphenyl)-piperazine hydrobromide, 336 mg (4 mmol) of sodium bicarbonate, 300 mg (2 mmol) of sodium iodide and 20 mℓ of acetonitrile. The residue was purified by chromatography on a silica gel column (eluent: methanol:chloroform = 3:97), whereby 337 mg of the title compound were obtained (yield: 67%).

### Example 54

### Synthesis of 2-[2-[4-(4-hydroxyphenyl)piperazin-1-yl]ethyl]-3,4-dihydro-2H-1,2-benzothiazine-4-spiro-2'-(1',3'-dithian) 1,1-dioxide (Compound No. 54)

In a similar manner to Example 53 except that Compound No. 16 was replaced by 350 mg (1 mmol) of Compound No. 15, the reaction and post-treatment were conducted. The residue was subjected to chromatography on a silica gel column (eluent: methanol: chloroform = 3:97). The crude crystals so obtained were recrystallized from acetonitrile-isopropyl ether, whereby 100 mg of the title compound were obtained (yield: 20%).

### Example 55

### Synthesis of 2-[3-[4-(4-fluorophenyl)piperazin-1-yl]propyl]-4,4-dimethoxy-3,4-dihydro-2H-1,2-benzothiazine 1,1-dioxide (Compound No. 55)

A suspension of 112 mg (0.3 mmol) of Compound No. 17, 81 mg (0.45 mmol) of 1-(4-fluorophenyl)piperazine and 83 mg (0.6 mmol) of potassium carbonate in 10 mℓ of 1,4-dioxane was stirred at 90°C for 15 hours. The reaction mixture was filtered and the filtrate was then concentrated under reduced pressure. The residue was purified by chromatography on a silica gel column (eluent: methanol:methylene chloride = 1:30), whereby 146 mg of the title compound were obtained (yield: 99%).

### Example 56

### Synthesis of 2-[3-[4-(4-fluorophenyl)piperazin-1-yl]propyl]-4-methoxy-2H-1,2-benzothiazine 1,1-dioxide (Compound No. 56)

A suspension of 664 mg (2 mmol) of Compound No. 18, 432 mg (2.4 mmol) of 1-(4-fluorophenyl)piperazine and 552 mg (4 mmol) of potassium carbonate in 30 mℓ of 1,4-dioxane was refluxed for 13 hours. The reaction mixture was filtered and the filtrate was then concentrated under reduced pressure. The residue was purified by chromatography on a silica gel column (eluent: methylene chloride:methanol = 40:1), whereby 785 mg of the title compound were obtained (yield: 91%).

### Example 57

### Synthesis of 2-[3-[4-(4-fluorophenyl)piperazin-1-yl]propyl]-4-methoxy-3,4-dihydro-2H-1,2-benzothiazine 1,1-dioxide (Compound No. 57)

A suspension of 250 mg (0.74 mmol) of Compound No. 19, 170 mg (0.90 mmol) of 1-(4-fluorophenyl)piperazine and 207 mg (1.5 mmol) of potassium carbonate in 10 mℓ of 1,4-dioxane was refluxed for 6 hours. The reaction mixture was filtered and the filtrate was then concentrated under reduced pressure. The residue was purified by chromatography on a silica gel column (eluent: methylene chloride:methanol = 30:1), whereby 303 mg of the title compound were obtained (yield: 94%).

### Example 58

### Synthesis of 2-[3-[4-(4-fluorobenzoyl)piperidino]-propyl]-4-methoxy-3,4-dihydro-2H-1,2-benzothiazine 1,1-dioxide (Compound No. 58)

A suspension of 145 mg (0.5 mmol) of Compound No. 20, 182 mg (0.75 mmol) of 4-(4-fluorobenzoyl)-piperidine hydrochloride, 168 mg (2 mmol) of sodium bicarbonate and 150 mg (1 mmol) of sodium iodide in 10 mℓ of acetonitrile was refluxed for 20 hours. The reaction mixture was post-treated as in Example 31. The residue was purified by chromatography on a silica gel column (eluent: methylene chloride: methanol = 20:1), whereby 220 mg of the title compound were obtained (yield: 95%).

### Example 59

### Synthesis of 4-ethoxy-2-[3-[4-(4-fluorophenyl)-piperazin-1-yl]propyl]-3,4-dihydro-2H-1,2-benzothiazine 1,1-dioxide (Compound No. 59)

A suspension of 152 mg (0.5 mmol) of Compound No. 24, 135 mg (0.75 mmol) of 1-(4-fluorophenyl)-piperazine, 84 mg (1.0 mmol) of sodium bicarbonate and 150 mg (1 mmol) of sodium iodide in 10 mℓ of acetonitrile was refluxed for 19 hours. The reaction mixture was post-treated as in Example 31. The residue was purified by chromatography on a silica gel column (eluent: methylene chloride:methanol = 30:1), whereby 190 mg of the title compound were obtained (yield: 85%).

### Example 60

### Synthesis of 2-[3-[4-(4-hydroxyphenyl)piperazin-1-yl]propyl]-4-methoxy-3,4-dihydro-2H-1,2-benzothiazine 1,1-dioxide (Compound No. 60)

A suspension of 145 mg (0.5 mmol) of Compound No. 20, 155 mg (0.6 mmol) of 1-(4-hydroxyphenyl)-piperazine hydrobromide, 168 mg (2 mmol) of sodium bicarbonate and 150 mg (1 mmol) of sodium iodide in 10 mℓ of acetonitrile was refluxed for 20 hours. The reaction mixture was post-treated as in Example 31. The residue was purified by chromatography on a silica gel column (eluent: methylene chloride:methanol = 15:1), whereby 70 mg of the title compound were obtained (yield: 32%).

### Example 61

### Synthesis of 4-benzyloxy-2-[3-[4-(4-fluorophenyl)-piperazin-1-yl]propyl]-3,4-dihydro-2H-1,2-benzothiazine 1,1-dioxide (Compound No. 61)

A suspension of 109 mg (0.3 mmol) of Compound No. 25, 81 mg (0.45 mmol) of 1-(4-fluorophenyl)-piperazine, 50 mg (0.6 mmol) of sodium bicarbonate and 90 mg (0.6 mmol) of sodium iodide in 6 mℓ of acetonitrile was refluxed for 18 hours. The reaction mixture was post-treated as in Example 31. The residue was purified by chromatography on a silica gel column (eluent: methylene chloride:methanol = 40:1), whereby 120 mg of the title compound were obtained (yield: 78%).

### Example 62

### Synthesis of 2-[3-[4-(4-fluorophenyl)piperazin-1-yl]propyl]-3,4-dihydro-2H-1,2-benzothiazin-4-one 1,1-dioxide ethylene acetal (Compound No. 62)

Under ice cooling and stirring, a solution of 2.42 g (10 mmol) of 3,4-dihydro-2H-1,2-benzothiazin-4-one 1,1-dioxide ethylene acetal in 20 mℓ of DMF was added to a suspension of 480 mg (12 mmol) of 60% sodium hydride in 60 mℓ of DMF. The reaction mixture was stirred for one hour at 0°C and for further one hour at room temperature.

The reaction mixture was thereafter cooled to 0°C, to which a solution of 3.85 g (15 mmol) of 1-(3-chloropropyl)-4-(4-fluorophenyl)piperazine in 20 mℓ of DMF was added. The resulting mixture was stirred at room temperature for 58 hours. The reaction mixture was post-treated as in Example 28. The residue was purified by chromatography on a silica gel column (eluent: ethyl acetate:hexane = 3:1 → ethyl acetate), whereby 4.30 g of the title compound were obtained (yield: 93%).

### Example 63

### Synthesis of 2-[3-[4-(4-fluorophenyl)piperazin-1-yl]propyl]-3,4-dihydro-2H-1,2-benzothiazine-4-spiro-2'-[(4'R,5'R)-dimethyl-1',3'-dioxolan] 1,1-dioxide (Compound No. 63)

As in Example 62, the reaction was conducted using 24 mg (0.6 mmol) of 60% sodium hydride, 135 mg (0.5 mmol) of Compound No. 2, 192 mg (0.75 mmol) of 1-(3-chloropropyl)-4-(4-fluorophenyl)piperazine and 6 mℓ of DMF. The reaction mixture was post-treated as in Example 22. The residue was purified by chromatography on a silica gel column (eluent: methylene chloride: methanol = 20: 1), whereby 176 mg of the title compound were obtained (yield: 72%).

### Example 64

### Synthesis of 2-[3-[4-(4-fluorophenyl)piperazin-1-yl]propyl]-3,4-dihydro-2H-1,2-benzothiazine-4-spiro-2'-(1',3'-dioxane) 1,1-dioxide (Compound No. 64)

As in Example 62, the reaction, post-treatment and purification were conducted employing 185 mg (4.63 mmol) of 60% sodium hydride, 987 mg (3.86 mmol) of Compound No. 1, 1.49 g (5.79 mmol) of 1-(3-chloropropyl)-4-(4-fluorophenyl)piperazine and 50 mℓ of DMF, whereby 1.33 g of the title compound were obtained (yield: 72%).

### Example 65

### Synthesis of 2-[3-[4-(4-fluorophenyl)piperazin-1-yl]propyl]-3,4-dihydro-2H-1,2-benzothiazine-4-spiro-2'-(1',3'-oxathiolan) 1,1-dioxide (Compound No. 65)

The reaction, post-treatment and purification were conducted as in Example 62 by employing 96 mg (2.4 mmol) of 60% sodium hydride, 515 mg (2 mmol) of Compound No. 3, 770 mg (3 mmol) of 1-(3-chloropropyl)-4-(4-fluorophenyl)piperazine and 25 mℓ of DMF, whereby 635 mg of the title compound were obtained (yield: 66%).

### Example 66

### Synthesis of 2-[3-[4-(4-fluorophenyl)piperazin-1-yl]propyl]-3,4-dihydro-2H-1,2-benzothiazine-4-spiro-2'-(1',3'-dithiolan) 1,1-dioxide (Compound No. 66)

The reaction, post-treatment and purification were conducted as in Example 62 by employing 88 mg (2.2 mmol) of 60% sodium hydride, 547 mg (2 mmol) of Compound No. 4, 770 mg (3 mmol) of 1-(3-chloropropyl)-4-(4-fluorophenyl)piperazine and 25 mℓ of DMF, whereby 950 mg of the title compound were obtained (yield: 96%).

### Example 67

### Synthesis of 2-[3-[4-(4-fluorophenyl)piperazin-1-yl]propyl]-3,4-dihydro-2H-1,2-benzothiazine-4-spiro-2'-(1',3'-dithian) 1,1-dioxide (Compound No. 67)

As in Example 62, the reaction was conducted using 44 mg (1.1 mmol) of 60%-sodium hydride, 287 mg (1 mmol) of Compound No. 5, 385 mg (1.5 mmol) of 1-(3-chloropropyl)-4-(4-fluorophenyl)piperazine and 20 mℓ of DMF.

The reaction mixture was concentrated under reduced pressure. A half-saturated aqueous solution of potassium carbonate was added to the residue, followed by extraction with ethyl acetate. The organic layer was washed with saturated brine, dried over anhydrous sodium sulfate and concentrated under reduced pressure. The residue was purified by chromatography on a silica gel column (eluent: chloroform), whereby 442 mg of the title compound were obtained (yield: 87%).

### Example 68

### Synthesis of 2-[2-[4-(4-fluorophenyl)piperazin-1-yl]ethyl]-3,4-dihydro-2H-1,2-benzothiazine-4-spiro-2'-(1',3'-dithian) 1,1-dioxide (Compound No. 68)

In a similar manner to Example 67 except that 1-(3-chloropropyl)-4-(4-fluorophenyl)piperazine was replaced by 364 mg (1.5 mmol) of 1-(2-chloroethyl)-4-(4-fluorophenyl)piperazine, the reaction was conducted.

As in Example 51, the post-treatments and purification were conducted, whereby 247 mg of the title compound were obtained (yield: 50%).

### Example 69

### Synthesis of 4,4-bis(ethylthio)-2-[3-[4-(4-fluorophenyl)piperazin-1-yl]propyl]-3,4-dihydro-2H-1,2-benzothiazine 1,1-dioxide (Compound No. 69)

In a similar manner to Example 65 except that Compound No. 3 was replaced by 607 mg (2 mmol) of Compound No. 6, the reaction, post-treatment and purification were conducted, whereby 814 mg of the title compound were obtained (yield: 78%).

### Example 70

### Synthesis of 2-[3-[4-(4-fluorophenyl)piperazin-1-yl]propyl]-3,4-dihydro-2H-1,2-benzothiazin-4-one 1,1-dioxide (Compound No. 70)

A mixture of 923 mg (2 mmol) of Compound No. 62, 25 mℓ of 3N hydrochloric acid and 25 mℓ of methanol was refluxed for 2 hours. The reaction mixture was concentrated under reduced pressure. To the residue, 200 mℓ of a half-saturated aqueous solution of potassium carbonate were added, followed by extraction with chloroform. The organic layer was washed with a saturated brine, dried over anhydrous sodium sulfate and then, concentrated under reduced pressure. The residue was purified by chromatography on a silica gel column (eluent: ethyl acetate:hexane = 2:1), whereby 772 mg of the title compound were obtained (yield: 92%).

### Example 71

### Synthesis of 2-[3-(4-phenylpipierazin-1-yl)propyl]-3,4-dihydro-2H-1,2-benzothiazin-4-one 1,1-dioxide (Compound No. 71)

In a similar manner to Example 70 except that Compound No. 62 was replaced by 887 mg (2 mmol) of Compound No. 28, the reaction, post-treatment and purification were conducted, whereby 719 mg of the title compound were obtained (yield: 90%).

### Example 72

### Synthesis of 2-[3-[4-(4-fluorobenzoyl)piperidino]-propyl]-3,4-dihydro-2H-1,2-benzothiazin-4-one 1,1-dioxide (Compound No. 72)

A mixture of 600 mg (1.2 mmol) of Compound No. 40, 5 mℓ of 3N hydrochloric acid and 5 mℓ of methanol was refluxed for 1.5 hours. The reaction mixture was post-treated as in Example 70. The residue was purified by chromatography on a silica gel column (eluent: methylene chloride:methanol = 30:1), whereby 483 mg of the title compound were obtained (yield: 90%).

### Example 73

### Synthesis of 4-hydroxyimino-2-[3-(4-phenylpiperazin-1-yl)propyl]-3,4-dihydro-2H-1,2-benzothiazine 1,1-dioxide (Compound No. 73)

A suspension of 230 mg (0.797 mmol) of Compound No. 22, 517 mg (3.19 mmol) of 1-phenylpiperazine and 1.19 g (7.97 mmol) of sodium iodide in 20 mℓ of DMF was stirred at 80°C for 16 hours. The post-treatment and purification were conducted as in Example 62, whereby 239 mg of the title compound were obtained (yield: 72%).

### Example 74

### Synthesis of 2-[3-[4-(4-fluorobenzoyl)piperidino]-propyl]-4-hydroxyimino-3,4-dihydro-2H-1,2-benzothiazine 1,1-dioxide (Compound No. 74)

A suspension of 722 mg (2.5 mmol) of Compound No. 22, 609 mg (2.5 mmol) of 4-(4-fluorobenzoyl)-piperidine hydrochloride, 840 mg (10 mmol) of sodium bicarbonate and 749 mg (5 mmol) of sodium iodide in 50 mℓ of acetonitrile was refluxed for 24 hours.

The reaction mixture was concentrated under reduced pressure. To the residue, a half-saturated aqueous solution of potassium carbonate was added, followed by extraction with dichloromethane and ethyl acetate. The organic layer was washed with a saturated brine, dried over anhydrous sodium sulfate and then, concentrated under reduced pressure. The solid matters so obtained were washed with methanol and then, recrystallized from acetonitrile, whereby 533 mg of the title compound were obtained (yield: 46%).

### Example 75

### Synthesis of 2-[3-[4-(4-fluorophenyl)piperazin-1-yl]propyl]-4-hydroxyimino-3,4-dihydro-2H-1,2-benzothiazine 1,1-dioxide (Compound No. 75)

A solution of 1.25 g (3 mmol) of Compound No. 70, 417 mg (6 mmol) of hydroxylamine hydrochloride and 492 mg (6 mmol) of sodium acetate in 30 mℓ of methanol was refluxed for 3 hours. The reaction mixture was post-treated as in Example 22. The residue was purified by chromatography on a silica gel column (eluent: methanol:chloroform = 3:97), whereby 1.27 g of the title compound were obtained (yield: 98%).

### Example 76

### Synthesis of 2-[3-[4-(4-fluorobenzoyl)piperidino]-propyl]-4-hydroxy-3,4-dihydro-2H-1,2-benzothiazine 1,1-dioxide (Compound No. 76)

In a similar manner to Example 74 except that Compound No. 22 was replaced by 689 mg (2.5 mmol) of Compound No. 23, the reaction was conducted. The reaction mixture was post-treated as in Example 31. The residue was purified by chromatography on a silica gel column (eluent: methanol:chloroform = 7.5:92.5), whereby 818 mg of the title compound were obtained (yield: 92%).

### Example 77

### Synthesis of 2-[3-[4-(4-fluorophenyl)piperazin-1-yl]propyl]-4-hydroxy-3,4-dihydro-2H-1,2-benzothiazine 1,1-dioxide (Compound No. 77)

Under ice cooling and stirring, 300 mg (7.9 mmol) of sodium borohydride were added in portions to a solution of 209 mg (0.5 mmol) of Compound No. 70 in 30 mℓ of ethanol. The reaction mixture was stirred for one hour at 0°C and for further 16 hours at room temperature.

The reaction mixture was post-treated as in Example 23. The residue was then purified by chromatography on a silica gel column (eluent: ethyl acetate), whereby 201 mg of the title compound were obtained (yield: 96%).

### Example 78

### Synthesis of 2-[3-[4-(4-fluorophenyl)piperazin-1-yl]propyl]-2H-1,2-benzothiazine 1,1-dioxide (Compound No. 78)

A suspension of 69 mg (0.27 mmol) of Compound No. 26, 58 mg (0.32 mmol) of 1-(4-fluorophenyl)piperazine, 45 mg (0.54 mmol) of sodium bicarbonate and 81 mg (0.54 mmol) of sodium iodide in 5 mℓ of acetonitrile was refluxed for 20 hours. The reaction mixture was post-treated and purified as in Example 63, whereby 97 mg of the title compound were obtained (yield: 89%).

### Example 79

### Synthesis of 2-[3-[4-(4-fluorophenyl)piperazin-1-yl]propyl]-3,4-dihydro-2H-1,2-benzothiazine 1,1-dioxide (Compound No. 79)

As in Example 78, the reaction, post-treatment and purification were conducted employing 73 mg (0.28 mmol) of Compound No. 27, 60 mg (0.34 mmol) of 1-(4-fluorophenyl)piperazine, 47 mg (0.56 mmol) of sodium bicarbonate, 84 mg (0.56 mmol) of sodium iodide and 5 mℓ of acetonitrile, whereby 108 mg of the title compound were obtained (yield: 96%).

The structural formulas and physical properties of the compounds obtained in the above examples are summarized in the following Table 1.

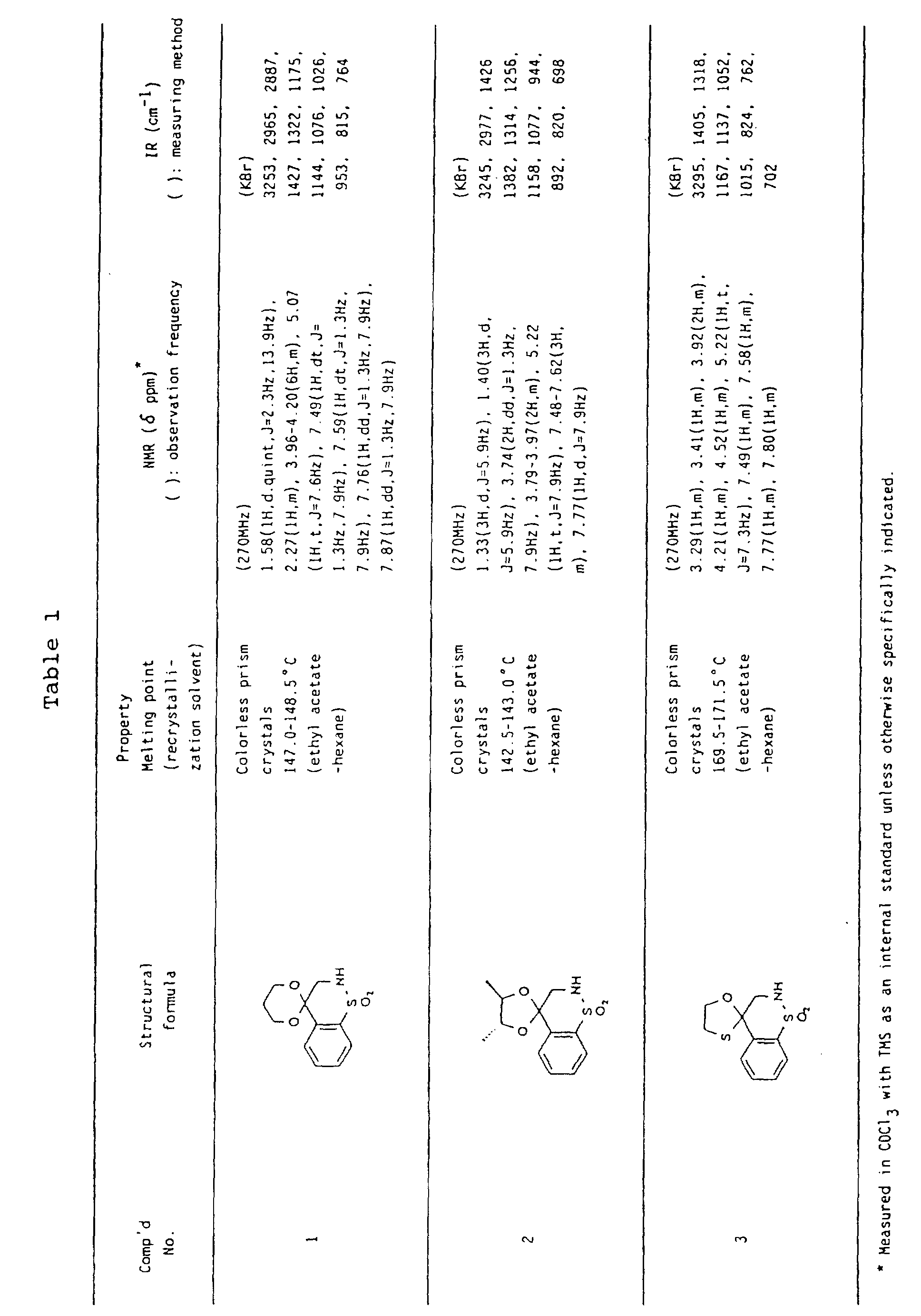

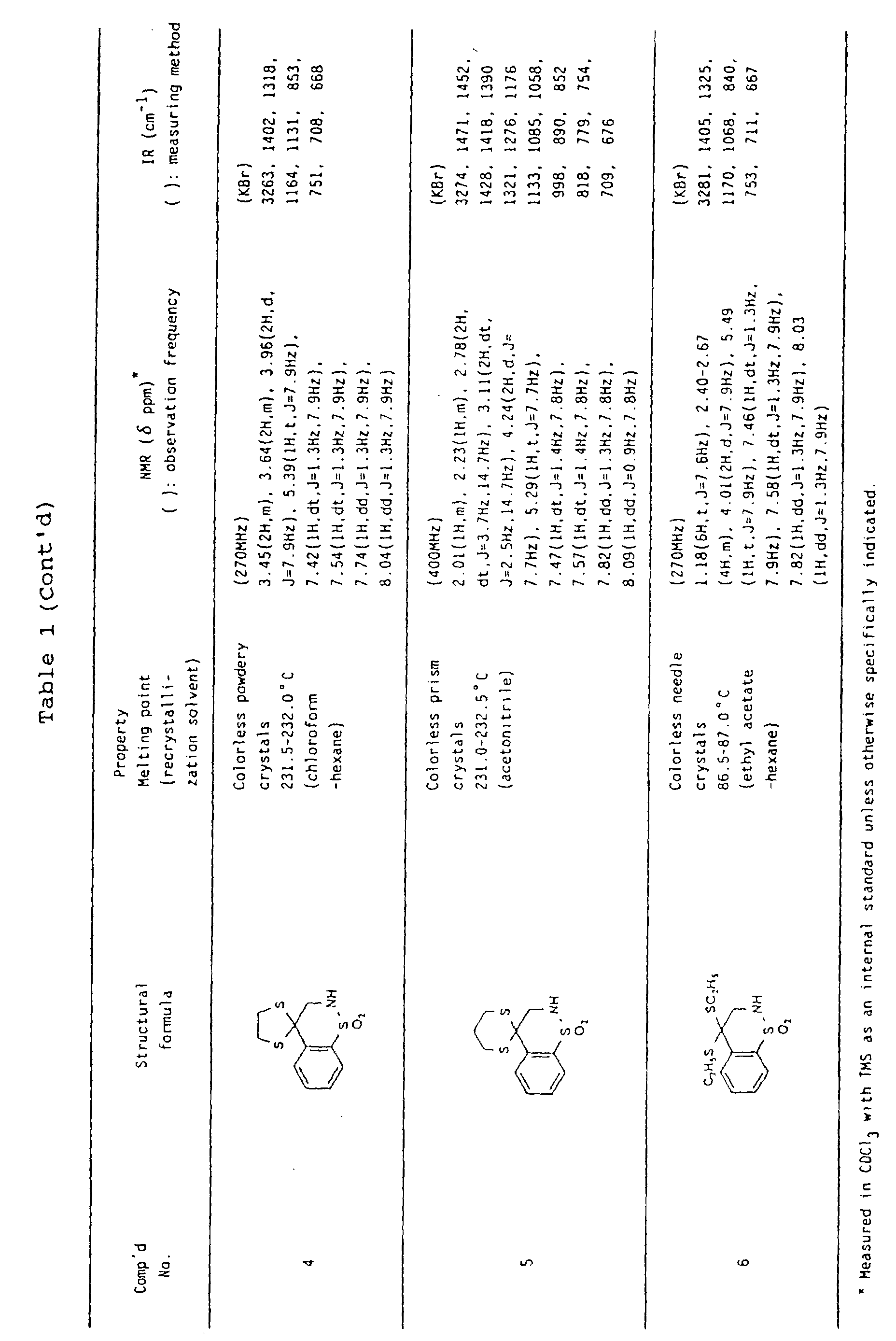

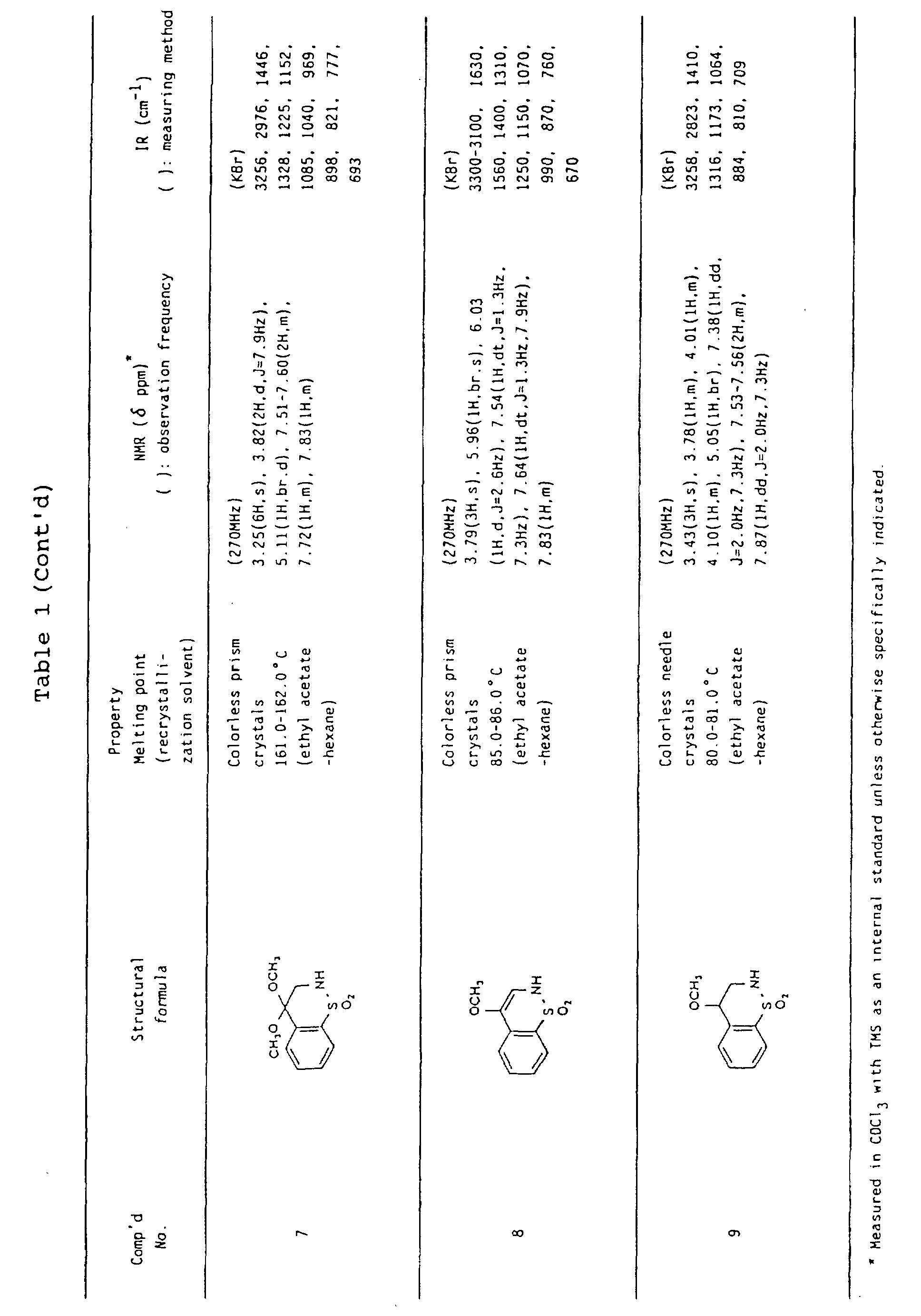

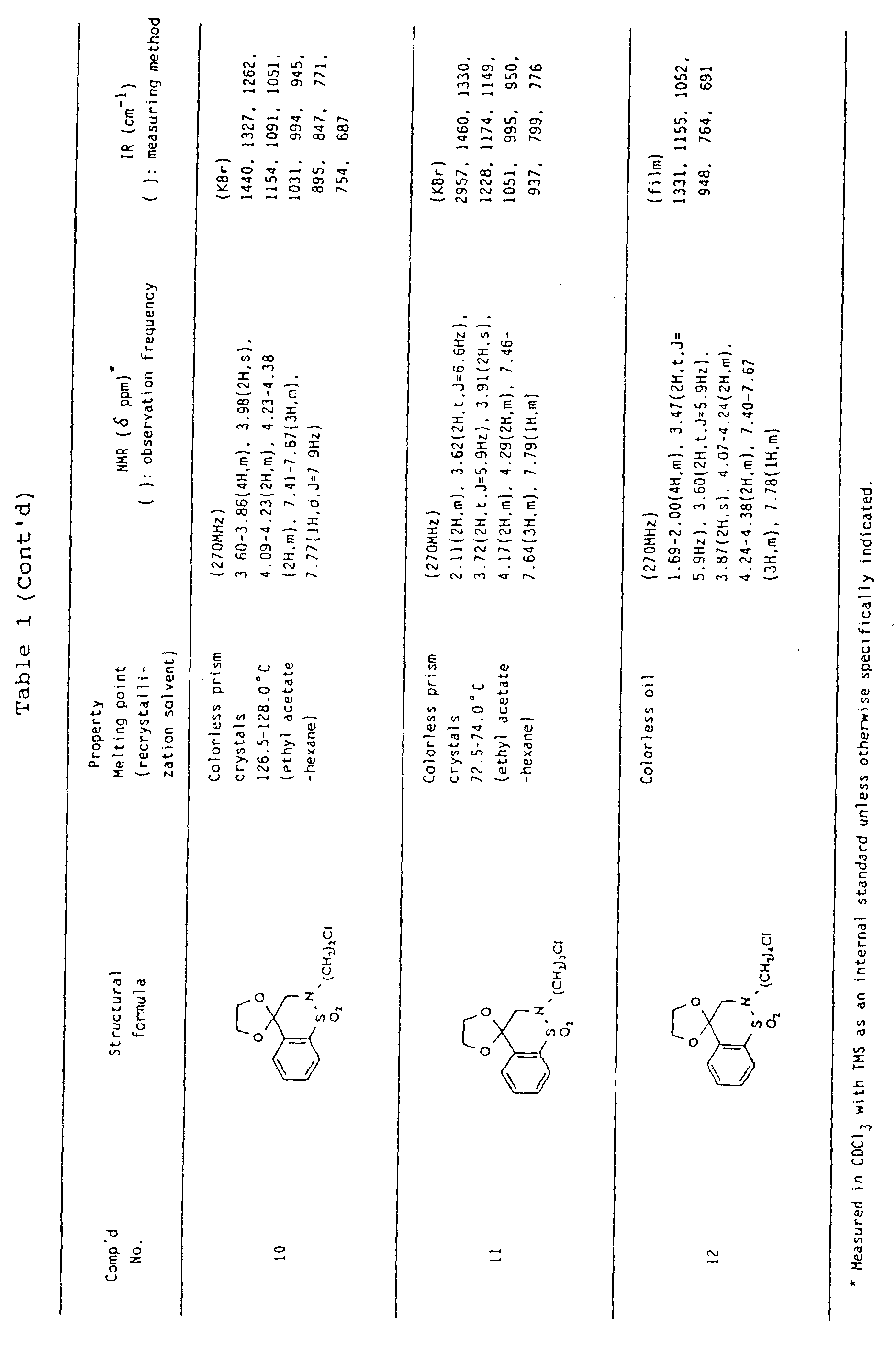

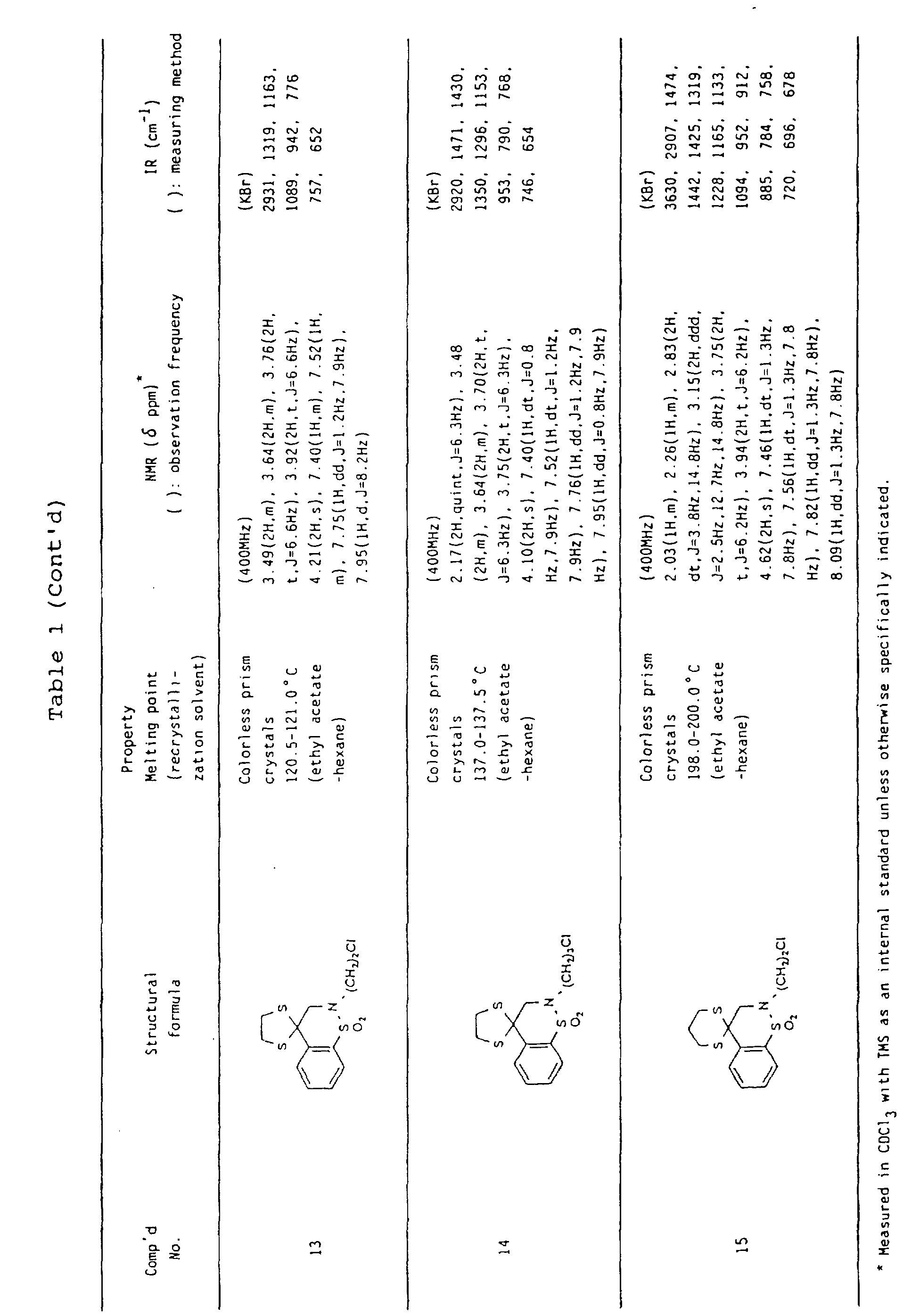

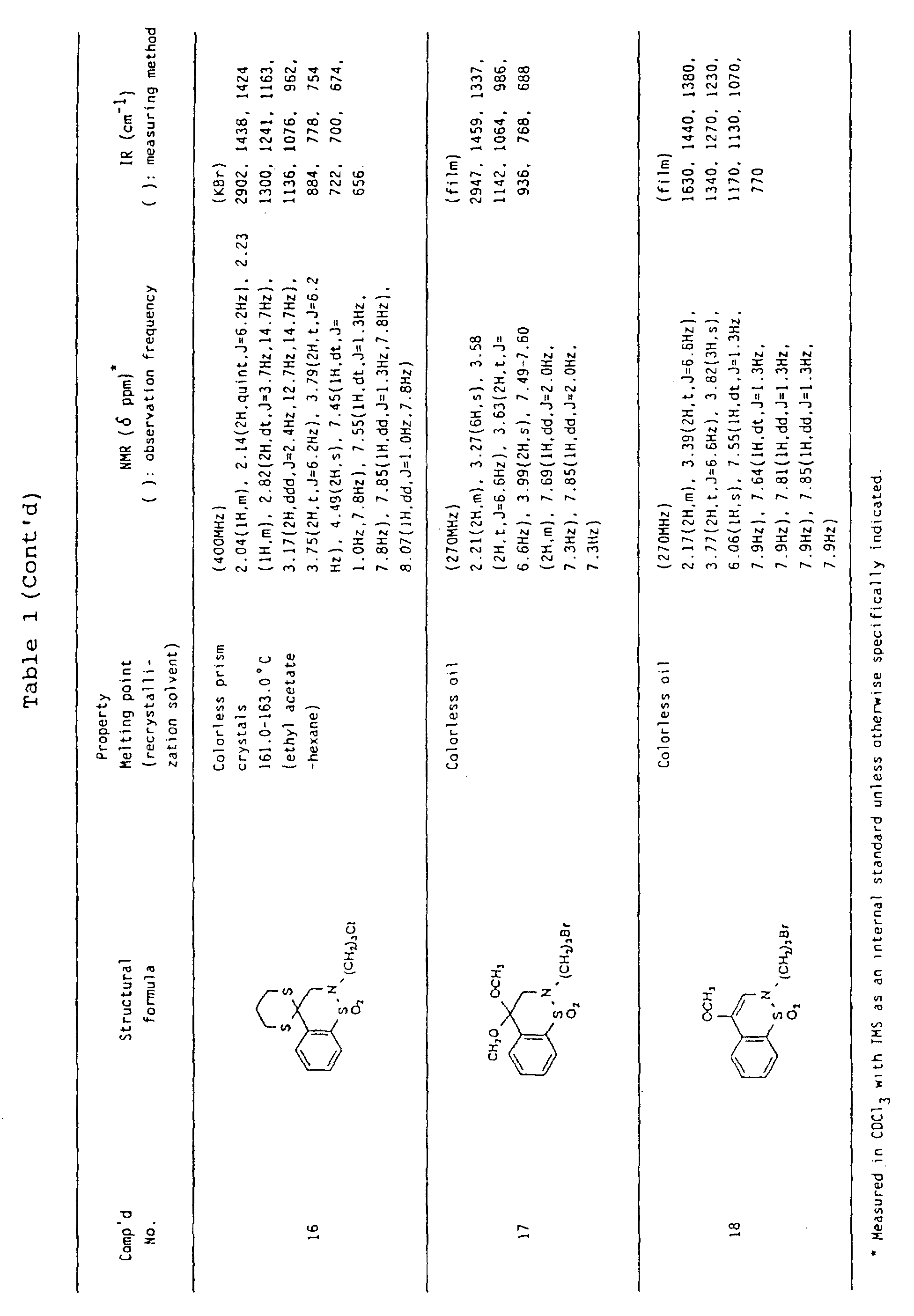

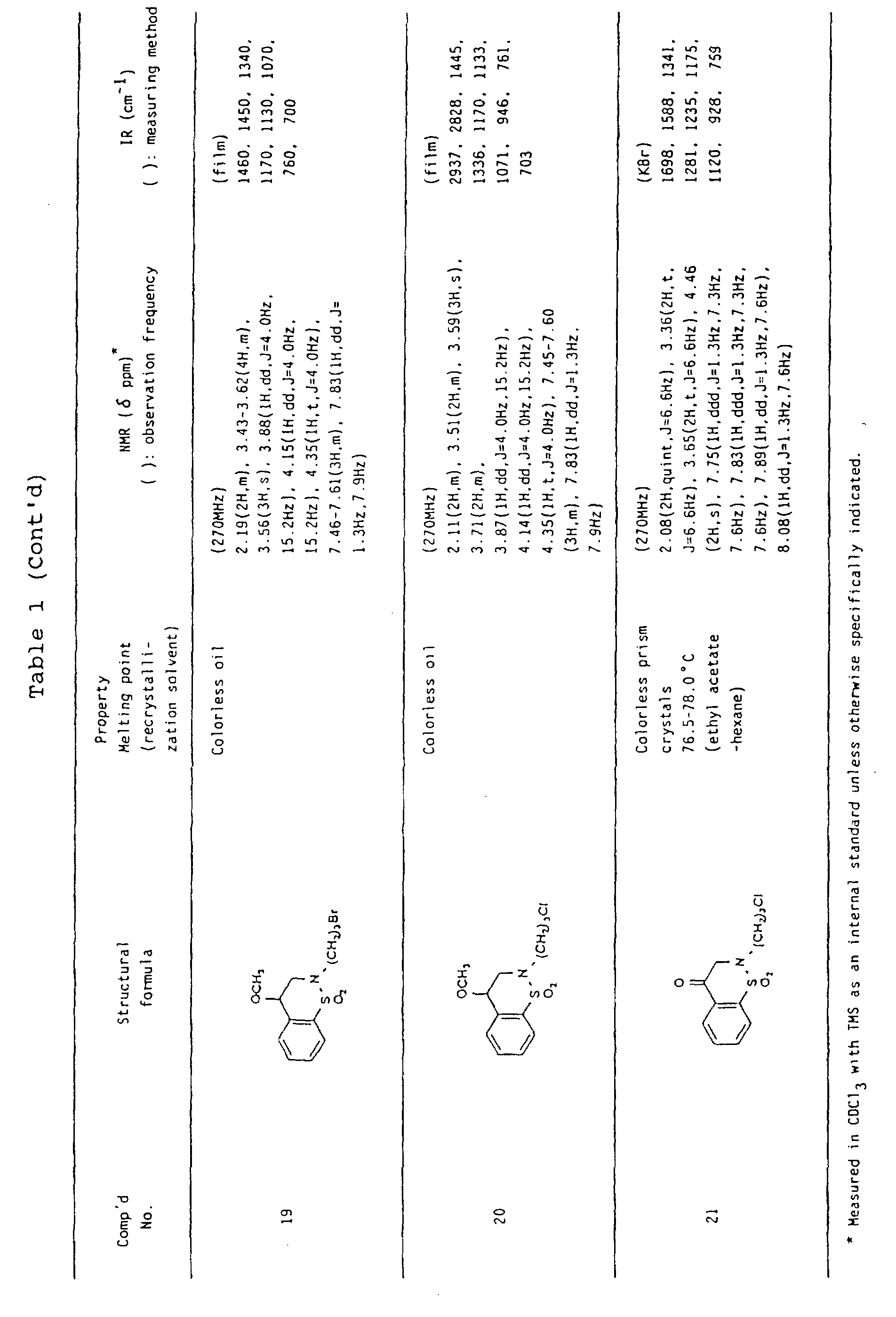

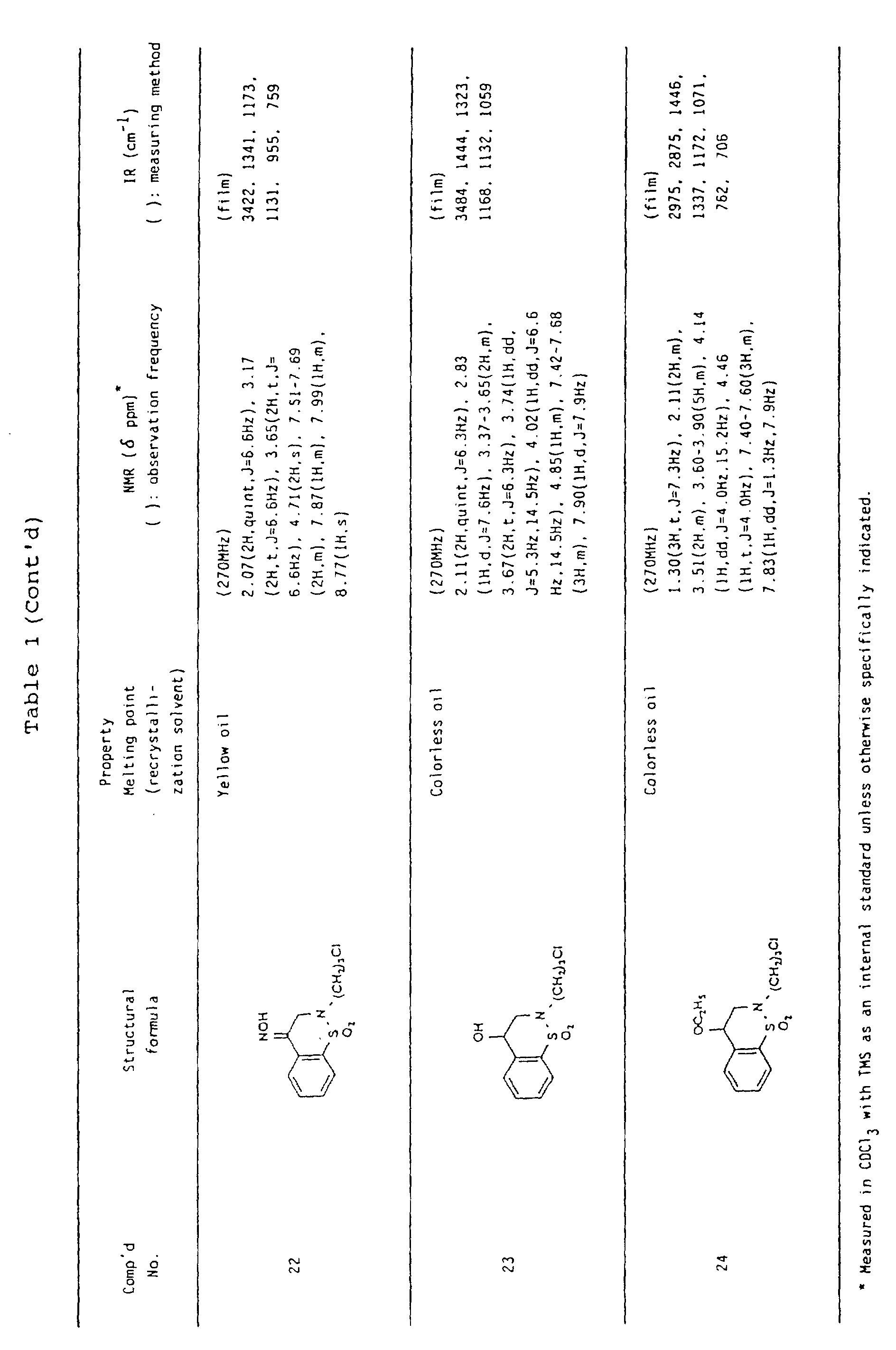

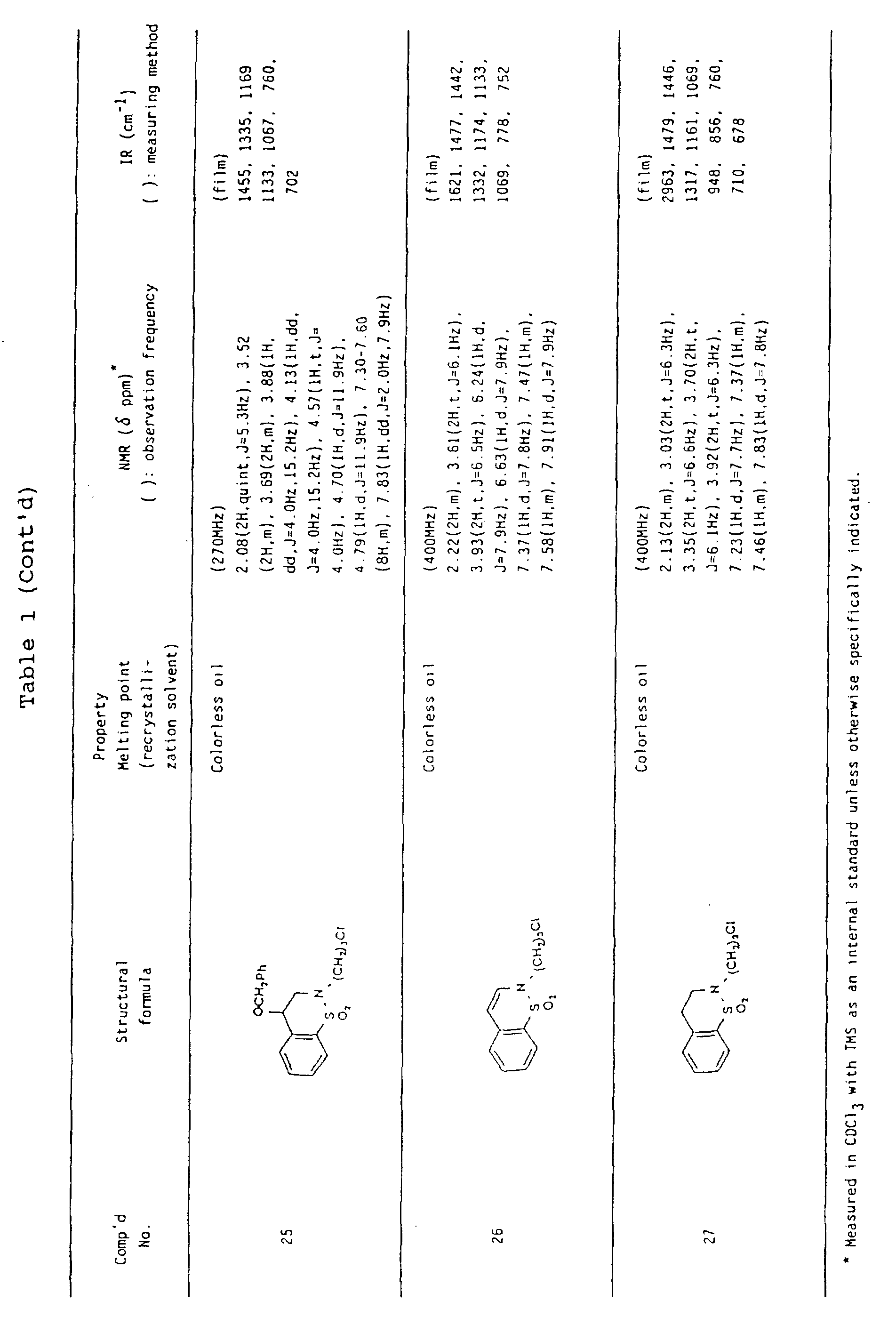

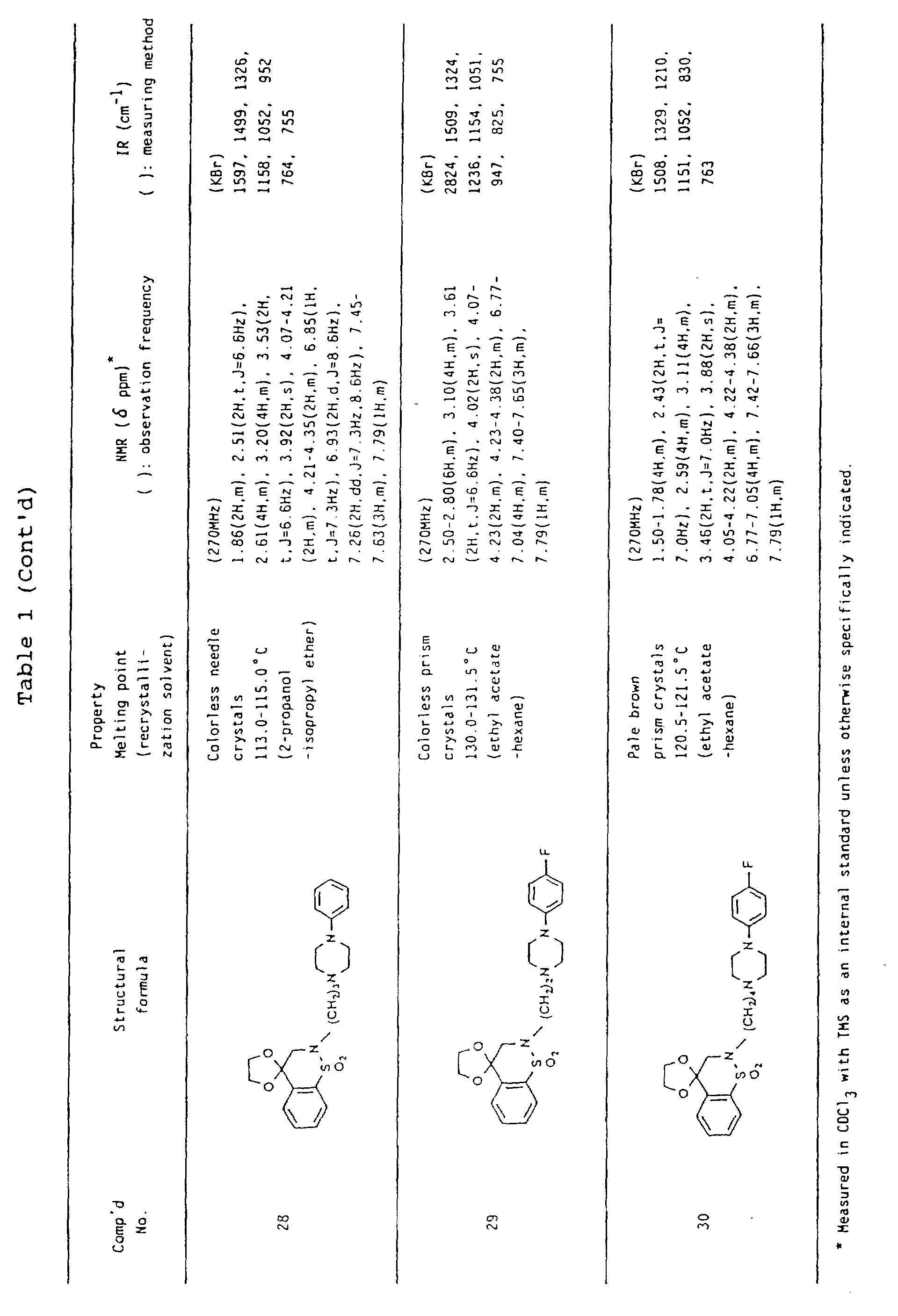

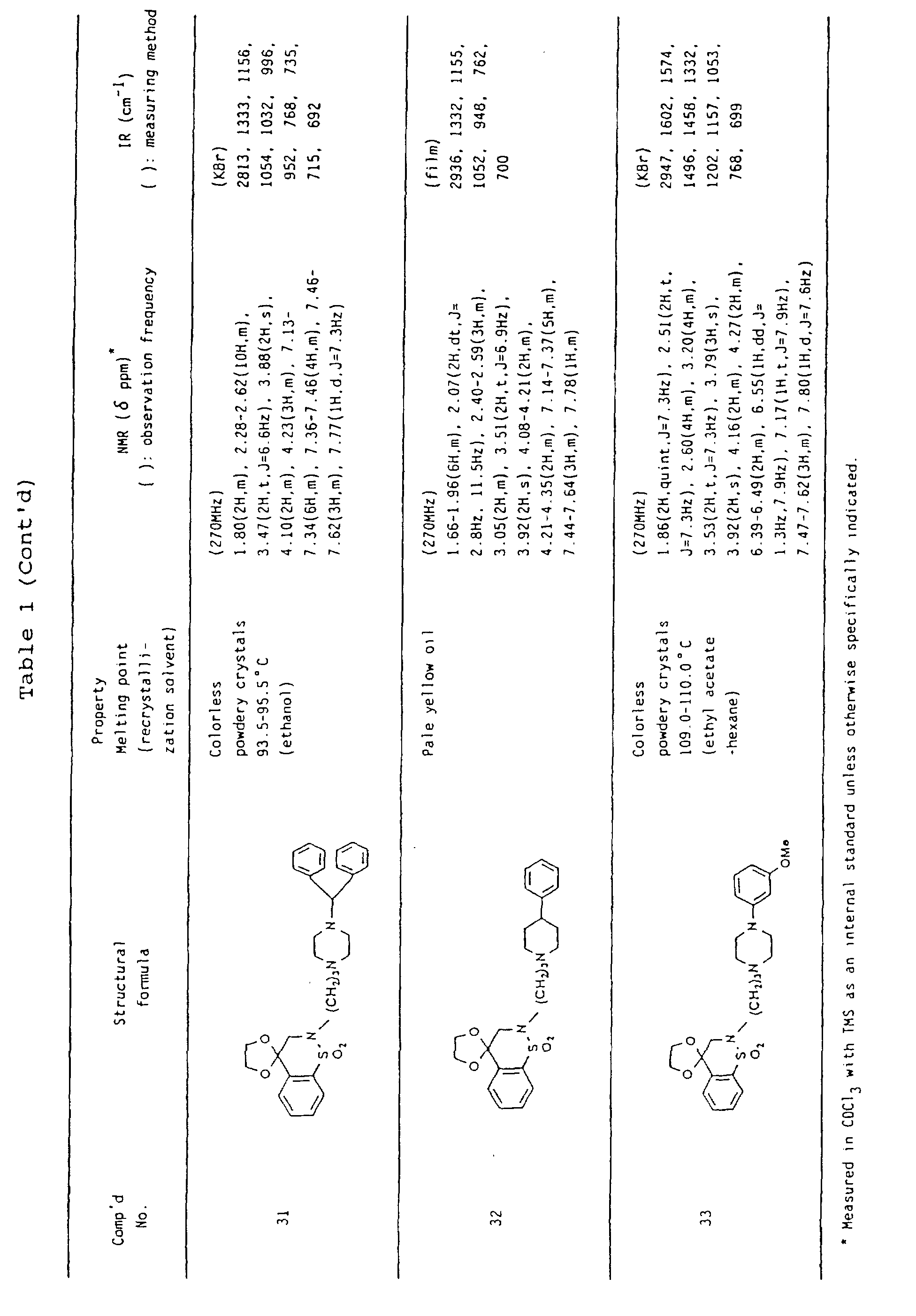

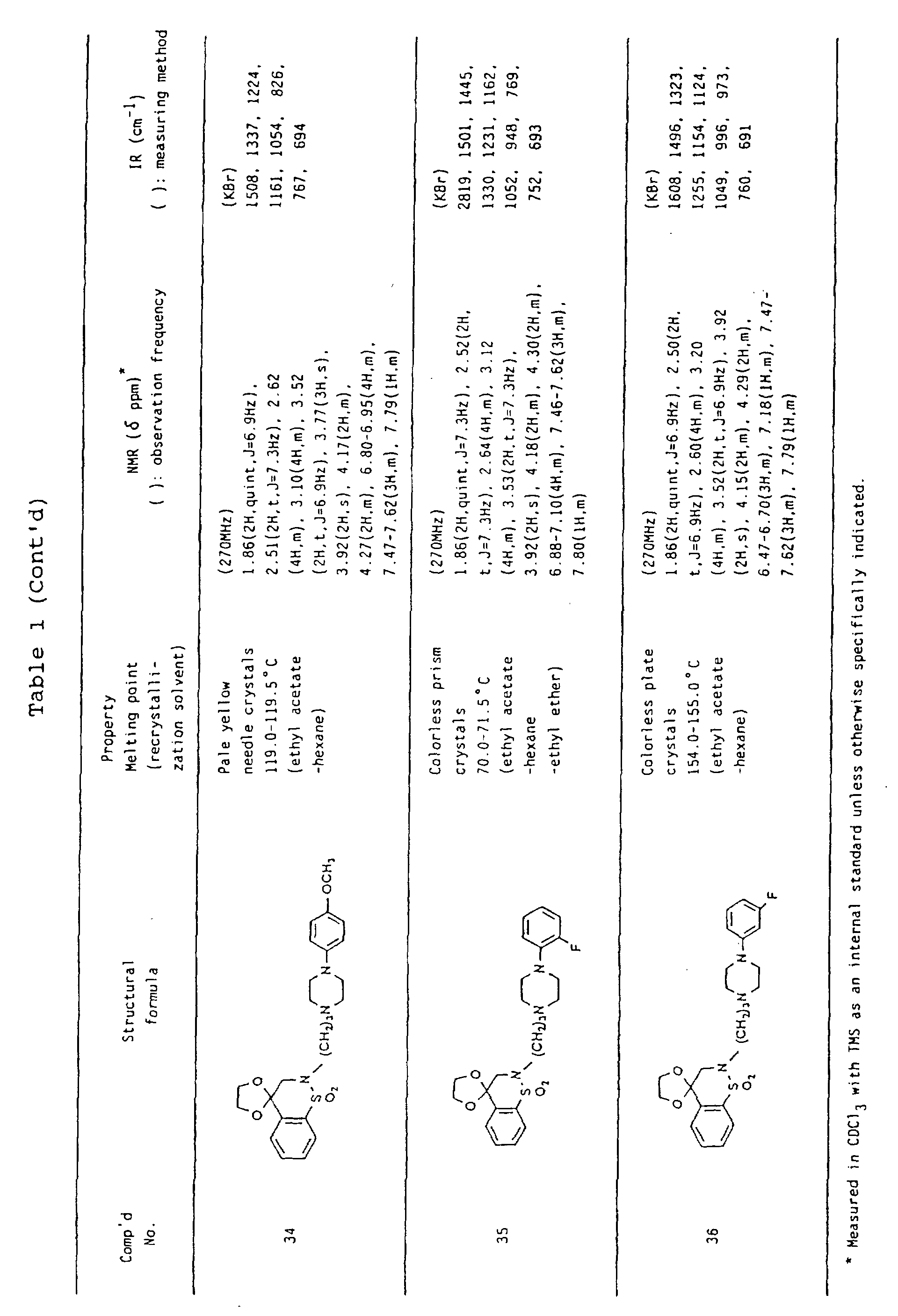

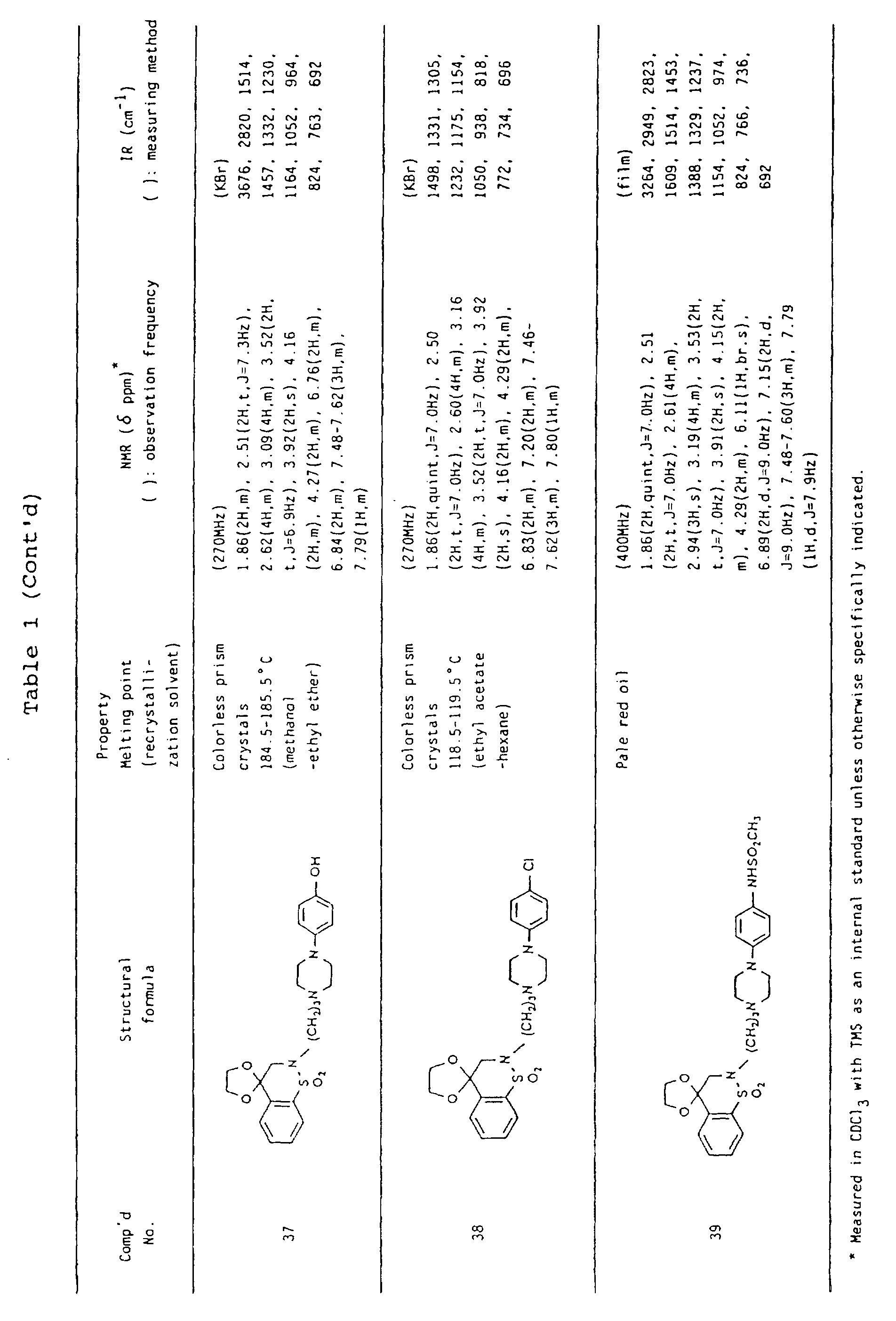

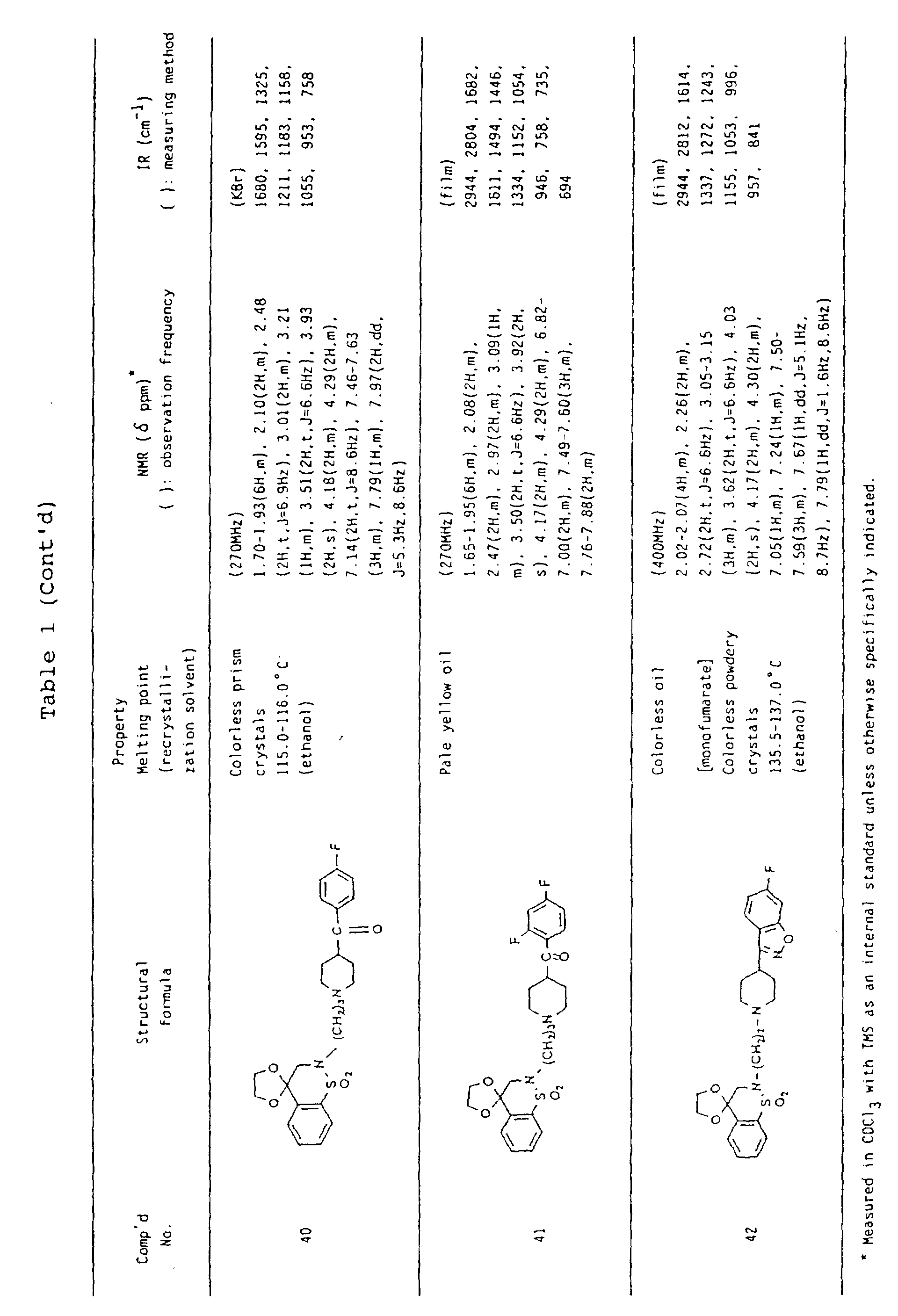

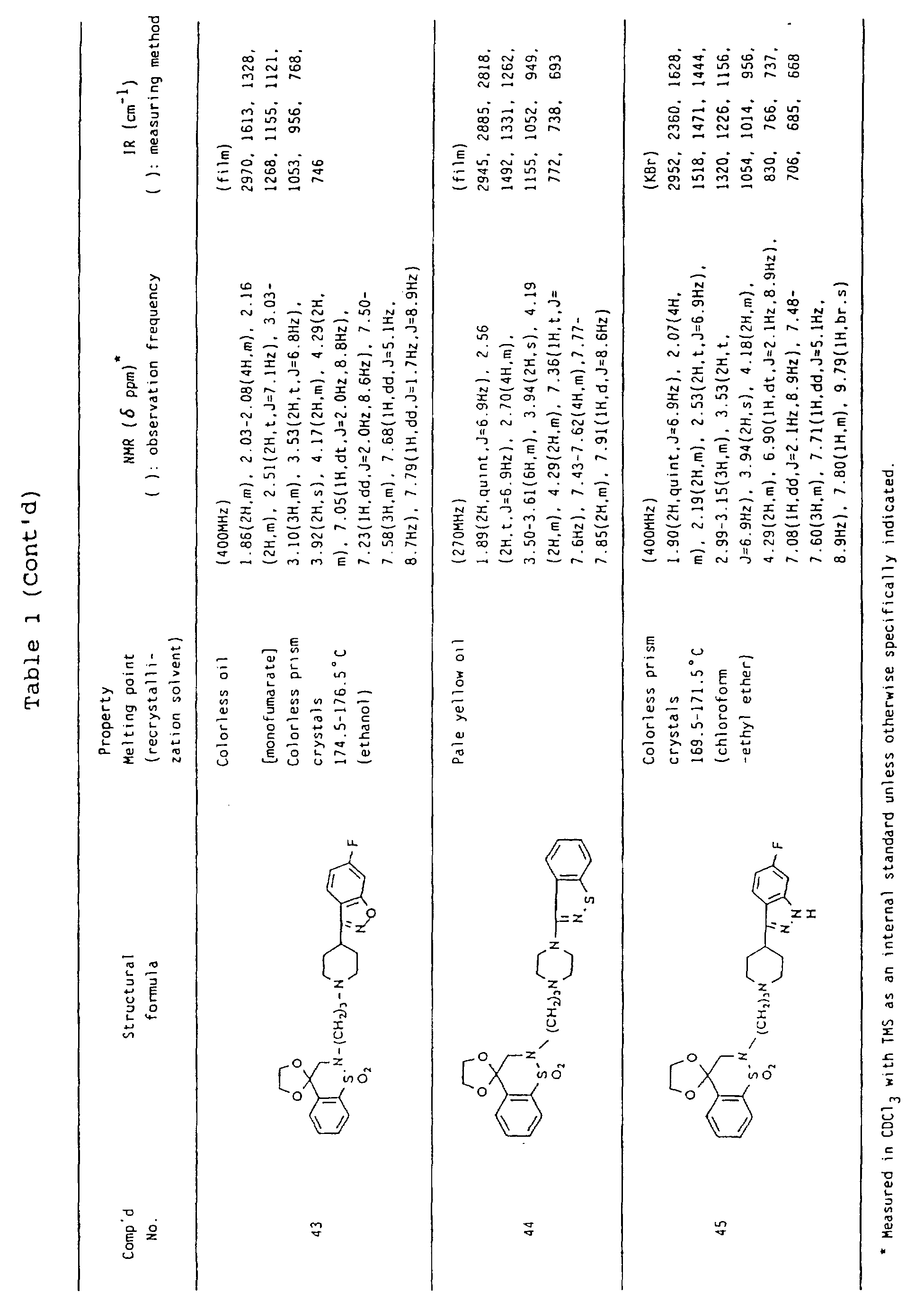

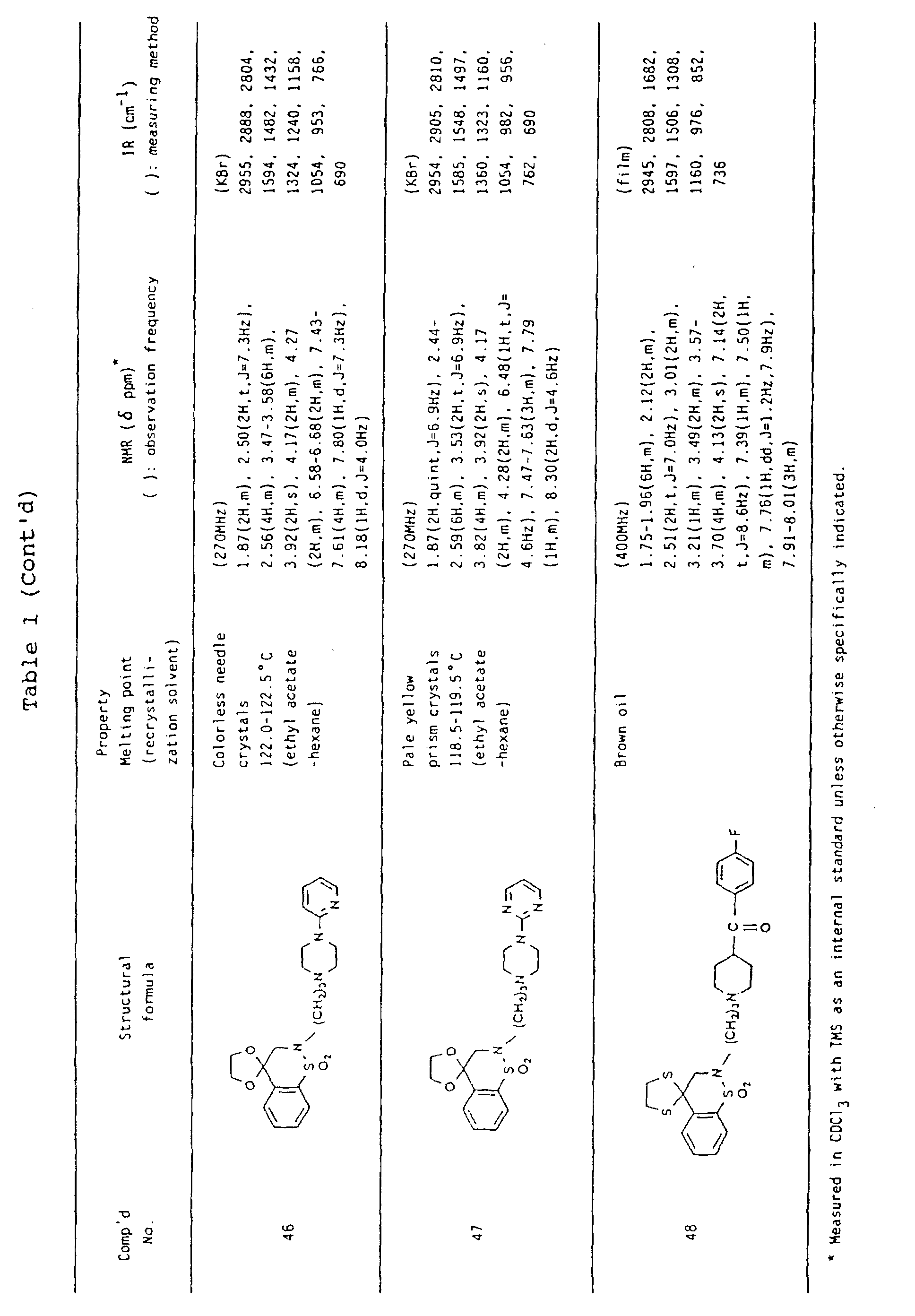

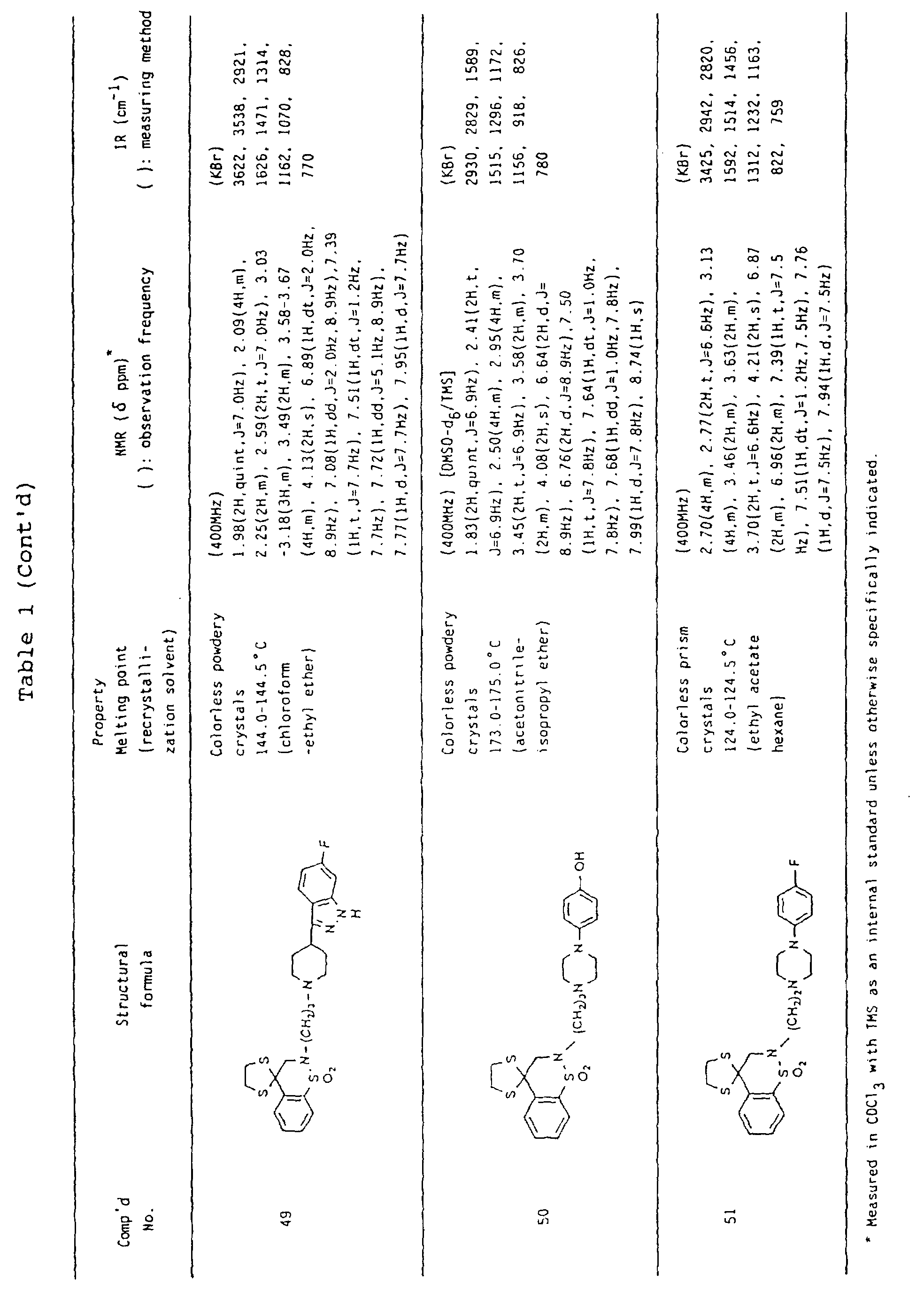

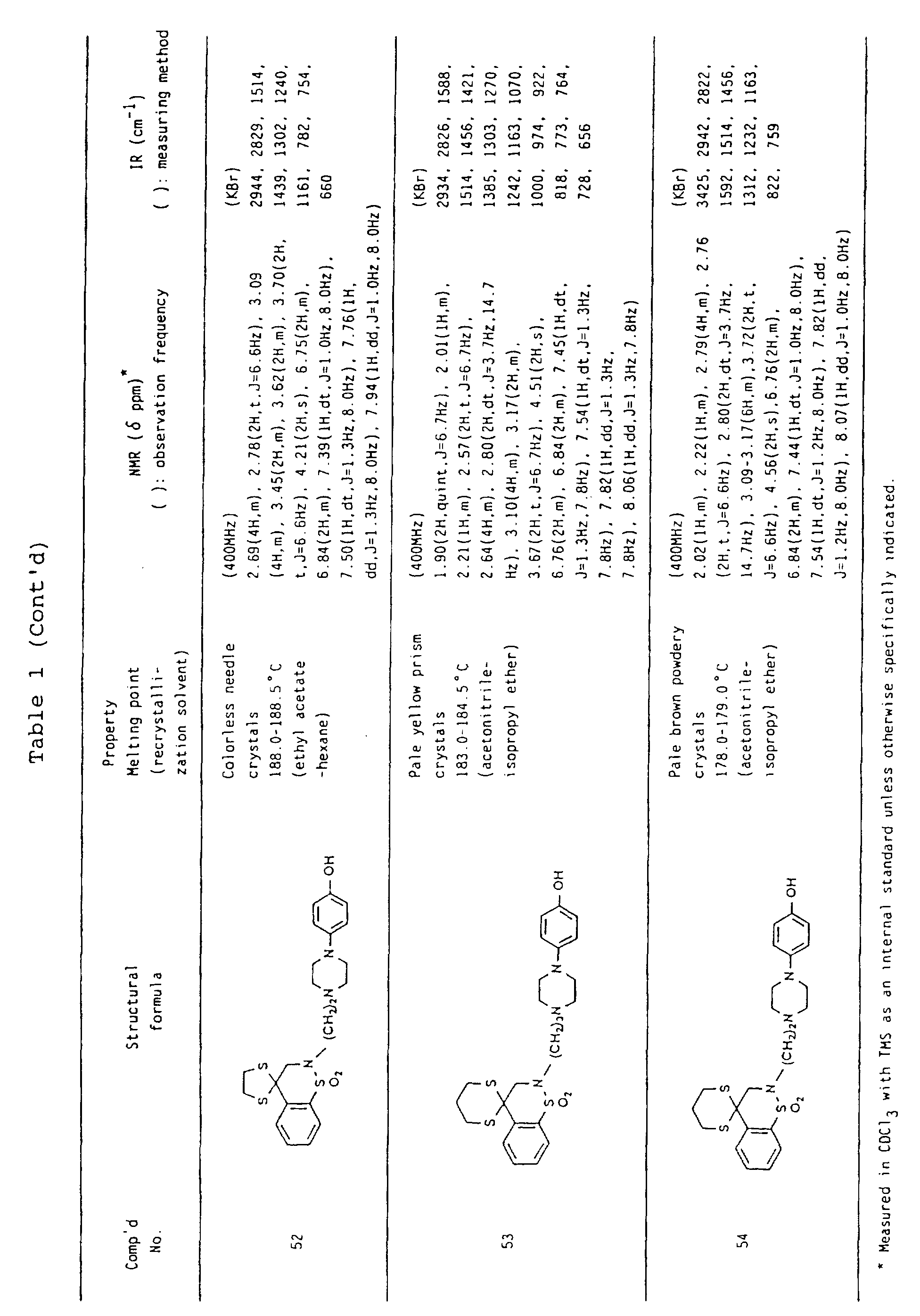

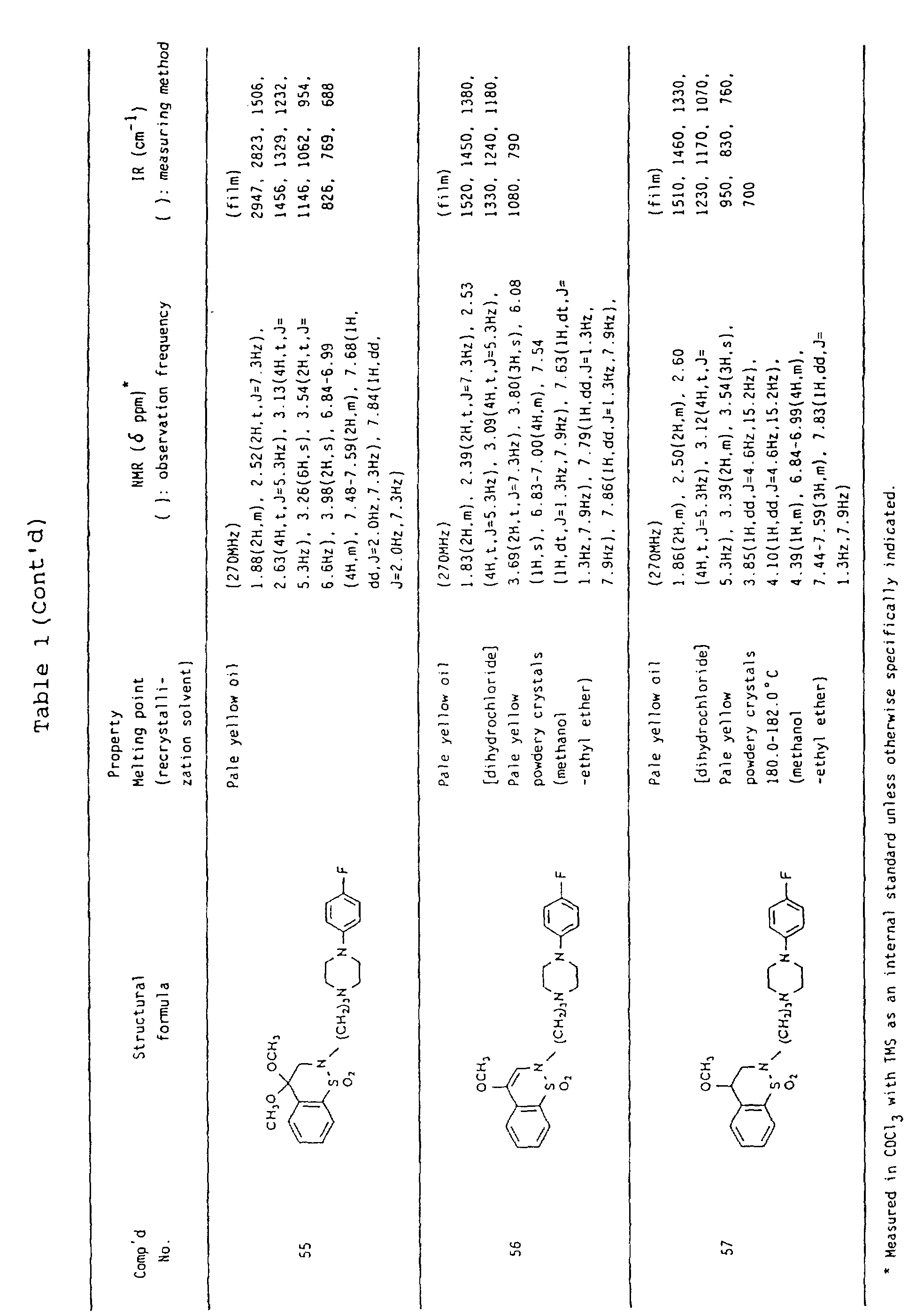

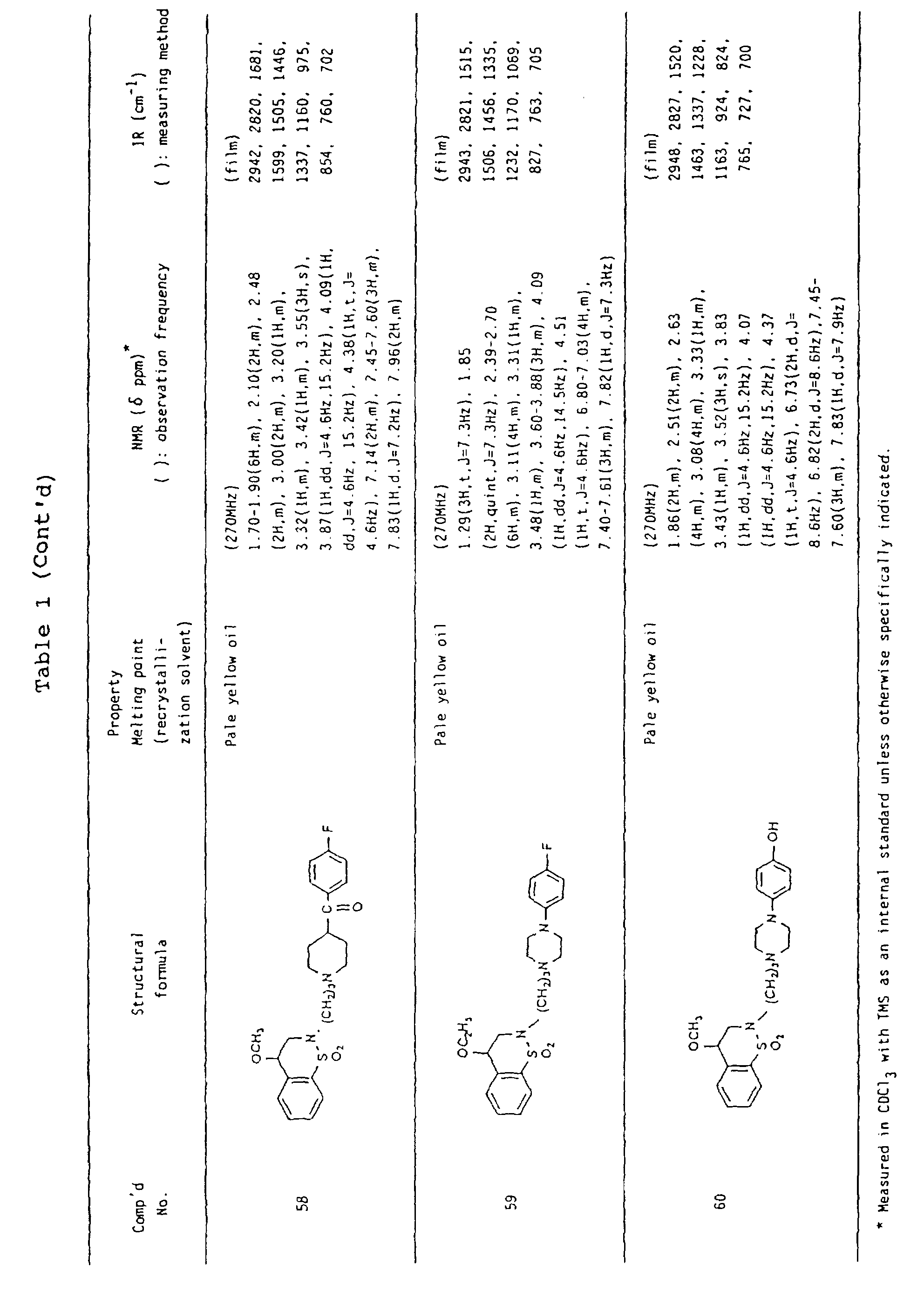

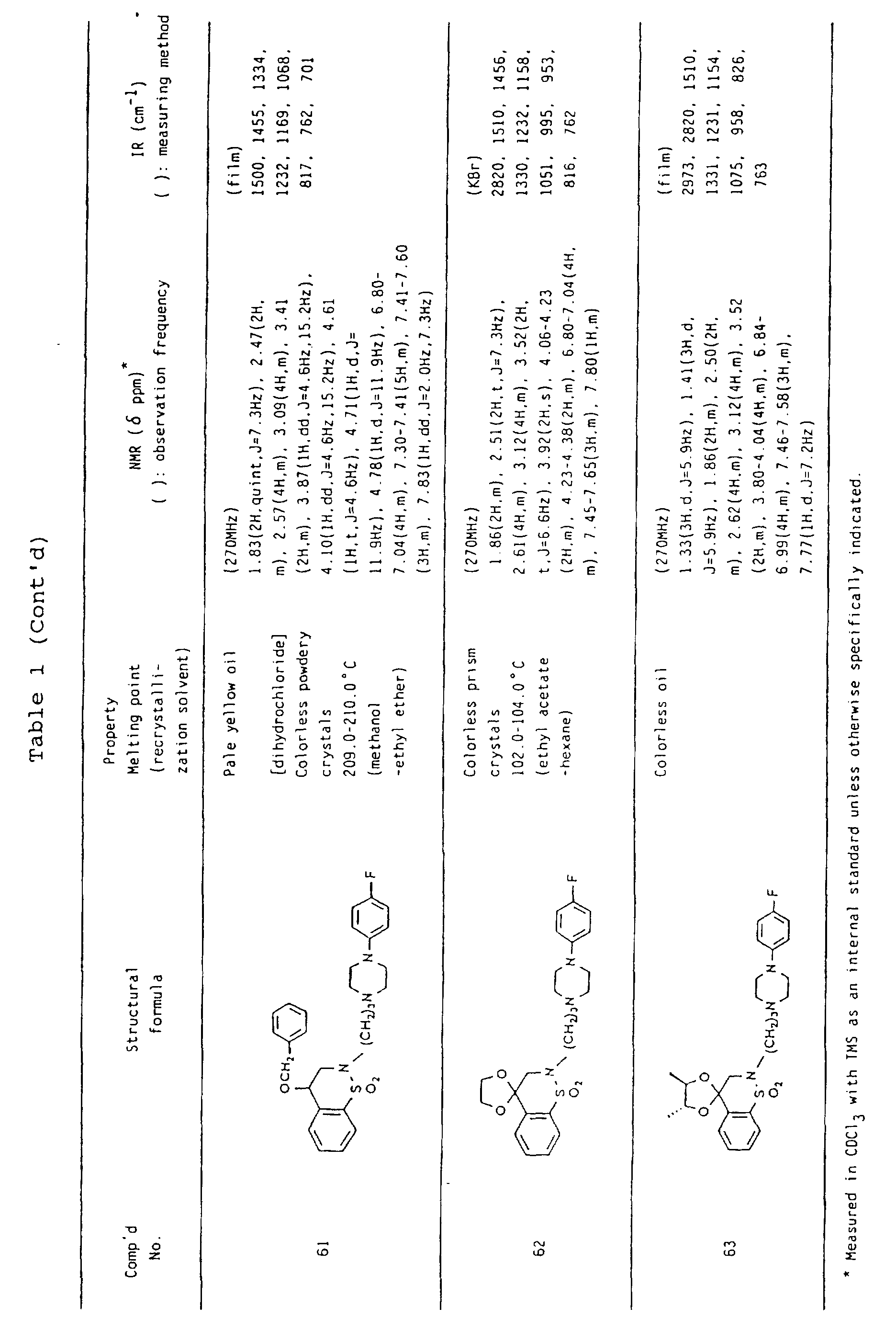

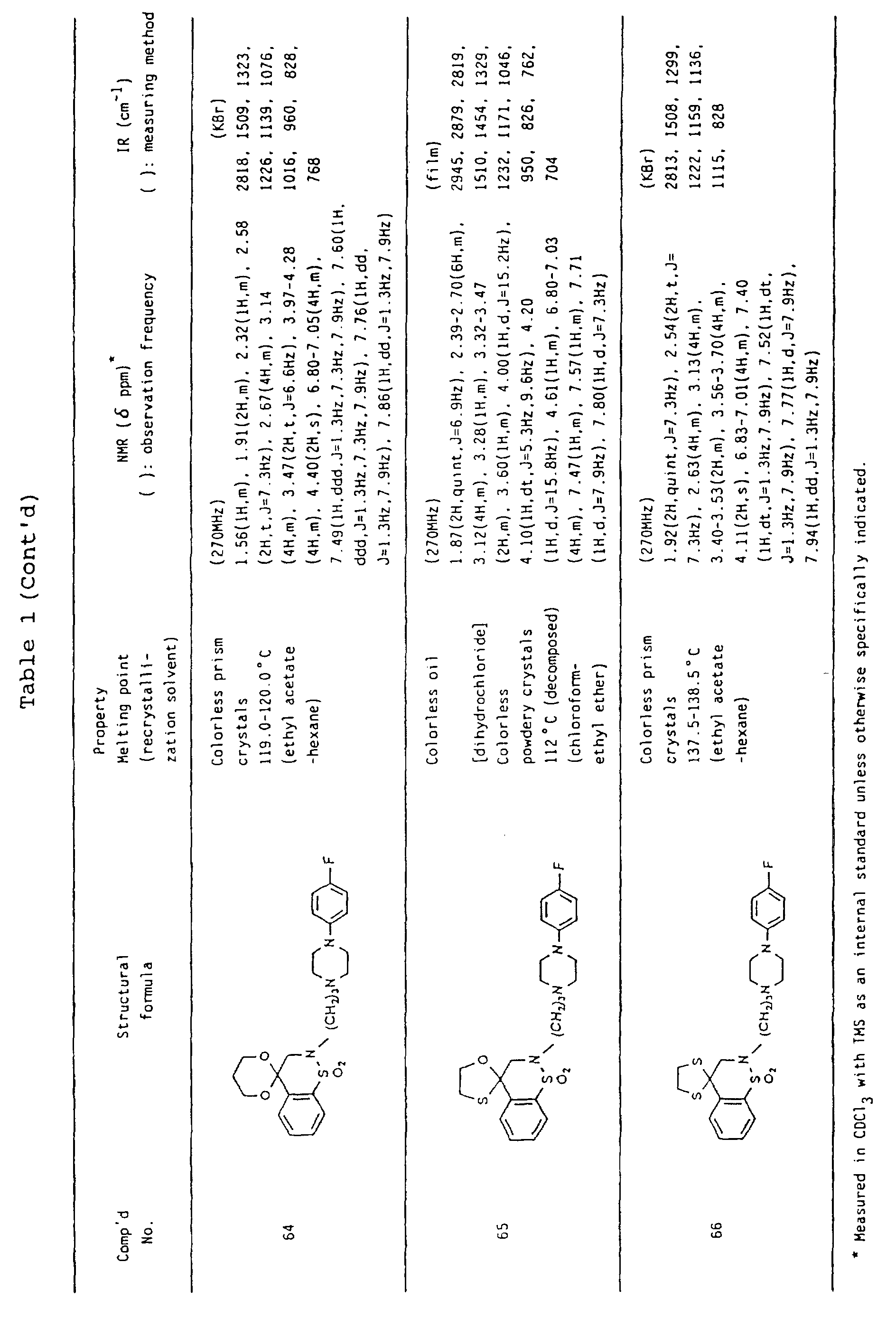

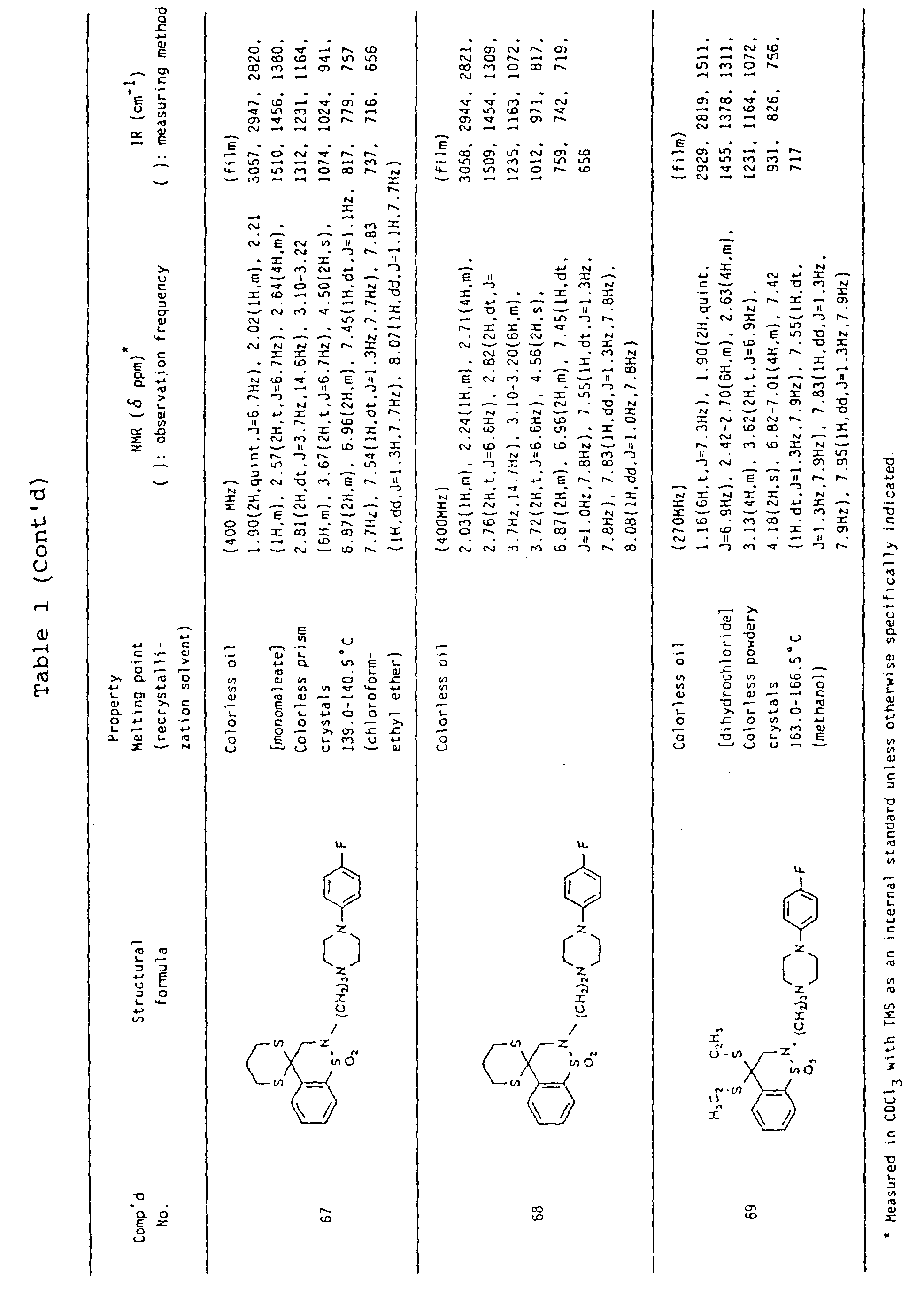

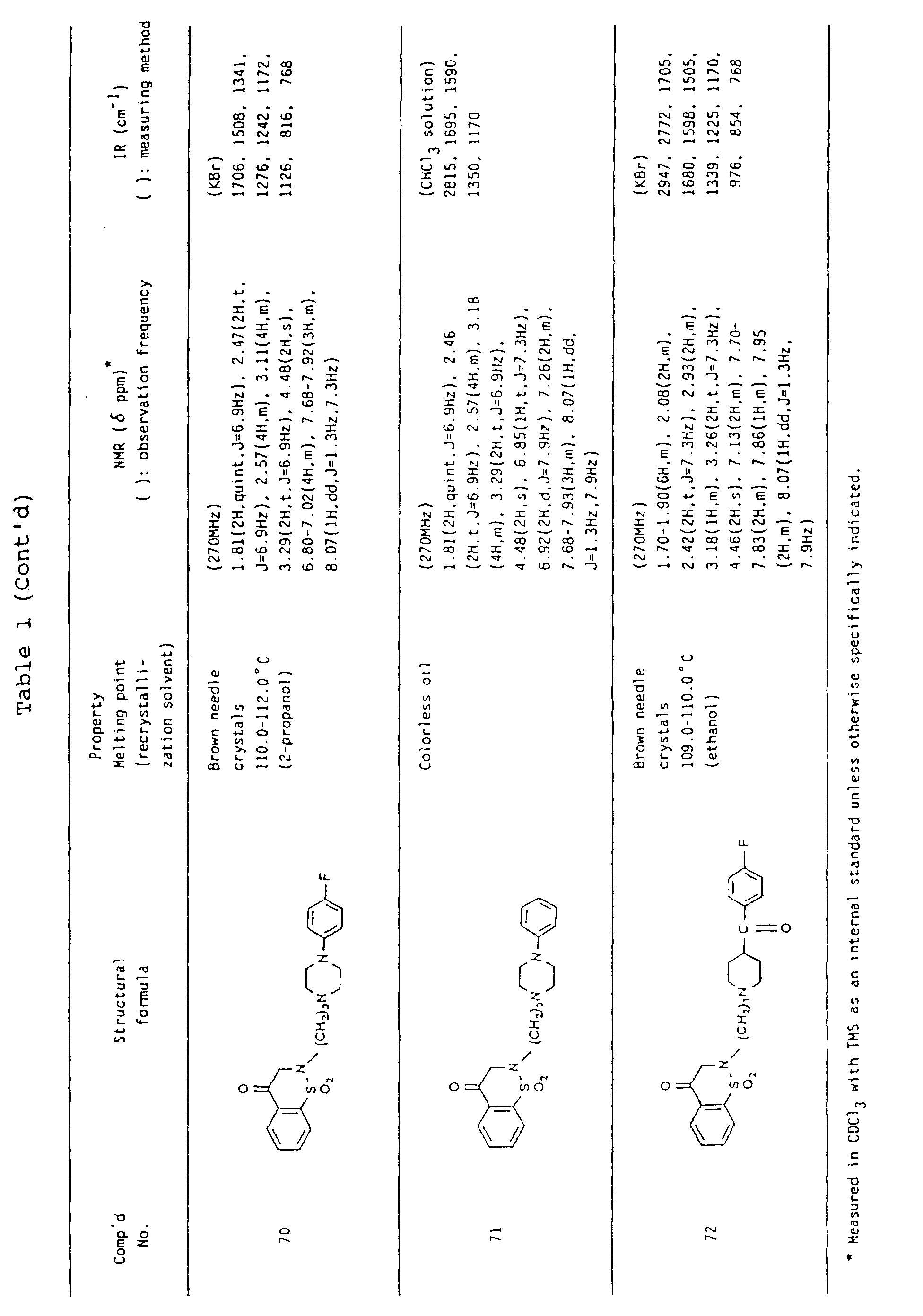

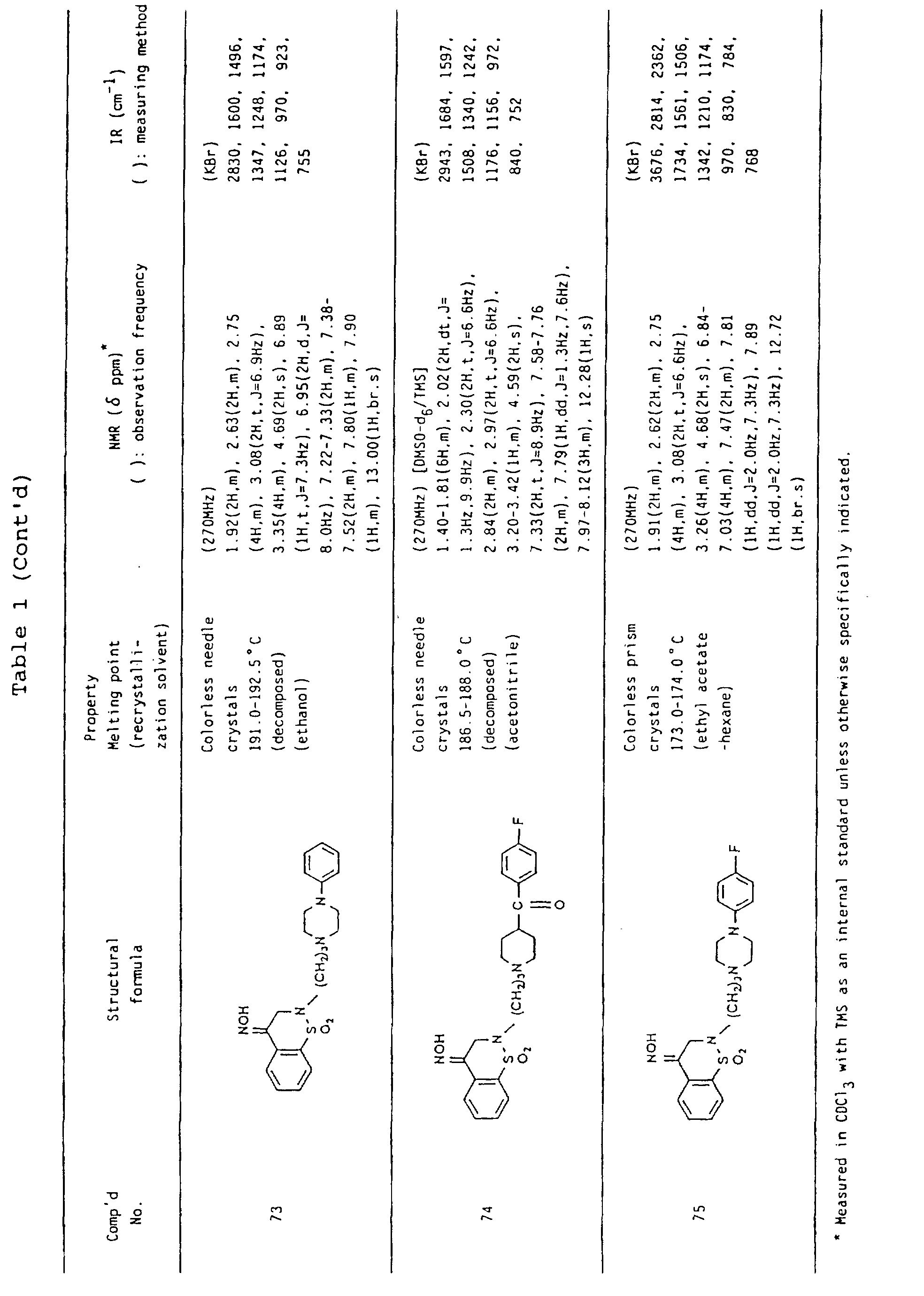

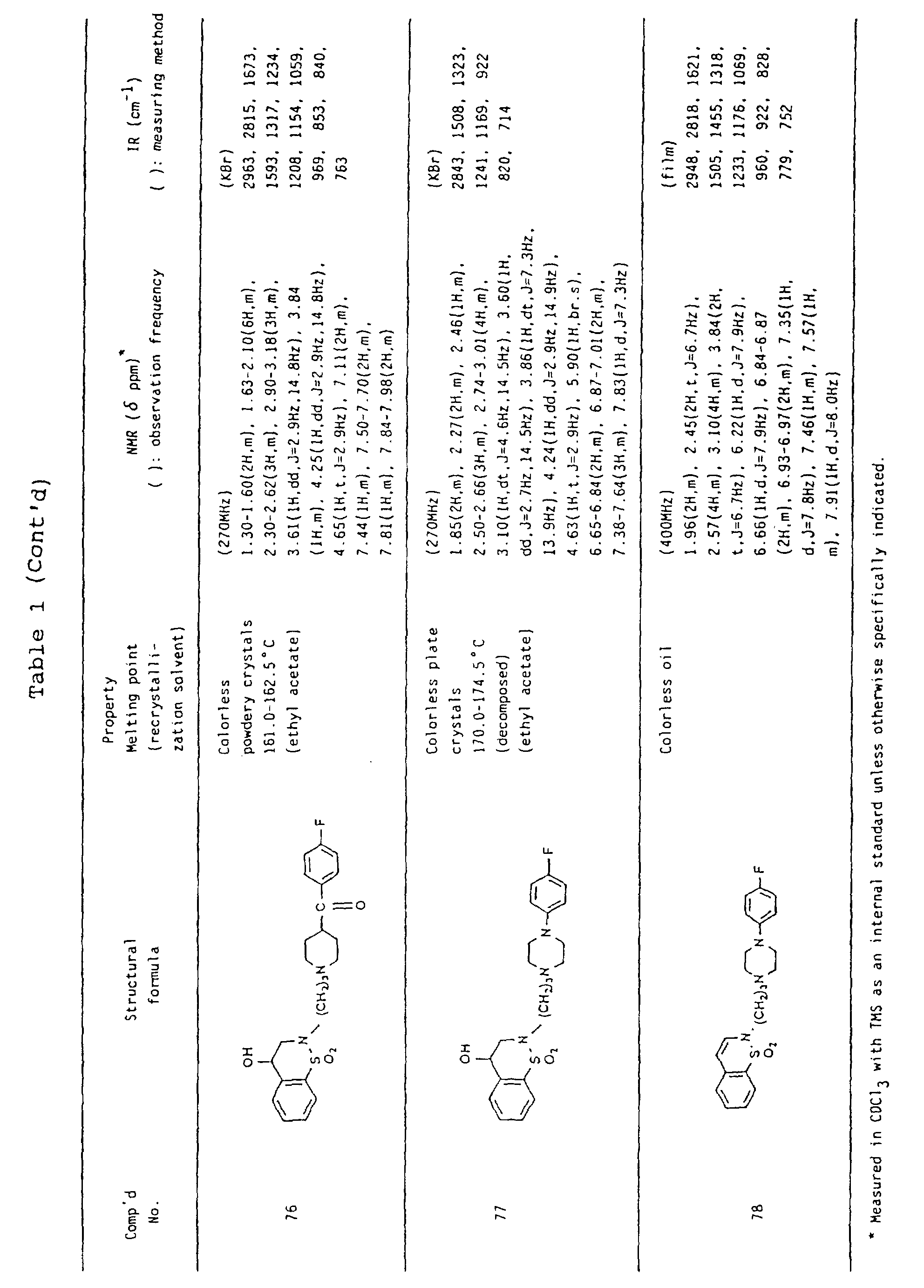

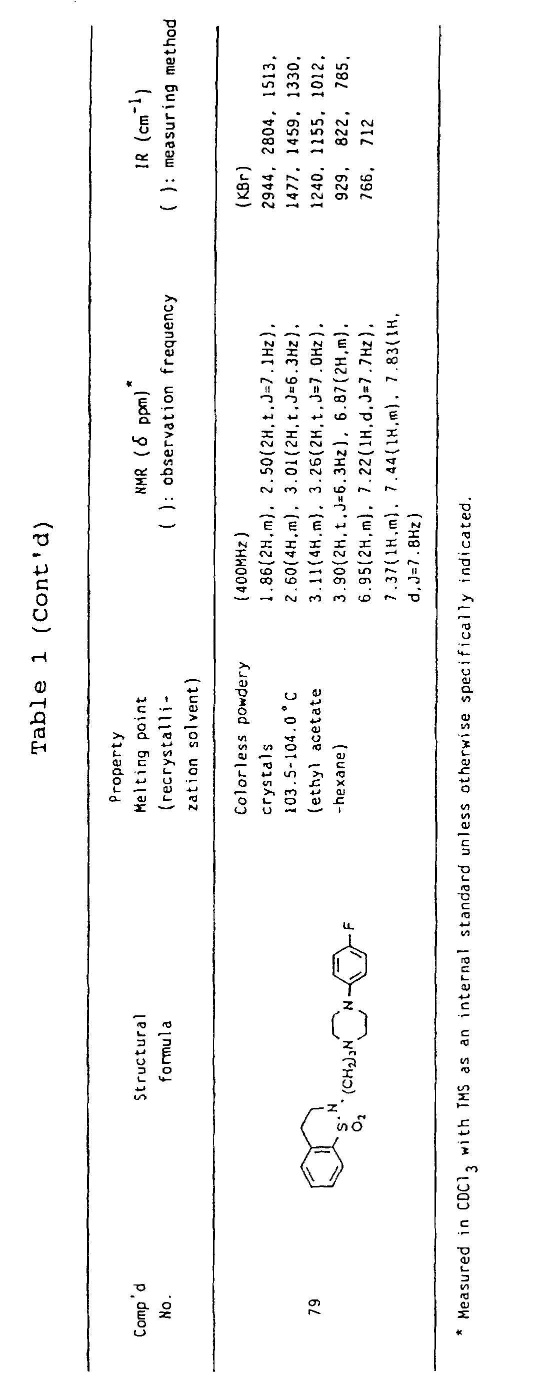

### Test

With respect to the compounds of the present invention, their anti-serotonin (5-HT) action and anti-α1 action were investigated by the methods which will be described below. The results of some representative compounds are shown in Table 2.

### (1) Anti-serotonin action (anti-5-HT action)

The superior mesenteric artery of each Hartley male guinea pig (body weight: 300-500 g) was excised. A preparation cut in a helical form was suspended under resting tension of 0.3 g in a Magnus cylinder filled with the Tyrode solution which had been aerated with a gas mixture of 95% O₂ and 5% CO₂ and maintained at 37°C. Using an isometric transducer ("UL-10", manufactured by SHINKOH H.K.) and a pressure preamplifier ("DSA-605A", manufactured by SHINKOH K.K.), variations in tension were measured. The isometric tensions were recorded on a pen-writing recorder ("VP-6537A", manufactured by NATIONAL K.K.). Taking the contraction induced by 10⁻⁵ M serotonin (5-HT) as 100%, the percent contractions in the presence of each test drug at 10⁻⁷ and 10⁻⁶ M were determined as anti-5-HT action.

### (1) Anti-α₁ action

The thoracic aorta of each Hartley male guinea pig (body weight: 300-500 g) was excised. A preparation cut in a helical form was suspended under 1 g load in a Magnus cylinder filled with the Tyrode solution which had been aerated with a gas mixture of 95% O₂ and 5% CO₂ and maintained at 37°C. Using an isometric transducer ("TB-612J", manufactured by NIHON KOHDEN) and a pressure preamplifier ("AP-620G", manufactured by NIHON KOHDEN), variations in tension were measured. The isometric tensions were recorded on a thermal pen-writing recorder ("WT-647G", manufactured by NIHON KOHDEN).

Taking the tonic contraction induced by 10⁻⁵ M norepinephrine (NE) as 100%, the percent contractions upon addition of each test drug at 10⁻⁸ and 10⁻⁷ M were determined as anti-α₁ action.

### (Results)

**Table 2**

| Comp'd No. | Anti 5-HT action (% of Control) | | Anti α ₁ action (% of Control) | |
|---|---|---|---|---|
| | 10 ⁻⁷ M | 10 ⁻⁶ M | 10 ⁻⁸ M | 10 ⁻⁷ M |
| 35 | 38.3 | 7.5 | 99.0 | 89.3 |
| 37 | 15.7 | 8.3 | 100 | 96.2 |
| 40 | 37.4 | 13.4 | 98.3 | 73.2 |
| 46 | 39.8 | 5.5 | 100 | 97.4 |
| 55 | 23.4 | 7.4 | 100 | 89.2 |
| 56* | 60.8 | 16.9 | 95.4 | 69.5 |
| 57* | 22.0 | 10.0 | 97.3 | 81.6 |
| 62 | 14.1 | 3.3 | 98.1 | 53.1 |
| 64 | 24.8 | 13.8 | 97.3 | 84.9 |
| 66 | 66.6 | 2.8 | 100 | 86.5 |
| 76 | 53.9 | 21.7 | 100 | 88.2 |
| 77 | 45.7 | 18.2 | 100 | 79.6 |

| | | | | |
|---|---|---|---|---|
| * The compound in the form of a dihydrochloride was used as the test compound. | | | | |

### Industrial Applicability

The benzothiazine derivatives (I) and their salts according to the present invention have strong serotonin-2 blocking action, have excellent selectivity to α₁ blocking action and have high safety. Accordingly, the present invention has made it possible to provide pharmaceuticals making use of antagonistic action against serotonin-2 receptors, for example, therapeutics for various circulatory diseases such as ishemic heart diseases, cerebrovascular disturbance and peripheral circulatory disturbance.

## Claims

1. A benzothiazine derivative represented by the following formula (I): 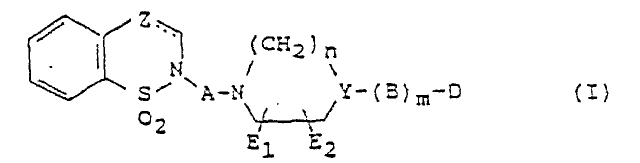 wherein the dashed line indicates the presence or absence of a bond, and when the dashed line indicates the presence of the bond, Z represents one of the following groups: 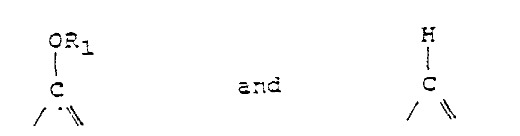
in which R₁ represents a branched or linear C₁₋₄ alkyl group which may be unsubstituted or substituted by one or more halogen atoms, and/or C₁-C₄ alkoxy groups, or a C₇₋₂₂ aralkyl group, which may be unsubstituted or substituted by one or more halogen atoms, C₁-C₄ alkyl groups and/or C₁-C₄ alkoxy groups, but when the dashed line indicates the absence of the bond, Z represents one of the following groups: 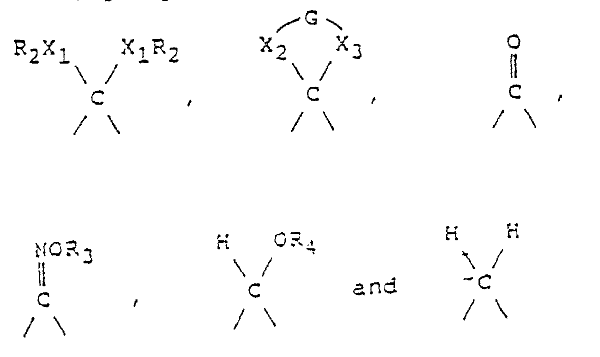
in which R₂ represents a branched or linear C₁₋₄ alkyl group, which may be unsubstituted or substituted by one or more halogen atoms, and/or C₁-C₄ alkoxy groups, a C₆₋₁₄ aryl group which may be unsubstituted or substituted by one or more halogen atoms, C₁-C₄ alkyl groups and/or C₁-C₄ alkoxy groups, or a C₇₋₂₂ aralkyl group, which may be unsubstituted or substituted by one or more halogen atoms, C₁-C₄ alkyl groups and/or C₁-C₄ alkoxy groups,
R₃ represents a hydrogen atom, a branched or linear C₁-C₄ alkyl group, which may be unsubstituted or substituted by one or more halogen atoms, and/or C₁-C₄ alkoxy groups, a C₆₋₁₄ aryl group, which may be unsubstituted or substituted by one or more halogen atoms, C₁-C₄ alkyl groups and/or C₁-C₄ alkoxy groups, or a C₇₋₂₂ aralkyl group, which may be unsubstituted or substituted by one or more halogen atoms, C₁-C₄ alkyl groups and/or C₁-C₄ alkoxy groups,
R₄ represents a hydrogen atom, a branched or linear C₁-C₄ alkyl group which may be unsubstituted or substituted by one or more halogen atoms and/or C₁-C₄ alkoxy groups, or a C₇₋₂₂ aralkyl group, which may be unsubstituted or substituted by one or more halogen atoms, C₁-C₄ alkyl groups and/or C₁-C₄ alkoxy groups,
X₁, X₂, and X₃ each independently represents an oxygen atom or a sulfur atom, G represents an ethylene group,
A represents a C₂₋₁₀ alkylene group, a C₄₋₁₀ alkenylene group or a C₄₋₁₀ alkynylene group all of which may be branched or linear and may be substituted by one or more halogen atoms;
Y represents CH or a nitrogen atom; and, when Y represents CH, m stands for 0 or 1, n stands for 1 or 2, B represents a carbonyl group,
but when Y represents a nitrogen atom, m stands for 0 or 1, n stands for 2 or 3, B represents a group -CHR₇- in which R₇ represents a C₆₋₁₄ aryl group, which may be unsubstituted or substituted by one or more halogen atoms, C₁-C₄ alkyl groups and/or C₁-C₄ alkoxy groups,
E₁ and E₂ each independently represent a hydrogen atom or a C₁-C₄ alkyl group and,
D represents a C₆₋₂₈ aromatic hydrocarbon group or a aromatic heterocyclic group, which is monocyclic or bicyclic and contains three or less oxygen, sulfur and/or nitrogen atoms, whereby the aromatic hydrocarbon or aromatic heterocyclic group may be substituted with halogen atoms, C₁₋₄ alkyl groups, C₁₋₄ alkoxyl groups, C₆₋₁₄ aryl groups, C₇₋₂₂ aralkyl groups, C₇₋₂₂ aralkyloxy groups, cyano groups; nitro groups; carboxyl groups; alkoxycarbonyl groups having a C₁₋₆ alcohol moiety; lower alkylsulfonylamino groups having a C₁₋₄ alkyl moiety; carbamoyl groups; and hydroxyl groups; wherein the substituents for the C₁₋₄ alkyl groups, the C₇₋₂₂ aralkyl groups and C₆₋₁₄ aryl groups are halogen atoms, C₁₋₄ alkyl groups and/or C₁₋₄ alkoxy groups, or a salt thereof, with the proviso that when
E₁ and E₂ represent hydrogen
n has a value of 2
Y is CH
B is methylene
m has a value of 1
Z is carbonyl,
A is an unsubstituted C₂ or C₃ alkylene bridge
D does not represent 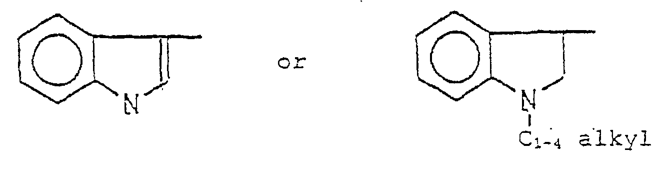
wherein the benzyl ring is unsubstituted or substituted with halogen

2. A benzothiazine derivative or a salt thereof according to claim 1, wherein in the formula (I), Z represents a group: 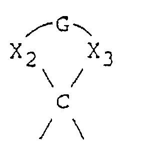 wherein G, X₂ and X₃ have the same meanings as defined above.

3. A benzothiazine derivative or a salt thereof according to claim 1, wherein in the formula (I), Z represents one of groups:

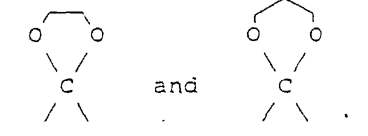

4. A benzothiazine derivative or a salt thereof according to claim 1, wherein in the formula (I), Z represents :

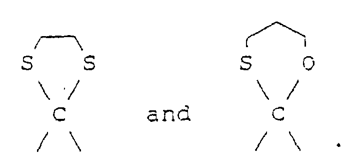

5. A benzothiazine derivative or a salt thereof according to claim 1, wherein in the formula (I), Z represents a group: 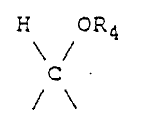 wherein R₄ has the same meaning as defined above.

6. A benzothiazine derivative or a salt thereof according to claim 1, wherein in the formula (I), Z represents a group: 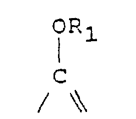 wherein R₁ has the same meaning as defined above.

7. A benzothiazine derivative or a salt thereof according to any one of claims 1, 2, 3, 4, 5 and 6,
wherein in the formula (I), A represents an ethylene or trimethylene group.

8. A benzothiazine derivative or a salt thereof according to any one of claims 1, 2, 3, 4, 5, 6 and 7,
wherein in the formula (I), Y represents CH, n stands for 2, B represents a carbonyl group, m stands for 0 or 1, and D represents a substituted or unsubstituted phenyl group.

9. A benzothiazine derivative or a salt thereof according to any one of claims 1, 2, 3, 4, 5, 6 and 7,
wherein in the formula (I), Y represents a nitrogen atom, n stands for 2, m stands for 0, and D represents a substituted or unsubstituted phenyl group.

10. A benzothiazine derivative or a salt thereof according to any one of claims 1, 2, 3, 4, 5, 6, 7, 8 and 9, wherein in the formula (I), E₁ and E₂ each represents a hydrogen atom.

11. A process for the preparation of a benzothiazine derivative, according to claim 1
wherein
Z represents a group: 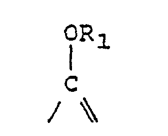 in which R₁ has the same meaning as defined above but, when the bond indicated by the dashed line is absent, Z represents one of the groups represented by the following formulas: 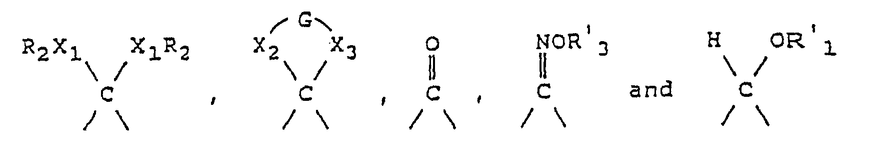 in which R'₁ represents a akyl group
which may be unsubstituted or substituted by one or more halogen atoms, and/or C₁-C₄ alkoxy groups,
R'₃ represents a alkyl group, which may be unsubstituted or substituted by one or more halogen atoms, and/or C₁-C₄ alkoxy groups, an aryl group which may be unsubstituted or substituted by one or more halogen atoms, C₁-C₄ alkyl groups and/or C₁-C₄ alkoxy groups, or a aralkyl group, which may be unsubstituted or substituted by one or more halogen atoms, C₁-C₄ alkyl groups and/or C₁-C₄ alkoxy groups,
and G, R₂, X₁, X₂ and X₃ have the same meanings as defined above,
and A, B, D, E₁, E₂, Y, m and n have the same meanings as defined above, which comprises:
reacting a compound, which is represented by the following formula (II): 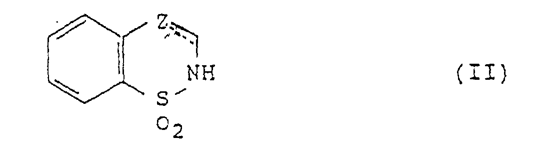
wherein Z has the same meaning as defined above, with a compound represented by the following formula (III):
W-A-W' (III)
wherein A has the same meaning as defined above and W and W' may be the same or different and individually represent a substituent easily replaceable with an amino group, to obtain a compound represented by the following formula (IV): 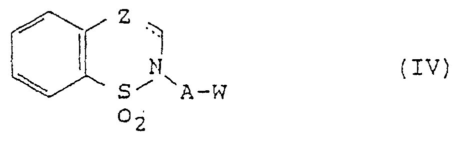 wherein A, W and Z have the same meanings as defined above; and
then reacting the compound of the formula (IV) with a nitrogen-containing compound represented by the following formula (V): 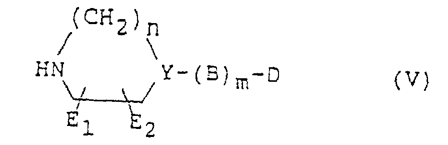 wherein B, D, E₁, E₂, Y, m and n have the same meanings as defined above.

12. A process for the preparation of a benzothiazine derivative according to claim 1 represented by the formula (I),
wherein A, B, D, E₁, E₂, Y, Z , m and n have the same meanings defined In claim 11, which comprises:
reacting a compound, which is represented by the following formula (II): 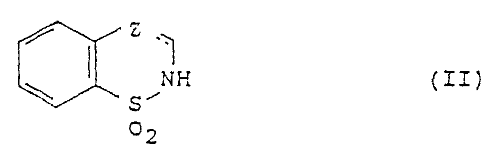
wherein Z has the same meaning as defined in claim 11 with a nitrogen-containing compound represented by the following formula (VI): 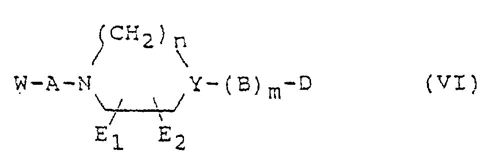 wherein A, B, D, E₁, E₂, W, Y, m and n have the same meanings as defined above.

13. A process for the preparation of a benzothiazine derivative according to claim 1 represented by the following formula (Ic): 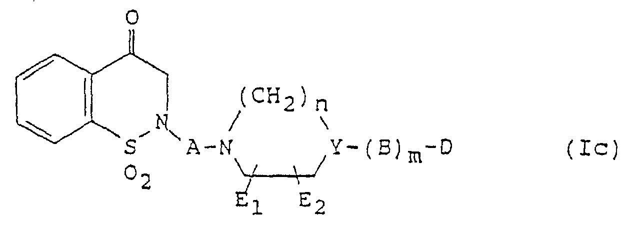 wherein A, B, D, E₁, E₂, Y, m and n have the same meanings defined above, which comprises converting Z₂ of a benzothiazine derivative to a carbonyl group, said derivative being represented by the following formula (Ib) : 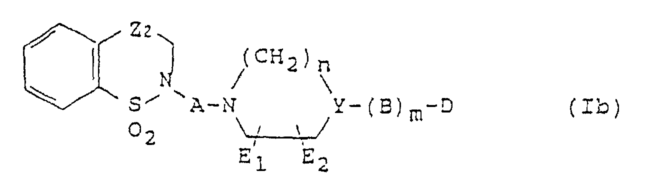 wherein Z2 represents a group: 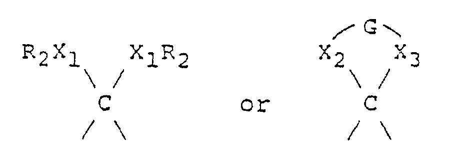 in which G, R₂, X₁, X₂ and X₃ have the same meanings as defined above, and A, B, D, E₁, E₂, Y, m and n have the same meanings as defined above.

14. A process for the preparation of a benzothiazine derivative according to claim 1 represented by the following formula (Ie): 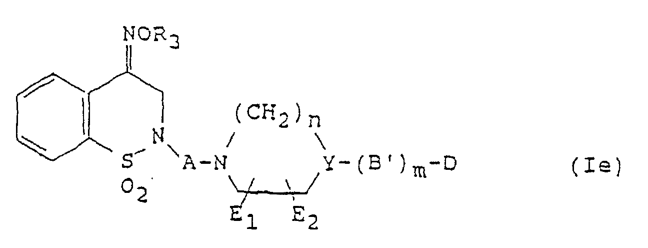 and B' has the same meaning as B, A, D, E₁, E₂, E₃, m and n have the same meanings as defined above, which comprises reacting a benzothiazine derivative, which is represented by the following formula (Id): 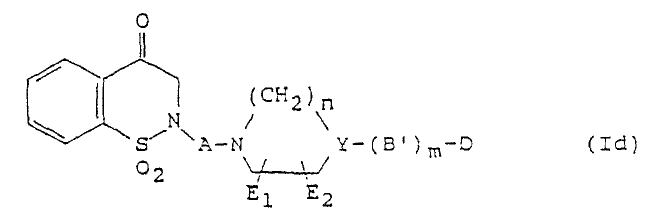 wherein A, B', D, E₁, E₂, Y, m and n have the same meanings as defined above, with a hydroxylamine represented by the following formula (VII):
NH₂OR₃ (VII)
wherein R₃ has the same meaning as defined above or with a derivative thereof.

15. A process for the preparation of a benzothiazine derivative according to claim 1 represented by the following formula (If): 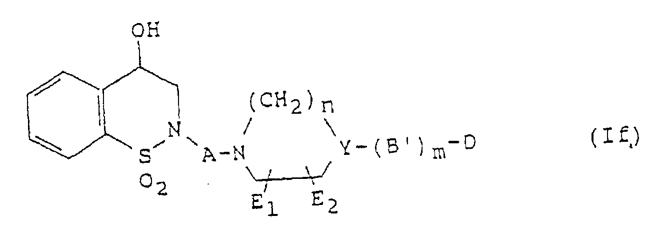 wherein A, B', D, E₁, E₂, Y, m and n have the same meanings as defined above, which comprises reducing a benzothiazine derivative represented by the following formula (Id): 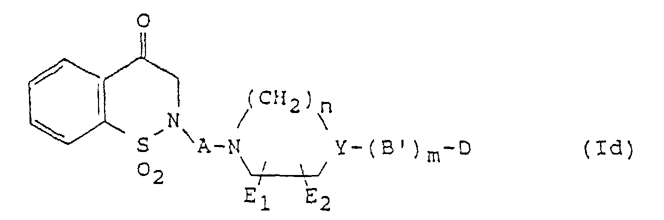 wherein A, B', D, E₁, E₂, Y, m and n have the same meanings as defined above.

16. A process for the preparation of a benzothiazine derivative according to claim 1 represented by the following formula (Ig): 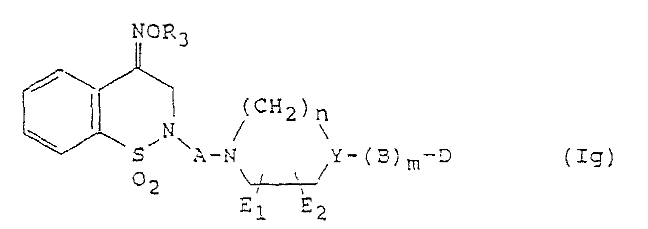 wherein A, B, D, E₁, E₂, R₃, Y, m and n have the same meanings as defined above, which comprises:
reacting a compound, which is represented by the following formula (VIII): 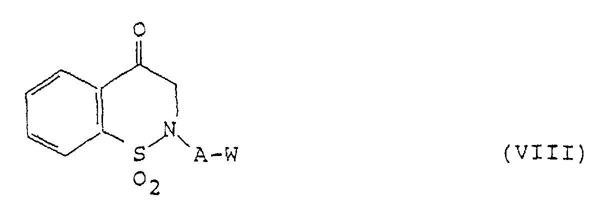
wherein A and W have the same meanings as defined above, with a hydroxylamine represented by the following formula (VII):
NH₂OR₃ (VII)
wherein R₃ has the same meaning as defined above or with a derivative thereof to obtain a compound represented by the following formula (IX): 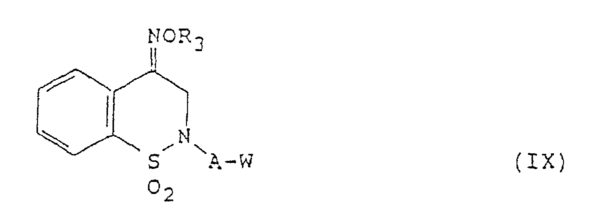 wherein A, R₃ and W have the same meanings as defined above; and then
reacting the compound of the formula (IX) with a nitrogen-containing compound represented by the following formula (V): 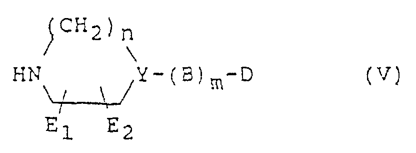 wherein B, D, E₁, E₂, Y, m and n have the same meanings as defined above.

17. A process for the preparation of a benzothiazine derivative according to claim 1 represented by the following formula (Ih): 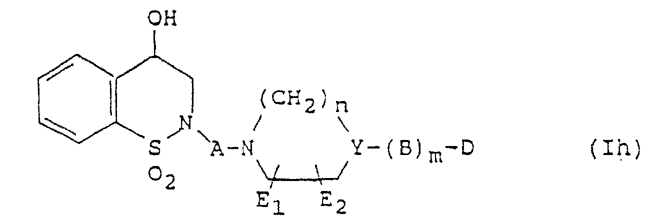 wherein A, B, D, E₁, E₂, Y, m and n have the same meanings as defined above, which comprises reducing a compound, which is represented by the following formula (VIII): 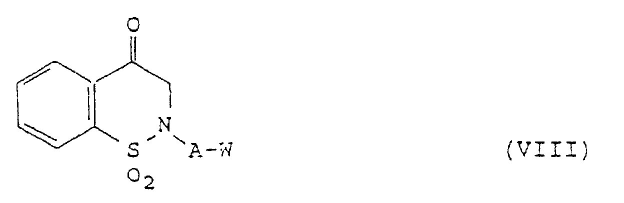 wherein A and W have the same meanings as defined above, to obtain a compound represented by the following formula a (X): 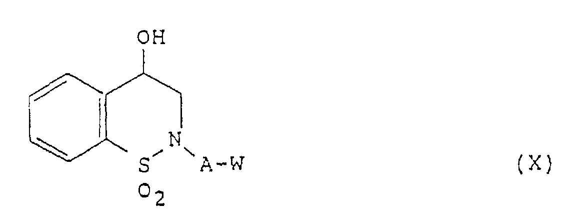 wherein A and W have the same meanings as defined above, and then
reacting the compound of the formula (X) with a nitrogen-containing compound represented by the following formula (V): 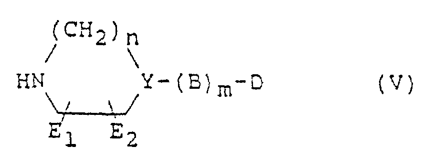 wherein B, D, E₁, E₂, Y, m and n have the same meanings as defined above.

18. A process for the preparation of a benzothiazine derivative according to claim 1 represented by the following formula (Ii): 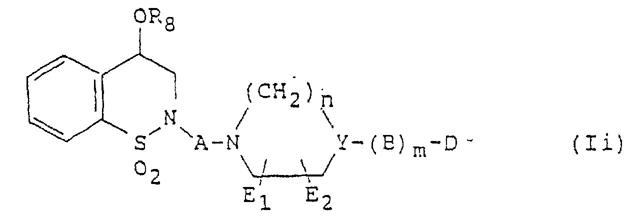 wherein R₈ represents a substituted or unsubstituted alkyl group or a substituted or unsubstituted aralkyl group, and A, B, D, E₁, E₂, Y, m and n have the same meanings as defined above, which comprises:
reacting a compound, which is represented by the following formula (X): 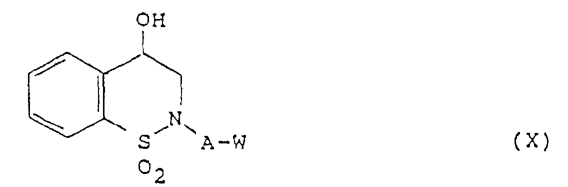
wherein A and W have the same meanings as defined above, with a compound represented by the following formula (XI) :
R₈-W" (XI)
wherein W" represents an eliminative substituent and R₈ has the same meaning as defined above, to obtain a compound represented by the following formula (XII): 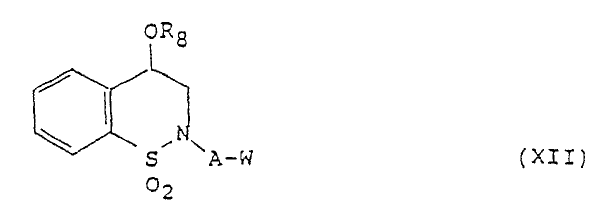 wherein A, R₈ and W have the same meanings at defined above and then,
reacting the compound of the formula (XII) with a nitrogen-containing compound represented by the following formula (V): 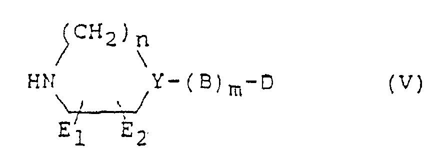 wherein B, D, E₁, E₂, Y, m and n have the same meanings as defined above.

19. A process for the preparation of a compound according to claim 1 represented by the following formula (Ij): 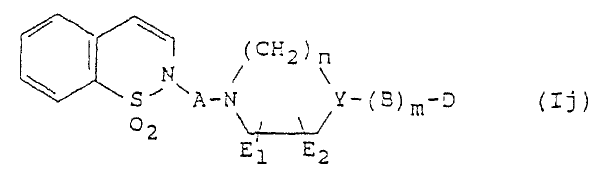 wherein A, B, D, E₁, E₂, Y, m and n have the same meanings as defined above, which comprises dehydrating a compound represented by the following formula (X): 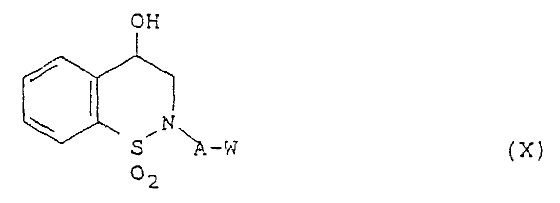 wherein A and W have the same meanings as defined above, to obtain a compound represented by the following formula (XIII): 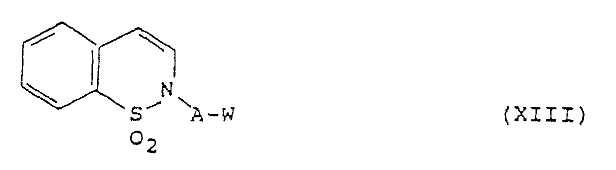 wherein A and W have the same meanings as defined above and then reacting the compound of the formula (XIII) with a nitrogen-containing compound represented by the following formula (V): 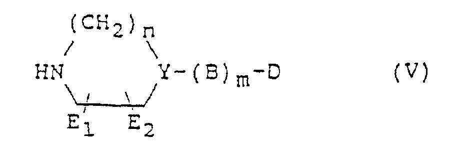 wherein B, D, E₁, E₂, Y, m and n have the same meanings as defined above.

20. A process for the preparation of a compound according to claim 1 represented by the following formula (Ik): 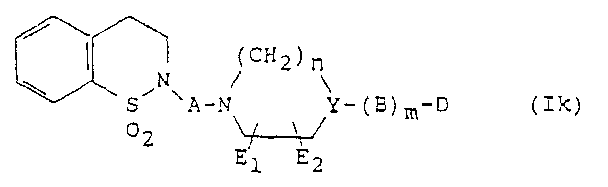 wherein A, B, D, E₁, E₂, Y, m and n have the same meanings as defined above, which comprises reducing the compound represented by the following formula (XIII): 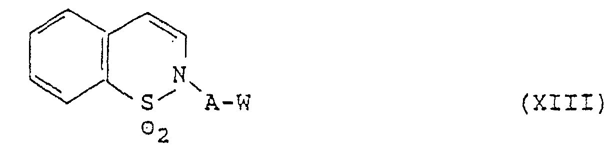 wherein A and W have the same meanings as defined above to obtain a compound represented by the following formula (XIV): 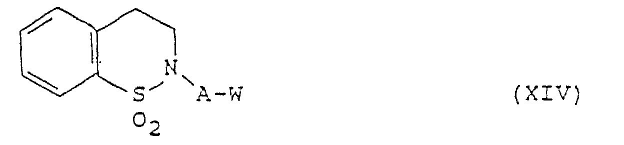 wherein A and W have the same meanings as defined above then reacting the compound of the formula (XIV) with a nitrogen-containing compound represented by the following formula (V) : 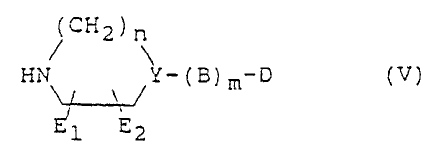 wherein B, D, E₁, E₂, Y, m and n have the same meanings as defined above.

21. A sarotonin-2 receptor antagonist, comprising as an effective ingredient a benzothiazine derivetive or a salt thereof according to claim 1.

22. A therapeutic composition comprising as an effective ingredient a benzothiazine derivative or a salt thereof according to claim 1.

23. Use of a benzothiazine derivative as defined in claims 1-10 for the preparation of a therapeutic composition for the treatment of circulatory diseases.

## Patentansprüche

1. Benzothiazinderivat, welches durch die folgende Formel (I) dargestellt ist: 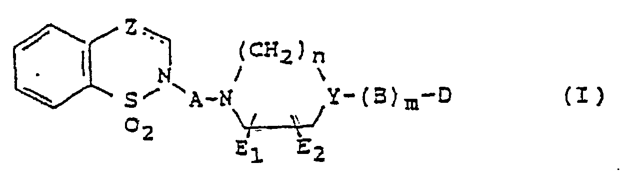 wobei die gestrichelte Linie die Anwesenheit oder Abwesenheit einer Bindung anzeigt, und
wenn die gestrichelte Linie die Anwesenheit einer Bindung anzeigt, Z einen der folgenden Reste darstellt: 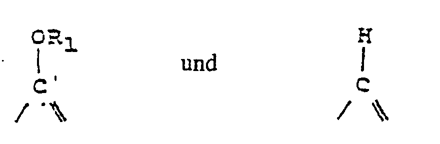 in denen R₁ einen verzweigten oder geradkettigen C₁₋₄-Alkylrest, welcher unsubstituiert oder mit einem oder mehreren Halogenatomen und/oder C₁-C₄-Alkoxyresten substituiert sein kann, oder einen C₇₋₂₂-Aralkylrest darstellt, welcher unsubstituiert oder mit einem oder mehreren Halogenatomen, C₁-C₄-Alkylresten und/oder C₁-C₄-Alkoxyresten substituiert sein kann,
wenn jedoch die gestrichelte Linie die Abwesenheit der Bindung anzeigt, Z einen der folgenden Reste darstellt: 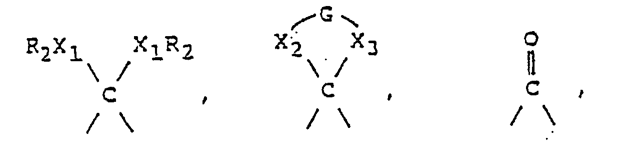 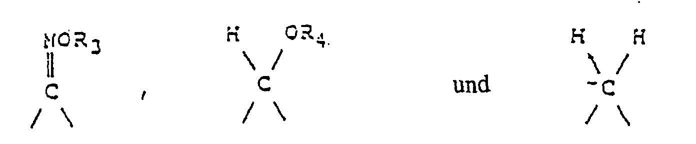
in denen R₂ einen verzweigten oder geradkettigen C₁₋₄-Alkylrest, welcher unsubstituiert oder mit einem oder mehreren Halogenatomen und/oder C₁-C₄-Alkoxyresten substituiert sein kann, einen C₆₋₁₄-Arylrest, welcher unsubstituiert oder mit einem oder mehreren Halogenatomen, C₁-C₄-Alkylresten und/oder C₁-C₄-Alkoxyresten substituiert sein kann, oder einen C₇₋₂₂-Aralkylrest darstellt, welcher unsubstituiert oder mit einem oder mehreren Halogenatomen, C₁-C₄-Alkylresten und/oder C₁-C₄-Alkoxyresten substituiert sein kann,
R₃ ein Wasserstoffatom, einen verzweigten oder geradkettigen C₁₋₄-Alkylrest, welcher unsubstituiert oder mit einem oder mehreren Halogenatomen und/oder C₁-C₄-Alkoxyresten substituiert sein kann, einen C₆₋₁₄-Arylrest, welcher unsubstituiert oder mit einem oder mehreren Halogenatomen, C₁-C₄-Alkylresten und/oder C₁-C₄-Alkoxyresten substituiert sein kann, oder einen C₇₋₂₂-Aralkylrest darstellt, welcher unsubstituiert oder mit einem oder mehreren Halogenatomen, C₁-C₄-Alkylresten und/oder C₁-C₄-Alkoxyresten substituiert sein kann,
R₄ ein Wasserstoffatom, einen verzweigten oder geradkettigen C₁₋₄-Alkylrest, welcher unsubstituiert oder mit einem oder mehreren Halogenatomen und/oder C₁-C₄-Alkoxyresten substituiert sein kann, oder einen C₇₋₂₂-Aralkylrest darstellt, welcher unsubstituiert oder mit einem oder mehreren Halogenatomen, C₁-C₄-Alkylresten und/oder C₁-C₄-Alkoxyresten substituiert sein kann,
X₁, X₂ und X₃ jeweils unabhängig ein Sauerstoff- oder Schwefelatom darstellen, G eine Ethylengruppe, A einen C₂₋₁₀-Alkylenrest, einen C₄₋₁₀-Alkenylenrest oder einen C₄₋₁₀-Alkinylenrest darstellt, wobei alle Reste verzweigt oder geradkettig und mit einem oder mehreren Halogenatomen substituiert sein können,
Y eine CH-Gruppe oder ein Stickstoffatom darstellt und, wenn Y eine CH-Gruppe darstellt, m den Wert 0 oder 1, n den Wert 1 oder 2 hat und B einen Carbonylrest darstellt, wenn jedoch Y ein Stickstoffatom darstellt, m den Wert 0 oder 1, n den Wert 2 oder 3 hat, B einen -CHR₇-Rest bedeutet, in welchem R₇ einen C₆₋₁₄-Arylrest darstellt, welcher unsubstituiert oder mit einem oder mehreren Halogenatomen, C₁-C₄-Alkylresten und/oder C₁-C₄-Alkoxyresten substituiert sein kann,
E₁ und E₂ jeweils unabhängig ein Wasserstoffatom oder einen C₁-C₄-Alkylrest darstellen und
D einen aromatischen C₆₋₂₈-Kohlenwasserstoffrest oder einen aromatischen heterocyclischen Rest darstellt, welcher monocyclisch oder bicyclisch ist und drei oder weniger Sauerstoff-, Schwefel- und/oder Stickstoffatome enthält, wobei der aromatische Kohlenwasserstoffrest oder der aromatische heterocyclische Rest mit Halogenatomen, C₁₋₄-Alkyl-, C₁₋₄-Alkoxyl-, C₆₋₁₄-Aryl-, C₇₋₂₂-Aralkyl-, C₇₋₂₂-Aralkyloxyresten, Cyano-, Nitrogruppen, Carboxylresten, Alkoxycarbonylresten mit einer C₁₋₆-Alkoholeinheit, Niederalkylsulfonylaminoresten mit einer C₁₋₄-Alkyleinheit, Carbamoylresten und Hydroxylgruppen substituiert sein können, wobei die Substituenten für die C₁₋₄-Alkylreste, die C₇₋₂₂-Aralkylreste und C₆₋₁₄-Arylreste Halogenatome, C₁₋₄-Alkylreste und/oder C₁₋₄-Alkoxyreste sind, oder ein Salz davon, mit der Maßgabe, dass wenn
E₁ und E₂ ein Wasserstoffatom darstellen,
n den Wert 2 hat,
Y eine CH-Gruppe bedeutet,
B eine Methylengruppe ist,
m den Wert 1 hat,
Z ein Carbonylrest ist,
A eine unsubstituierte C₂-oder C₃-Alkylenbrücke ist,
D nicht 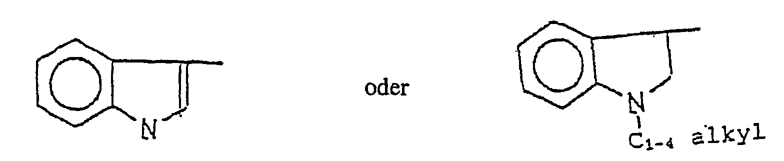 darstellt, wobei der Benzylring unsubstituiert oder mit einem Halogenatom substituiert ist.

2. Benzothiazinderivat oder ein Salz davon gemäß Anspruch 1, wobei in der Formel (I) Z einen Rest 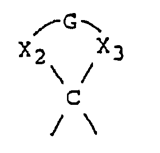 darstellt, wobei G, X₂ und X₃ wie vorstehend definiert sind.

3. Benzothiazinderivat oder ein Salz davon gemäß Anspruch 1, wobei in der Formel (I) Z einen der Reste 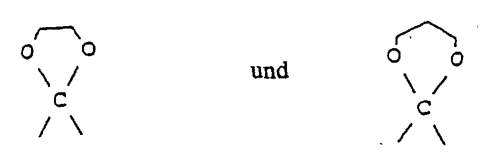 darstellt.

4. Benzothiazinderivat oder ein Salz davon gemäß Anspruch 1, wobei in der Formel (I) Z einen der Reste 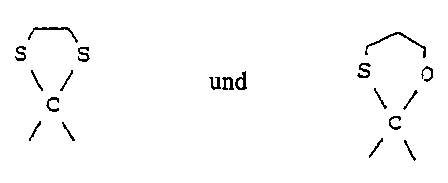 darstellt.

5. Benzothiazinderivat oder ein Salz davon gemäß Anspruch 1, wobei in der Formel (I) Z einen Rest 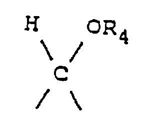 darstellt, wobei R₄ wie vorstehend definiert ist.

6. Benzothiazinderivat oder ein Salz davon gemäß Anspruch 1, wobei in der Formel (I) Z einen Rest 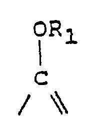 darstellt, wobei R₁ wie vorstehend definiert ist.

7. Benzothiazinderivat oder ein Salz davon gemäß einem der Ansprüche 1, 2, 3, 4, 5 und 6, wobei in der Formel (I) A eine Ethylen- oder Trimethylengruppe darstellt.

8. Benzothiazinderivat oder ein Salz davon gemäß einem der Ansprüche 1, 2, 3, 4, 5, 6, und 7, wobei in der Formel (I) Y eine CH-Gruppe darstellt, n den Wert 2 hat, B einen Carbonylrest darstellt, m den Wert 0 oder 1 hat und D einen substituierten oder unsubstituierten Phenylrest darstellt.

9. Benzothiazinderivat oder ein Salz davon gemäß einem der Ansprüche 1, 2, 3, 4, 5, 6 und 7, wobei in der Formel (I) Y ein Stickstoffatom darstellt, n den Wert 2, m den Wert 0 hat und D einen substituierten oder unsubstituierten Phenylrest darstellt.

10. Benzothiazinderivat oder ein Salz davon gemäß einem der Ansprüche 1, 2, 3, 4, 5, 6, 7, 8 und 9, wobei in der Formel (I) E₁ und E₂ jeweils ein Wasserstoffatom darstellen.

11. Verfahren zur Herstellung eines Benzothiazinderivats gemäß Anspruch 1,
wobei
Z einen Rest 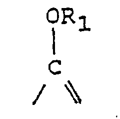 darstellt, in dem R₁ wie vorstehend definiert ist, aber, wenn die durch die gestrichelte Linie angezeigte Bindung nicht vorhanden ist, Z einen der Reste, dargestellt durch die folgenden Formeln 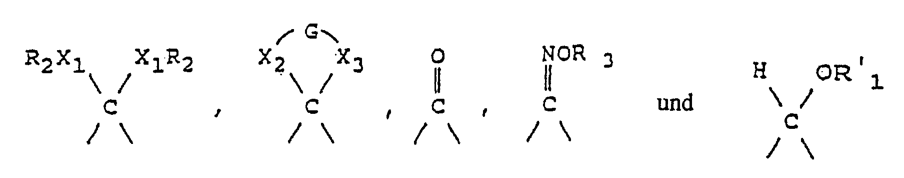 darstellt, in denen R' ₁ einen Alkylrest darstellt,
welcher unsubstituiert oder mit einem oder mehreren Halogenatomen und/oder C₁-C₄-Alkoxyresten substituiert sein kann,
R'₃ einen Alkylrest, welcher unsubstituiert oder mit einem oder mehreren Halogenatomen und/oder C₁-C₄-Alkoxyresten substituiert sein kann, einen Arylrest, welcher unsubstituiert oder mit einem oder mehreren Halogenatomen, C₁-C₄-Alkylresten und/oder C₁-C₄-Alkoxyresten substituiert sein kann, oder einen Aralkylrest darstellt, welcher unsubstituiert oder mit einem oder mehreren Halogenatomen, C₁-C₄-Alkylresten und/oder C₁-C₄-Alkoxyresten substituiert sein kann,
und G, R₂, X₁, X₂ und X₃ wie vorstehend definiert sind,
und A, B, D, E₁, E₂, Y, m und n wie vorstehend definiert sind, welches umfasst:
Umsetzen einer Verbindung, welche durch die folgende Formel (II) dargestellt ist: 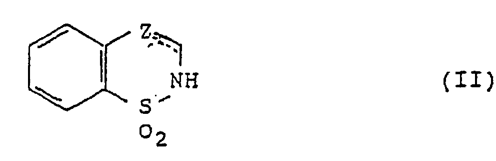
wobei Z wie vorstehend definiert ist, mit einer Verbindung, welche durch die folgende Formel (III) dargestellt ist:
W-A-W' (III)
wobei A wie vorstehend definiert ist und W und W' gleich oder verschieden sein können und jeweils einen leicht durch eine Aminogruppe ersetzbaren Substituenten darstellen, um eine Verbindung zu erhalten, welche durch die folgende Formel (IV) dargestellt ist: 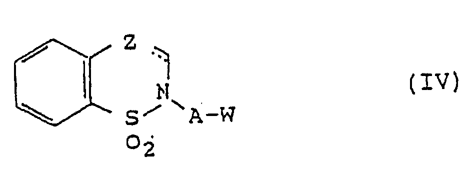 wobei A, W und Z wie vorstehend definiert sind, und
anschließendes Umsetzen der Verbindung der Formel (IV) mit einer stickstoffhaltigen Verbindung, welche durch die folgende Formel (V) dargestellt ist: 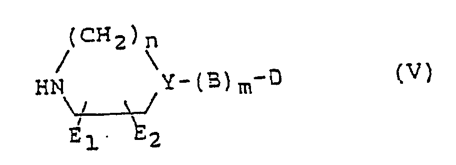 wobei B, D, E₁ E₂, Y, m und n wie vorstehend definiert sind.

12. Verfahren zur Herstellung eines Benzothiazinderivats gemäß Anspruch 1, welches durch die Formel (I) dargestellt ist,
wobei A, B, D, E₁, E₂, Y, Z, m und n wie in Anspruch 11 definiert sind, welches umfasst:
Umsetzen einer Verbindung, welche durch die folgende Formel (II) dargestellt ist: 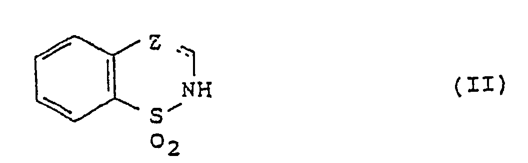
wobei Z wie in Anspruch 11 definiert ist, mit einer stickstoffhaltigen Verbindung, welche durch die folgende Formel (VI) dargestellt ist: 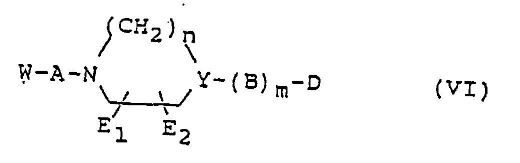 wobei A, B, D, E₁, E₂, W, Y, m und n wie vorstehend definiert sind.

13. Verfahren zur Herstellung eines Benzothiazinderivats gemäß Anspruch 1, welches durch die folgende Formel (Ic) dargestellt ist: 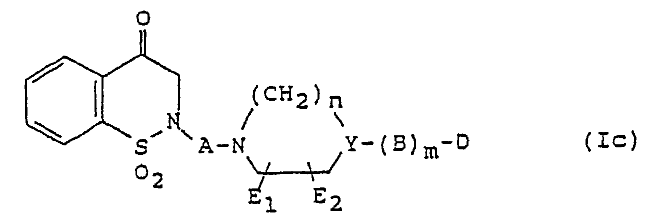 wobei A, B, D, E₁, E₂, Y, m und n wie vorstehend definiert sind, welches das Umwandeln des Restes Z₂ eines Benzothiazinderivats in einen Carbonylrest umfasst,
wobei das Derivat durch die folgende Formel (Ib) dargestellt ist: 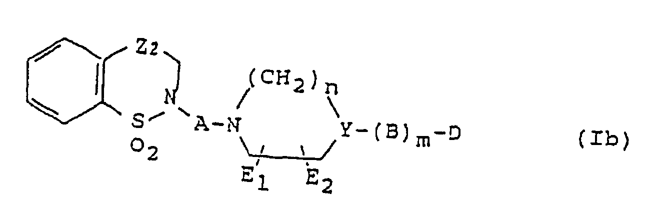 wobei Z₂ einen Rest 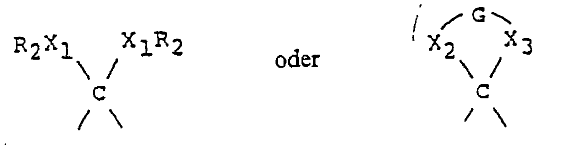 darstellt, in dem G, R₂, X₁, X₂ und X₃ wie vorstehend definiert sind und A, B, D, E₁, E₂, Y, m und n wie vorstehend definiert sind.

14. Verfahren zur Herstellung eines Benzothiazinderivats gemäß Anspruch 1, welches durch die folgende Formel (Ie) dargestellt ist: 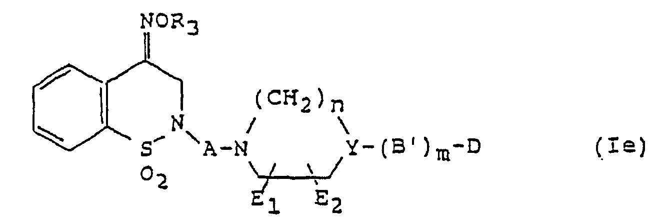 und B' die gleiche Bedeutung wie B hat, A, D, E₁, E₂, E₃, m und n wie vorstehend definiert sind, umfassend das Umsetzen eines Benzothiazinderivats, welches durch die folgende Formel (Id) dargestellt ist 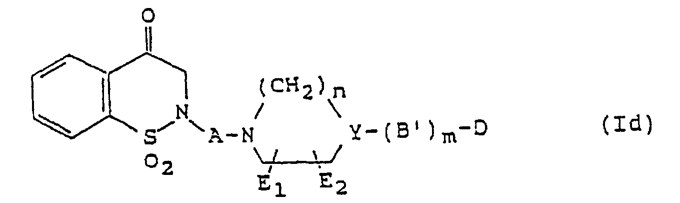 wobei A, B', D, E₁, E₂, Y, m und n wie vorstehend definiert sind, mit einem Hydroxylamin, welches durch die folgende Formel (VII) dargestellt ist:
NH₂OR₃ (VII)
wobei R₃ wie vorstehend definiert ist, oder mit einem Derivat davon.

15. Verfahren zur Herstellung eines Benzothiazinderivats gemäß Anspruch 1, welches durch die folgende Formel (If) dargestellt ist: 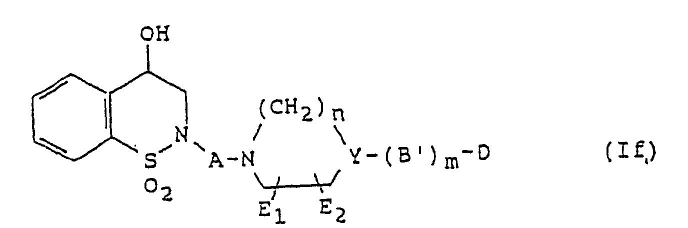 wobei A, B', D, E₁, E₂, Y, m und n wie vorstehend definiert sind, welches das Reduzieren eines Benzothiazinderivats, welches durch die folgende Formel (Id) dargestellt ist 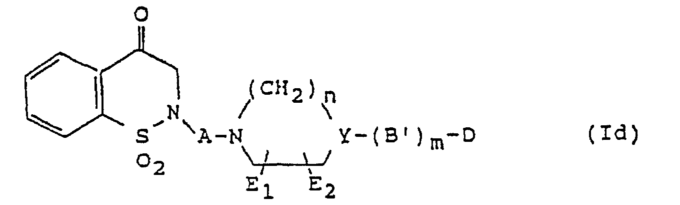 umfasst, wobei A, B', D, E₁, E₂, Y, m und n wie vorstehend definiert sind.

16. Verfahren zur Herstellung eines Benzothiazinderivats gemäß Anspruch 1, welches durch die folgende Formel (Ig) dargestellt ist: 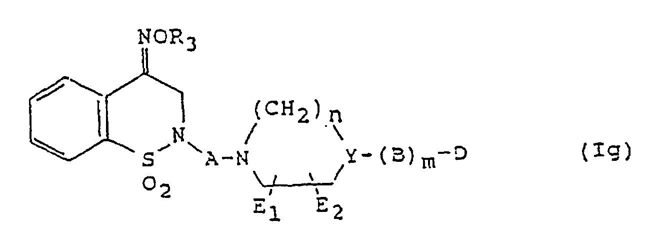 wobei A, B, D, E₁, E₂, R₃, Y, m und n wie vorstehend definiert sind, welches umfasst:
Umsetzen einer Verbindung, welche durch die folgende Formel (VIII) dargestellt ist: 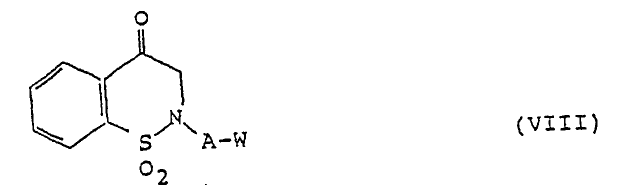
wobei A und W wie oben definiert sind, mit einem Hydroxylamin, welches durch die folgende Formel (VII) dargestellt ist:
NH₂OR₃ (VII)
wobei R₃ wie vorstehend definiert ist, oder mit einem Derivat davon, um eine Verbindung, welche durch folgende Formel (IX) dargestellt ist 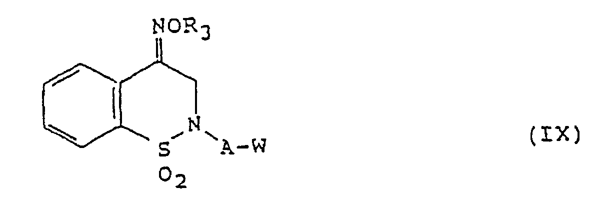 zu erhalten, wobei A, R₃ und W wie vorstehend definiert sind, und anschließendes Umsetzen der Verbindung der Formel (IX) mit einer stickstoffhaltigen Verbindung, welche durch die folgende Formel (V) dargestellt ist: 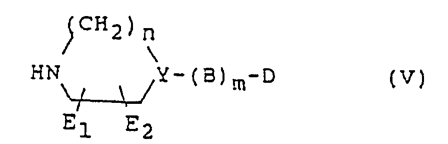 wobei B, D, E₁, E₂, Y, m und n wie vorstehend definiert sind.

17. Verfahren zur Herstellung eines Benzothiazinderivats gemäß Anspruch 1, welches durch die folgende Formel (Ih) dargestellt ist: 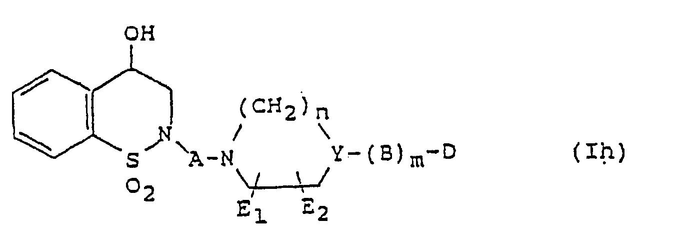 wobei A, B, D, E₁, E₂, Y, m und n wie vorstehend definiert sind, welches das Reduzieren einer Verbindung, welche durch die folgende Formel (VIII) dargestellt ist 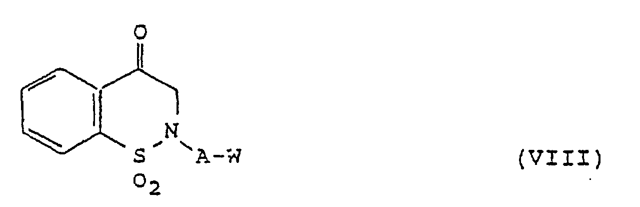 umfasst, wobei A und W wie vorstehend definiert sind, um eine Verbindung der folgenden Formel (X) 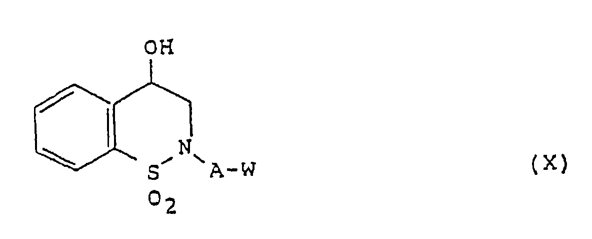 zu erhalten, wobei A und W wie vorstehend definiert sind, und
anschließendes Umsetzen der Verbindung der Formel (X) mit einer stickstoffhaltigen Verbindung, welche durch die folgende Formel (V) dargestellt ist: 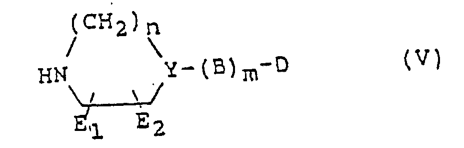 wobei B, D, E₁, E₂, Y, m und n wie vorstehend definiert sind.

18. Verfahren zur Herstellung eines Benzothiazinderivats gemäß Anspruch 1, welches durch die folgende Formel (Ii) dargestellt ist: 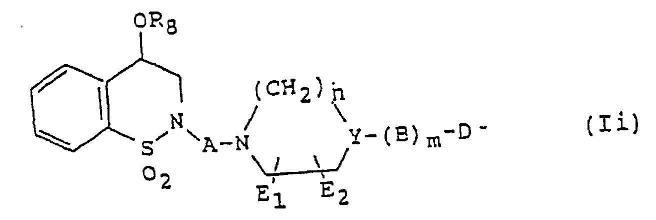 wobei R₈ einen substituierten oder unsubstituierten Alkyl- oder einen substituierten oder unsubstituierten Aralkylrest darstellt und A, B, D, E₁, E₂, Y, m und n wie vorstehend definiert sind, welches umfasst:
Umsetzen einer Verbindung, welche durch die folgende Formel (X) dargestellt ist: 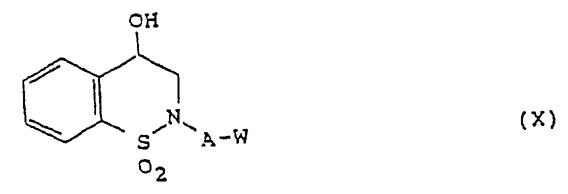
wobei A und W wie vorstehend definiert sind, mit einer Verbindung der folgenden Formel (XI):
R₈-W" (XI)
wobei W" einen eliminativen Substituenten darstellt und R₈ wie vorstehend definiert ist, um eine Verbindung der folgenden Formel (XII) 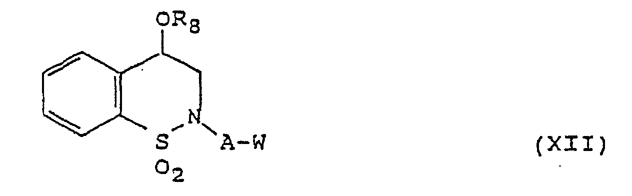 zu erhalten, wobei A, R₈ und W wie vorstehend definiert sind, und anschließendes Umsetzen der Verbindung der Formel (XII) mit einer stickstoffhaltigen Verbindung, welche durch die folgende Formel (V) dargestellt ist: 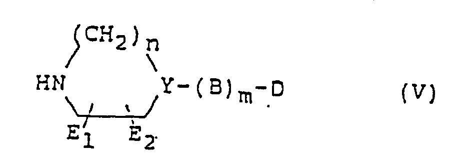 wobei B, D, E₁, E₂, Y, m und n wie vorstehend definiert sind.

19. Verfahren zur Herstellung einer Verbindung gemäß Anspruch 1, welche durch die folgende Formel (Ij) dargestellt ist: 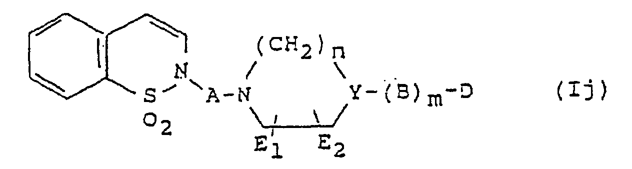 wobei A, B, D, E₁, E₂, Y, m und n wie vorstehend definiert sind, welches das Dehydratisieren einer Verbindung, welche durch die folgende Formel (X) dargestellt ist 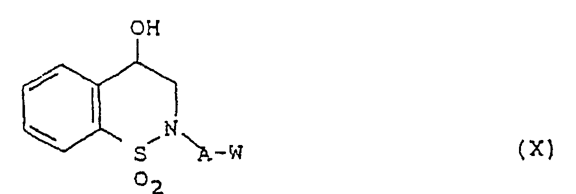 umfasst, wobei A und W wie vorstehend definiert sind, um eine Verbindung, welche durch die folgende Formel (XIII) dargestellt ist 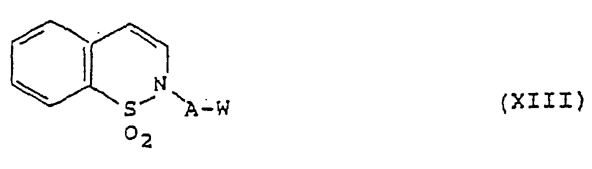 zu erhalten, wobei A und W wie vorstehend definiert sind, und anschließendes Umsetzen der Verbindung der Formel (XIII) mit einer stickstoffhaltigen Verbindung, welche durch die folgende Formel (V) dargestellt ist: 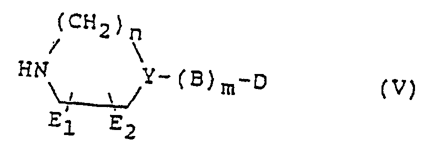 wobei B, D, E₁, E₂, Y, m und n wie vorstehend definiert sind.

20. Verfahren zur Herstellung einer Verbindung gemäß Anspruch 1, welche durch die folgende Formel (Ik) dargestellt ist: 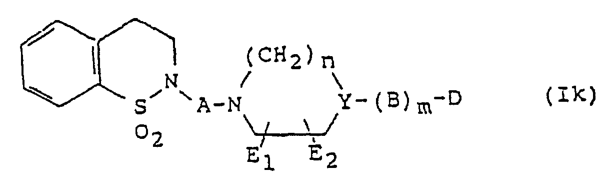 wobei A, B, D, E₁, E₂, Y, m und n wie vorstehend definiert sind, welches das Reduzieren der Verbindung, welche durch die folgende Formel (XIII) dargestellt ist 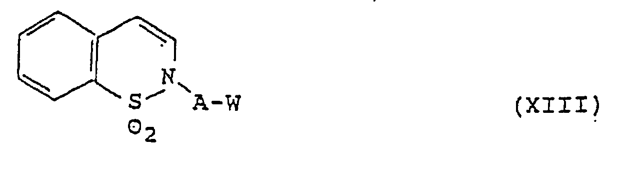 umfasst, wobei A und W wie vorstehend definiert sind, um eine Verbindung der folgenden Formel (XTV) 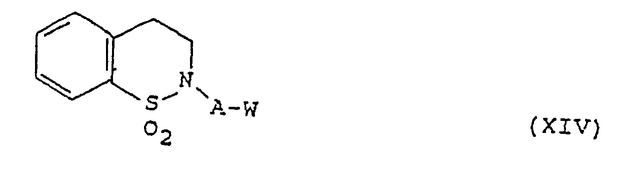 zu erhalten, wobei A und W wie vorstehend definiert sind, und anschließendes Umsetzen der Verbindung der Formel (XIV) mit einer stickstoffhaltigen Verbindung, welche durch die folgende Formel (V) dargestellt ist: 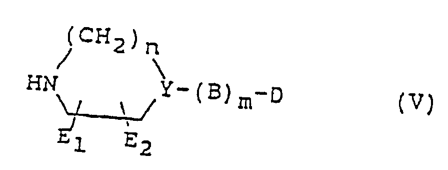 wobei B, D, E₁, E₂, Y, m und n wie vorstehend definiert sind.

21. Serotonin-2-Rezeptorantagonist, welcher als einen wirksamen Inhaltsstoff ein Benzothiazinderivat oder ein Salz davon gemäß Anspruch 1 enthält.

22. Therapeutische Zusammensetzung, welche als einen wirksamen Inhaltsstoff ein Benzothiazinderivat oder ein Salz davon gemäß Anspruch 1 enthält.

23. Verwendung eines Benzothiazinderivats gemäß Anspruch 1 bis 10 zur Herstellung einer therapeutischen Zusammensetzung zur Behandlung von Kreislauferkrankungen.

## Revendications

1. Dérivé de benzothiazine représenté par la formule suivante (I) : 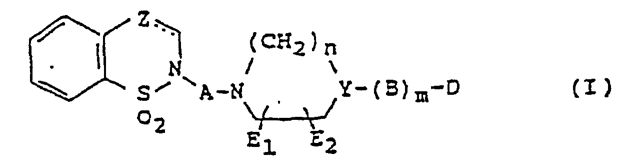 dans laquelle la ligne en pointillés indique la présence ou l'absence d'une liaison, et
lorsque la ligne en pointillés indique la présence de la liaison, Z représente un des groupes suivants : 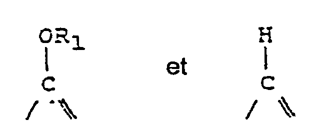 où R₁ représente un groupe alkyle en C₁-C₄ linéaire ou ramifié qui peut être non substitué ou substitué par un ou plusieurs atomes d'halogène, et/ou groupes alkoxy en C₁-C₄, ou un groupe aralkyle en C₇-C₂₂, qui peut être non substitué ou substitué par un ou plusieurs atomes d'halogène, groupes alkyle en C₁-C₄ et/ou groupes alkoxy en C₁-C₄, mais lorsque la ligne en pointillés indique l'absence de la liaison, Z représente un des groupes suivants : 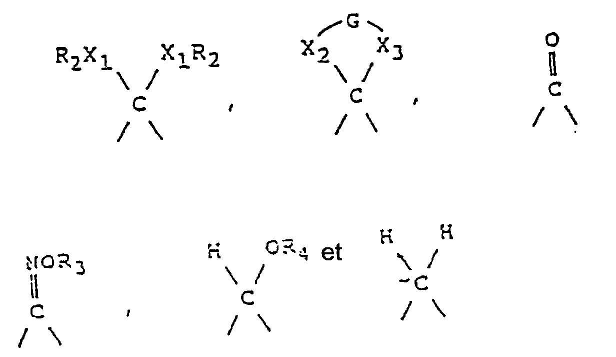 dans lesquels R₂ représente un groupe alkyle C₁-C₄ linéaire ou ramifié, qui peut être non substitué ou substitué par un ou plusieurs atomes d'halogène, et/ou groupes alkoxy en C₁-C₄, un groupe aryle en C₆-C₁₄, qui peut être non substitué ou substitué par un ou plusieurs atomes d'halogène, groupes alkyle en C₁-C₄ et/ou groupes alkoxy en C₁-C₄, ou un groupe aralkyle en C₇-C₂₂ qui peut être non substitué ou substitué par un ou plusieurs atomes d'halogène, groupes alkyle en C₁-C₄ et/ou groupes alkoxy en C₁-C₄,
R₃ représente un atome d'hydrogène, un groupe alkyle en C₁-C₄ linéaire ou ramifié, qui peut être non substitué ou substitué par un ou plusieurs atomes d'halogène, et/ou groupes alkoxy C₁-C₄, un groupe aryle en C₆-C₁₄, qui peut être non substitué ou substitué par un ou plusieurs atomes d'halogène, groupes alkyle en C₁-C₄ et/ou groupes alkoxy en C₁-C₄, ou un groupe aralkyle en C₇-C₂₂, qui peut être non substitué ou substitué par un ou plusieurs atomes d'halogène, groupes alkyle en C₁-C₄ et/ou groupes alkoxy en C₁-C₄,
R₄ représente un atome d'hydrogène un groupe alkyle en C₁-C₄ linéaire ou ramifié qui peut être non substitué ou substitué par un ou plusieurs atomes d'halogène et/ou groupes alkoxy C₁-C₄, ou un groupe aralkyle en C₇-C₂₂ qui peut être non substitué ou substitué par un ou plusieurs atomes d'halogène, groupes alkyle en C₁-C₄ et/ou groupes alkoxy en C₁-C₄,
X₁, X₂ et X₃ représentent chacun indépendamment un atome d'oxygène ou un atome de soufre, G représente un groupe éthylène,
A représente un groupe alkylène en C₂-C₁₀, un groupe alcénylène en C₄-C₁₀ ou un groupe alcynylène en C₄-C₁₀ tous pouvant être linéaires ou ramifiés et pouvant être substitués par un ou plusieurs atomes d'halogène ;
Y représente CH ou un atome d'azote ; et, lorsque Y représente CH, m est égal à 0 ou 1, n est égal à 1 ou 2, B représente un groupe carbonyle, mais lorsque Y représente un atome d'azote, m est égal à 0 ou 1, n est égal à 2 ou 3, B représente un groupe -CHR₇- dans lequel R₇ représente un groupe aryle en C₆-C₁₄, qui peut être non substitué ou substitué par un ou plusieurs atomes d'halogène, groupes alkyle en C₁-C₄ et/ou groupes alkoxy en C₁-C₄,
E₁ et E₂ représentent chacun indépendamment un atome d'hydrogène ou un groupe alkyle en C₁-C₄ et,
D représente un groupe hydrocarboné aromatique en C₆-C₂₈ ou un groupe hétérocyclique aromatique, qui est monocyclique ou bicyclique et contient jusqu'à trois atomes d'oxygène, de soufre et/ou d'azote, le groupe hydrocarboné aromatique ou hétérocyclique aromatique pouvant être substitué par des atomes d'halogène, des groupes alkyle en C₁-C₄, des groupes alkoxyle en C₁-C₄, des groupes aryle en C₆-C₁₄, des groupes aralkyle en C₇-C₂₂, des groupes aralkyloxy en C₇-C₂₂, des groupes cyano ; des groupes nitro ; des groupes carboxyle ; des groupes alkoxycarbonyle ayant un reste alcool en C₁-C₆ ; des groupes alkylsulfonylamino inférieur ayant un reste alkyle en C₁-C₄ ; des groupes carbamoyle ; et des groupes hydroxyle ; dans lesquels les substituants pour les groupes alkyle en C₁-C₄, les groupes aralkyle en C₇-C₂₂ et les groupes alkyle en C₆-C₁₄ sont des atomes d'halogène, des groupes alkyle en C₁-C₄ et/ou des groupes alkoxy en C₁-C₄, ou un sel de celui-ci, sous réserve que lorsque
E₁ et E₂ représentent hydrogène,
n a une valeur de 2
Y est CH
B est méthylène
m a une valeur de 1
Z est carbonyle
A est un pont alkylène en C₂ ou C₃ non substitué
D ne représente pas 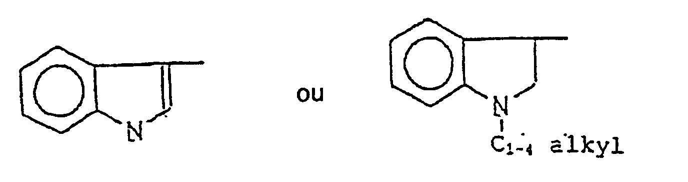
dans lesquels le noyau benzyle est non substitué ou substitué par halogène.

2. Dérivé de benzothiazine ou sel de celui-ci selon la revendication 1, dans lequel dans la formule (I), Z représente un groupe : 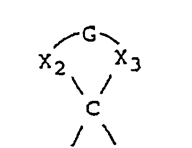 dans lequel G, X₂ et X₃ ont les mêmes significations que définies ci-dessus.

3. Dérivé de benzothiazine ou sel de celui-ci selon la revendication 1, dans lequel dans la formule (I), Z représente un des groupes:

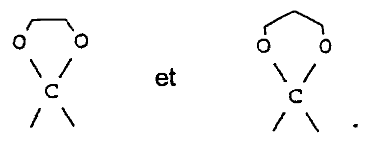

4. Dérivé de benzothiazine ou sel de celui-ci selon la revendication 1, dans lequel dans la formule (I), Z représente un des groupes:

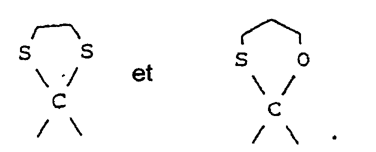

5. Dérivé de benzothiazine ou sel de celui-ci selon la revendication 1, dans lequel dans la formule (I), Z représente un groupe : 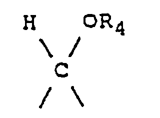 dans lequel R₄ a la même signification que définie ci-dessus.

6. Dérivé de benzothiazine ou sel de celui-ci selon la revendication 1, dans lequel dans la formule (I), Z représente un groupe : 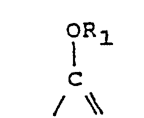 dans lequel R₁ a la même signification que définie ci-dessus.

7. Dérivé de benzothiazine ou sel de celui-ci selon l'une quelconque des revendications 1, 2, 3, 4, 5 et 6, dans lequel dans la formule (I), A représente un groupe éthylène ou triméthylène.

8. Dérivé de benzothiazine ou sel de celui-ci selon l'une quelconque des revendications 1, 2, 3, 4, 5, 6 et 7, dans lequel dans la formule (I), Y représente CH, n est égal à 2, B représente un groupe carbonyle, m est égal à 0 ou 1, et D représente un groupe phényle substitué ou non substitué.

9. Dérivé de benzothiazine ou sel de celui-ci selon l'une quelconque des revendications 1, 2, 3, 4, 5, 6 et 7, dans lequel dans la formule (I), Y représente un atome d'azote, n est égal à 2, m est égal à 0; et D représente un groupe phényle substitué ou non substitué.

10. Dérivé de benzothiazine ou sel de celui-ci selon l'une quelconque des revendications 1, 2, 3, 4, 5, 6, 7, 8 et 9, dans lequel dans la formule (I), E₁ et E₂ représentent chacun un atome d'hydrogène.

11. Procédé de préparation d'un dérivé de benzothiazine selon la revendication 1 dans lequel
Z représente un groupe : 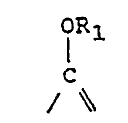 dans lequel R₁ a la même signification que définie ci-dessus mais, lorsque la liaison indiquée par la ligne en pointillés est absente, Z représente un des groupes représentés par les formules suivantes : 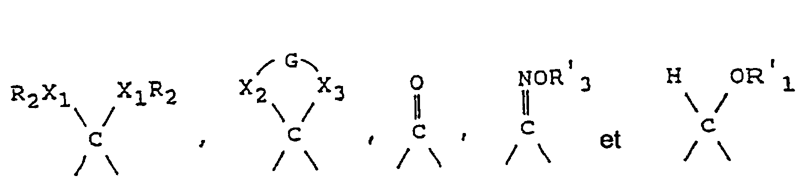
dans lesquelles R'₁ représente un groupe alkyle qui peut être non substitué ou substitué par un ou plusieurs atomes d'halogène, et/ou groupes alkoxy en C₁-C₄,
R'₃ représente un groupe alkyle, qui peut être non substitué ou substitué par un ou plusieurs atomes d'halogène, et/ou groupes alkoxy en C₁-C₄, un groupe aryle qui peut être non substitué ou substitué par un ou plusieurs atomes d'halogène, groupes alkyle en C₁-C₄ et/ou groupes alkoxy C₁-C₄, ou un groupe aralkyle qui peut être non substitué ou substitué par un ou plusieurs atomes d'halogène, groupes alkyle en C₁-C₄ et/ou groupes alkoxy en C₁-C₄,
et G, R₂, X₁, X₂ et X₃ ont les mêmes significations que définies ci-dessus,
et A, B, D, E₁, E₂, Y, m et n ont les mêmes significations que définies ci-dessus, qui comprend :
la réaction d'un composé qui est représenté par la formule suivante (II) : 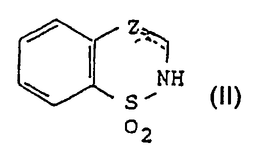
dans laquelle Z a la même signification que définie ci-dessus, avec un composé représenté par la formule suivante (III) :
W-A-W' (III)
dans laquelle A a la même signification que définie ci-dessus et W et W' peuvent être identiques ou différents et représentent individuellement un substituant facilement remplaçable par un groupe amino, pour obtenir un composé représenté par la formule suivante (IV) : 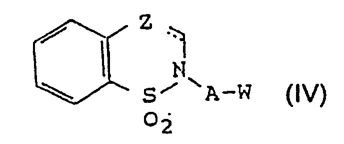 dans laquelle A, W et Z ont les mêmes significations que définies ci-dessus ; et
ensuite la réaction du composé de formule (IV) avec un composé contenant un azote représenté par la formule suivante (V) : 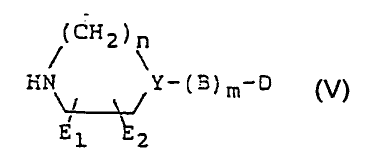 dans laquelle B, D, E₁, E₂, Y, m et n ont les mêmes significations que définies ci-dessus.

12. Procédé de préparation d'un dérivé de benzothiazine selon la revendication 1, représenté par la formule (I),
dans laquelle A, B, D, E₁, E₂, Y, Z, m et n ont les mêmes significations que définies à la revendication 11, qui comprend :
la réaction d'un composé, qui est représenté par la formule suivante (II) : 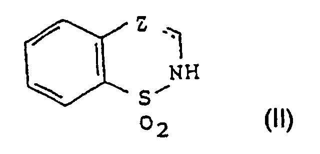
dans laquelle Z a la même signification que définie à la revendication 11 avec un composé contenant un azote représenté par la formule suivante (VI) : 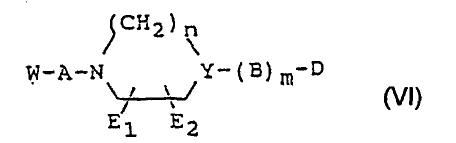 dans laquelle A, B, D, E₁, E₂, W, Y, m et n ont les mêmes significations que définies ci-dessus.

13. Procédé de préparation d'un dérivé de benzothiazine selon la revendication 1 représentée par la formule suivante (Ic): 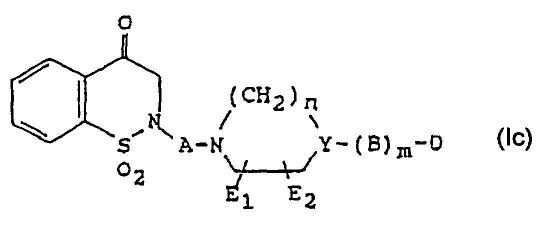 dans laquelle A, B, D, E₁, E₂, Y, m et n ont les mêmes significations que définies ci-dessus, qui comprend la conversion de Z₂ d'un dérivé de benzothiazine en un groupe carbonyle, ledit dérivé étant représenté par la formule suivante (Ib) : 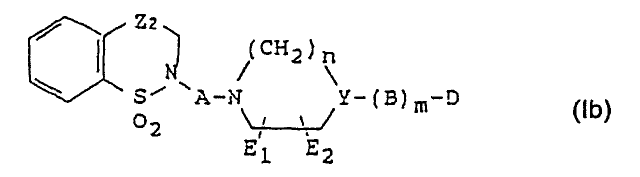 dans laquelle Z₂ représente un groupe : 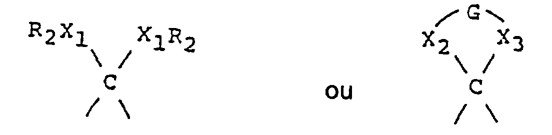 dans lesquels G, R₂, X₁, X₂ et X₃ ont les mêmes significations que définies ci-dessus, et A, B, D, E₁, E₂, Y, m et n ont les mêmes significations que définies ci-dessus.

14. Procédé de préparation d'un dérivé de benzothiazine selon la revendication 1 représenté par la formule suivante (le) : 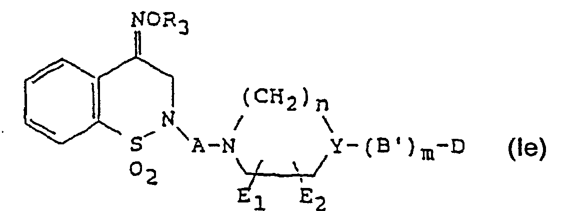 et B' a la même signification que B, A, D, E₁, E₂, E₃, m et n ont les mêmes significations que définies ci-dessus, qui comprend la réaction d'un dérivé de benzothiazine qui est représenté par la formule suivante (Id) : 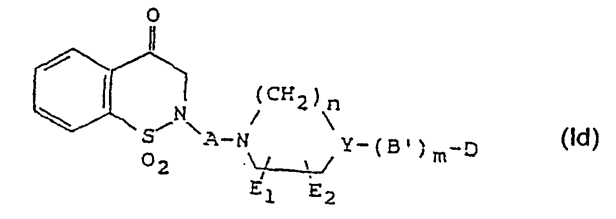 dans laquelle A, B', D, E₁, E₂, Y, m et n ont les mêmes significations que définies ci-dessus, avec une hydroxylamine représentée par la formule suivante (VII) :
NH₂OR₃ (VII)
dans laquelle R₃ a la même signification que définie ci-dessus ou un dérivé de celle-ci.

15. Procédé de préparation d'un dérivé de benzothiazine selon la revendication 1 représenté par la formule suivante (If) : 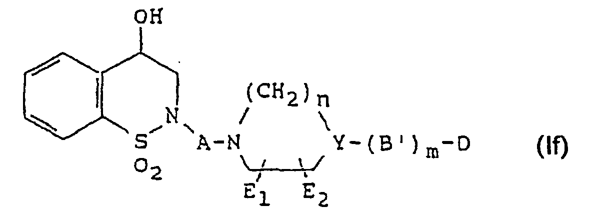 dans laquelle A, B', D, E₁, E₂, Y, m et n ont les memes significations que définies ci-dessus, qui comprend la réduction d'un dérivé de benzothiazine représenté par la formule suivante (Id) : 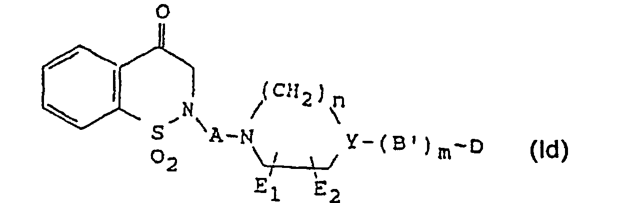 dans laquelle A, B', D, E₁, E₂, Y, m et n ont les mêmes significations que définies ci-dessus.

16. Procédé de préparation d'un dérivé de benzothiazine selon la revendication 1 représenté par la formule suivante (Ig) : 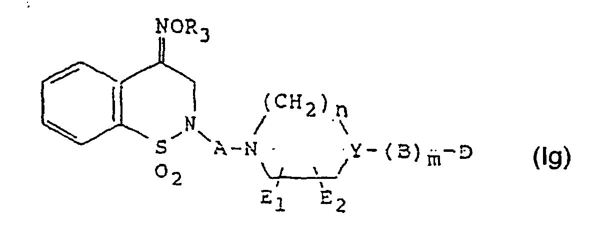 dans laquelle A, B, D, E₁, E₂, R₃, Y, m et n ont les mêmes significations que définies ci-dessus, qui comprend :
la réaction d'un composé, qui est représentée par la formule suivante (VIII) : 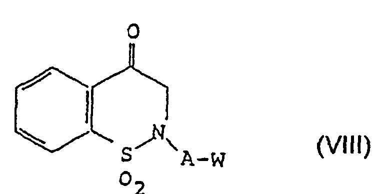
dans laquelle A et W ont les mêmes significations que définies ci-dessus, avec une hydroxylamine représentée par la formule suivante (VII) :
NH₂OR₃ (II)
dans laquelle R₃ a la même signification que définie ci-dessus ou avec un dérivé de celle-ci pour obtenir un composé représenté par la formule suivante (IX) : 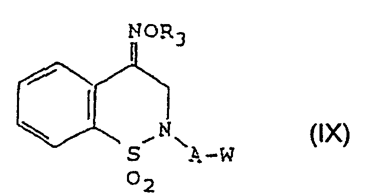 dans laquelle A, R₃ et W ont les mêmes significations que définies ci-dessus ; et ensuite
la réaction du composé de la formule (IX) avec un composé contenant un azote représenté par la formule suivante (V) : 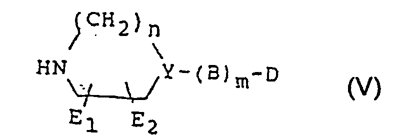 dans laquelle B, D, E₁, E₂, Y, m et n ont les mêmes significations que définies ci-dessus.

17. Procédé de préparation d'un dérivé de benzothiazine selon la revendication 1 représenté par la formule suivante (Ih) : 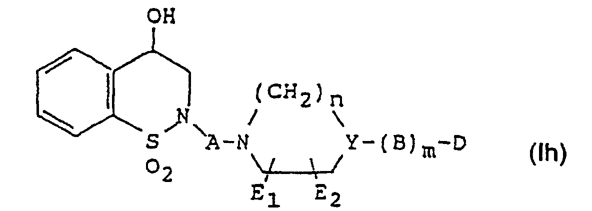 dans laquelle A, B, D, E₁, E₂, Y, m et n ont les mêmes significations que définies ci-dessus, qui comprend la réduction d'un composé, qui est représenté par la formule suivante (VIII) : 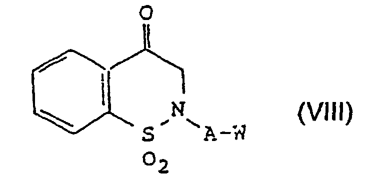 dans laquelle A et W ont les mêmes significations que définies ci-dessus, pour obtenir un composé représenté par la formule suivante (X) : 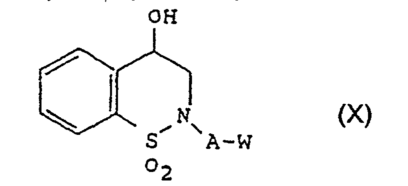 dans laquelle A et W ont les mêmes significations que définies ci-dessus, et ensuite
la réaction du composé de la formule (X) avec un composé contenant un azote représenté par la formule suivante (V) : 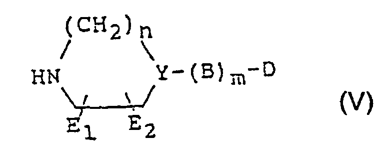 dans laquelle B, D, E₁, E₂, Y, m et n ont les mêmes significations que définies ci-dessus.

18. Procédé de préparation d'un dérivé de benzothiazine selon la revendication 1 représenté par la formule suivante (Ii) : 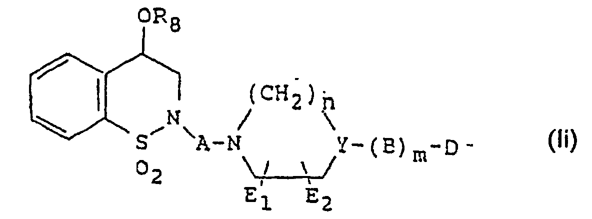 dans laquelle R₈ représente un groupe alkyle substitué ou non substitué ou un groupe aralkyle substitué ou non substitué, et A, B, D, E₁, E₂, Y, m et n ont les mêmes significations que définies ci-dessus, qui comprend :
la réaction d'un composé, qui est représenté par la formule suivante (X) : 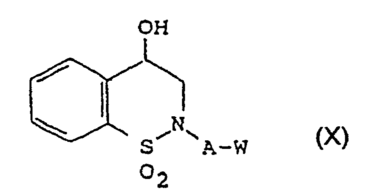
dans laquelle A et W ont les mêmes significations que définies ci-dessus, avec un composé représenté par la formule suivante (XI) :
R₈-W" (XI)
dans laquelle W" représente un substituant pouvant être éliminé et R₈ a la même signification que définie ci-dessus, pour obtenir un composé représenté par la formule suivante (XII) : 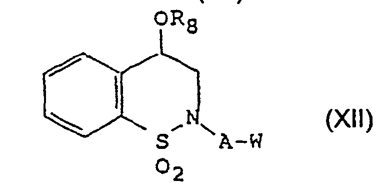 dans laquelle A, R₈ et W ont les mêmes significations que définies ci-dessus et ensuite,
la réaction du composé de formule (XII) avec un composé contenant un azote représenté par la formule suivante (V) : 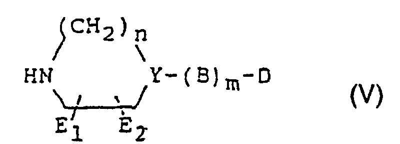 dans laquelle B, D, E₁, E₂, Y, m et n ont les mêmes significations que définies ci-dessus.

19. Procédé de préparation d'un composé selon la revendication 1 représenté par la formule suivante (Ij) : 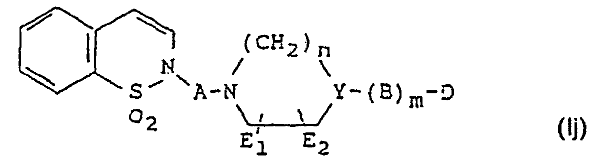 dans laquelle A, B, D, E₁, E₂, Y, m et n ont les mêmes significations que définies ci-dessus, qui comprend la déshydratation d'un composé représenté par la formule suivante (X) : 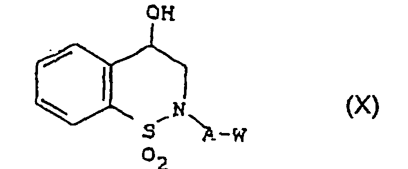 dans laquelle A et W ont les mêmes significations que définies ci-dessus, pour obtenir un composé représenté par la formule suivante (XIII) : 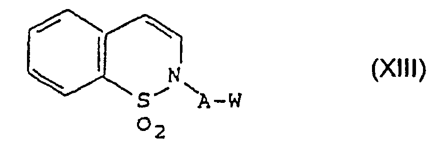 dans laquelle A et W ont les mêmes significations que définies ci-dessus et ensuite la réaction du composé de formule (XIII) avec un composé contenant un azote représenté par la formule suivante (V) : 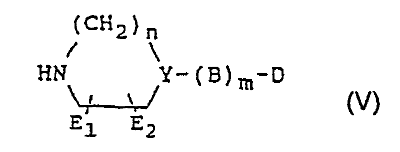 dans laquelle B, D, E₁, E₂, Y, m et n ont les mêmes significations que définies ci-dessus.

20. Procédé de préparation d'un composé selon la revendication 1 représenté par la formule suivante (Ik) : 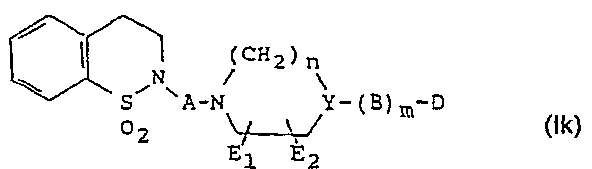 dans laquelle A, B, D, E₁, E₂, Y, m et n ont les mêmes significations que définies ci-dessus, qui comprend la réduction du composé représenté par la formule suivante (XIII) : 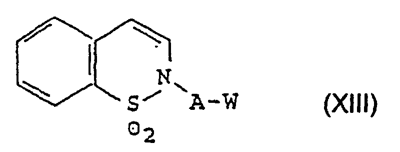 dans laquelle A et W ont les mêmes significations que définies ci-dessus pour obtenir un composé représenté par la formule suivante (XIV) : 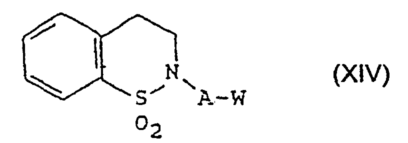 dans laquelle A et W ont les mêmes significations que définies ci-dessus puis la réaction du composé de formule (XIV) avec un composé contenant un azote représenté par la formule suivante (V) : 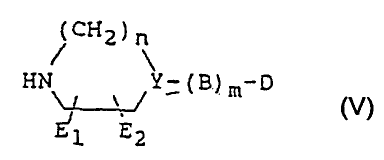 dans laquelle B, D, E₁, E₂, Y, m et n ont les mêmes significations que définies ci-dessus.

21. Antagoniste du récepteur-2 de la sérotonine, comprenant en tant qu'ingrédient actif un dérivé de benzothiazine ou un sel de celui-ci selon la revendication 1.

22. Composition thérapeutique, comprenant en tant qu'ingrédient actif un dérivé de benzothiazine ou un sel de celui-ci selon la revendication 1.

23. Utilisation d'un dérivé de benzothiazine tel que défini dans les revendications 1 à 10 pour la préparation d'une composition thérapeutique pour le traitement de maladies circulatoires.
